# EUROPEAN PATENT SPECIFICATION

(11) **EP 3 642 367 B1**
(45) Date of publication and mention of the grant of the patent: **05.08.2026**
(21) Application number: 18747030.7
(22) Date of filing: 20.06.2018
(51) Int. Cl.: C12Q 1/6883

(54) **ASSESSING CONDITIONS IN TRANSPLANT SUBJECTS USING DONOR-SPECIFIC CELL-FREE DNA**
BEWERTUNG DER ZUSTANDE IN EIN TRANPLANTIERTEN PATIENTEN MITELS SPENDER SPECIFISCHE CELLLOSE DNA
ÉTABLIR LES CONDITIONS DANS UN SUJET TRANSPLANTÉ UTILISANT L'ADN SANS CELLULE DU DONNEUR

(30) Priority: 20.06.2017 US 201762522556 P; 20.06.2017 US 201762522547 P; 20.06.2017 US 201762522560 P; 08.08.2017 US 201762542790 P; 08.08.2017 US 201762542787 P; 17.08.2017 US 201762546848 P; 17.08.2017 US 201762546872 P; 17.08.2017 US 201762546798 P; 12.10.2017 US 201762571709 P; 12.10.2017 US 201762571715 P; 24.10.2017 US 201762576641 P; 20.02.2018 US 201862632960 P; 05.04.2018 US 201862653261 P
(43) Date of publication of application: 29.04.2020
(73) Proprietor: The Medical College of Wisconsin, Inc., Milwaukee, WI 53226 (US)
(72) Inventor: MITCHELL, Aoy, Tomita, Elm Grove WI 53122 (US); MITCHELL, Michael, Elm Grove WI 53122 (US)
(74) Representative: Dolphin, Kirsty Mairi
(86) International application number: PCT/US2018/038604
(87) International publication number: WO 2018/237078

(56) References cited:
- WO-A1-2015/035177
- WO-A1-2015/069933
- WO-A1-2016/176662
- WO-A2-2013/159035
- KHUSH K K ET AL: "Circulating Cell-Free DNA as a Non-Invasive Marker of Pediatric Heart Transplant Rejection and Immunosuppressive Treatment", JOURNAL OF HEART AND LUNG TRANSPLANTATION, vol. 35, no. 4, 1 April 2016 (2016-04-01), XP029507330, ISSN: 1053-2498, DOI: 10.1016/J.HEALUN.2016.01.205
- HIDESTRAND MATS ET AL: "Highly Sensitive Noninvasive Cardiac Transplant Rejection Monitoring Using Targeted Quantification of Donor-Specific Cell-Free Deoxyribonucleic Acid", JOURNAL OF THE AMERICAN COLLEGE OF CARDIOLOGY, vol. 63, no. 12, 1 April 2014 (2014-04-01), pages 1224 - 1226, XP028831507, ISSN: 0735-1097, DOI: 10.1016/J.JACC.2013.09.029
- KEVIN P DALY: "Circulating donor-derived cell-free DNA: a true biomarker for cardiac allograft rejection?", 1 January 2015 (2015-01-01) - April 2015 (2015-04-01), pages 2305 - 58390135, XP055501307, Retrieved from the Internet <URL:http://atm.amegroups.com/article/view/5972/6670> DOI: 10.3978/j.issn.2305-5839.2015.01.35
- TARA K. SIGDEL ET AL: "A Rapid Noninvasive Assay for the Detection of Renal Transplant Injury", TRANSPLANTATION, vol. 96, no. 1, 1 July 2013 (2013-07-01), pages 97 - 101, XP055214444, ISSN: 0041-1337, DOI: 10.1097/TP.0b013e318295ee5a
- I. DE VLAMINCK ET AL: "Circulating Cell-Free DNA Enables Noninvasive Diagnosis of Heart Transplant Rejection", SCIENCE TRANSLATIONAL MEDICINE, vol. 6, no. 241, 18 June 2014 (2014-06-18), US, pages 241ra77 - 241ra77, XP055501377, ISSN: 1946-6234, DOI: 10.1126/scitranslmed.3007803
- ADAMEK MARTINA ET AL: "A fast and simple method for detecting and quantifying donor-derived cell-free DNA in sera of solid organ transplant recipients as a biomarker for graft function", CLINICAL CHEMISTRY AND LABORATORY MEDICINE : JOURNAL OF THE FORUM OF THE EUROPEAN SOCIETIES OF CLINICAL CHEMISTRY,, vol. 54, no. 7, 1 July 2016 (2016-07-01), pages 1147 - 1155, XP009502752, ISSN: 1437-4331, [retrieved on 20151117], DOI: 10.1515/CCLM-2015-0622
- RAGALIE WILLIAM S ET AL: "Noninvasive Assay for Donor Fraction of Cell-Free DNA in Pediatric Heart Transplant Recipients", JOURNAL OF THE AMERICAN COLLEGE OF CARDIOLOGY, vol. 71, no. 25, 26 June 2018 (2018-06-26), pages 2982 - 2983, XP085408908, ISSN: 0735-1097, DOI: 10.1016/J.JACC.2018.04.026

## Description

### FIELD OF THE INVENTION

This invention relates to methods for assessing an amount of donor-specific nucleic acids in a sample from a transplant subject. Such amounts can be used to assess certain conditions, such as a risk associated with a condition or disease of a transplanted organ, such as cardiac allograft vasculopathy or cardiac arrest, cellular rejection grade or a risk associated with the cellular rejection grade, antibody-mediated rejection or a risk associated with antibody-mediated rejection, or transplant organ injury, such as cellular injury, or a risk associated with transplant organ injury, such as cellular injury, of a subject following transplantation. This invention further relates to methods for determining a treatment or monitoring regimen for said subject. WO2015/035177 relates to transplant rejection detection but does not discloses MOMA based SNP quantification.

### SUMMARY OF INVENTION

The present disclosure is based, at least in part on the surprising discovery that the risk of certain conditions, such as cardiac allograft vasculopathy, cardiac arrest, cellular rejection, cellular injury, and/or antibody-mediated rejection, in a subject following organ transplantation may be predicted or assessed using the subject's donor-specific cell-free DNA level(s). Using any one of a variety of means to quantify the donor-specific cell-free DNA in a sample, the risk associated with cardiac allograft vasculopathy, cardiac arrest, cellular rejection, antibody-mediated rejection, transplant organ injury, and/or risk associated thereto can be determined, as well as monitored, over time.

Provided herein are methods related to such a determination. The invention is set out in the appended set of claims.

### BRIEF DESCRIPTION OF FIGURES

The accompanying figures are not intended to be drawn to scale. The figures are illustrative only and are not required for enablement of the disclosure.
**Fig. 1** provides an exemplary, non-limiting diagram of MOMA primers. In a polymerase chain reaction (PCR) assay, extension of the sequence containing SNV A is expected to occur, resulting in the detection of SNV A, which may be subsequently quantified. Extension of the SNV B, however, is not expected to occur due to the double mismatch.
**Fig. 2** shows results from a reconstruction experiment.
**Fig. 3** demonstrates the use of expectation maximization to predict donor genotype. Dashed line = first iteration, Solid line = second iteration, Final call= 10%.
**Fig. 4** demonstrates the use of expectation maximization to predict donor genotype. Final call= 5%. Shows results from a reconstruction experiment.
**Fig. 5** provides reconstruction experiment data demonstrating the ability to predict the donor genotype. Data have been generated with a set of 95 SNV targets.
**Fig. 6** illustrates an example of a computer system with which some embodiments may operate.
**Fig. 7** includes graphs showing the sensitivity and specificity of different methods to determine the threshold (cutpoint) of cellular grade 2 (or higher) rejection cutpoints. Method 1 and Method 2 (top row) are shown using donor-specific cell-free DNA (cf-DNA) from transplant patients.
**Fig. 8** shows the experimental determination of threshold values ("cutpoints") for CR0 and CR1 (top row), as well as CR0 and CR2 (bottom row) using donor-specific cf-DNA and two different methods.
**Fig. 9** shows the experimental determination of a threshold for CR0 using MOMA (with donor genotype information).
**Fig. 10** shows the experimental determination of a threshold for CR0 using MOMA (without donor genotype information).
**Fig. 11** shows a comparison of results of different methods of MOMA (with and without donor genotype information).
**Fig. 12** shows the experimental determination of a threshold point for cellular rejection grade 2 (CR2) using donor-specific cf-DNA and MOMA (with donor genotype information).
**Fig. 13** shows the experimental determination of a threshold point for cellular rejection grade 2 (CR2) using donor-specific cf-DNA and MOMA (with donor genotype information). The last sample obtained from each subject was used for analysis.
**Fig. 14** shows the experimental determination of a threshold point for cellular rejection grade 2 (CR2) using donor-specific cf-DNA and MOMA (without donor genotype information).
**Fig. 15** shows the experimental determination of a threshold point for cellular rejection grade 2 (CR2) using donor-specific cf-DNA and MOMA (without donor genotype information). The last sample obtained from each subject was used for analysis.
**Fig. 16** shows the experimental determination of a threshold point for cellular rejection grade 2 (CR2) using donor-specific cf-DNA and MOMA (with donor genotype information). Samples from subjects on mechanical support were excluded from the analysis.
**Fig. 17** shows the experimental determination of a threshold point for cellular rejection grade 2 (CR2) using donor-specific cf-DNA and MOMA (without donor genotype information). Samples from subjects on mechanical support were excluded from the analysis.
**Fig. 18** shows the experimental determination of a threshold point for cellular rejection grade 2 (CR2) using donor-specific cf-DNA and MOMA (without donor genotype information).
**Fig. 19** shows the experimental determination of a threshold point for cellular rejection grade 2 (CR2) using donor-specific cf-DNA and MOMA (without donor genotype information). Samples from subjects on mechanical support were excluded from analysis.
**Fig. 20** shows the experimental determination of a threshold point for cellular rejection grade 1 (CR1) using donor-specific cf-DNA and MOMA (with donor genotype information).
**Fig. 21** shows the experimental determination of a threshold point for cellular rejection grade 1 (CR1) using donor-specific cf-DNA and MOMA (with donor genotype information). Samples from subjects on mechanical support were excluded.
**Fig. 22** shows the experimental determination of a threshold point for cellular rejection grade 1 (CR1) using donor-specific cf-DNA and MOMA (with donor genotype information). The last sample obtained from each subject was used for analysis.
**Fig. 23** shows the experimental determination of a threshold point for cellular rejection grade 1 (CR1) using donor-specific cf-DNA and MOMA (without donor genotype information).
**Fig. 24** shows the experimental determination of a threshold point for cellular rejection grade 1 (CR1) using donor-specific cf-DNA and MOMA (without donor genotype information). Samples from subjects on mechanical support were excluded.
**Fig. 25** shows the experimental determination of a threshold point for cellular rejection grade 1 (CR1) using donor-specific cf-DNA and MOMA (without donor genotype information). The last sample obtained from each subject was used for analysis.
**Fig. 26** shows the experimental determination of a threshold point for cellular rejection grade 0 (CR0) using donor-specific cf-DNA and MOMA (with donor genotype information).
**Fig. 27** shows the experimental determination of a threshold point for cellular rejection grade 0 (CR0) using donor-specific cf-DNA and MOMA (with donor genotype information). Samples from subjects on mechanical support were excluded.
**Fig. 28** shows the experimental determination of a threshold point for cellular rejection grade 0 (CR0) using donor-specific cf-DNA and MOMA (with donor genotype information). The last sample obtained from each subject was used for analysis.
**Fig. 29** shows the experimental determination of a threshold point for cellular rejection grade 0 (CR0) using donor-specific cf-DNA and MOMA (without donor genotype information).
**Fig. 30** shows the experimental determination of a threshold point for cellular rejection grade 0 (CR0) using donor-specific cf-DNA and MOMA (without donor genotype information). Samples from subjects on mechanical support were excluded.
**Fig. 31** shows the experimental determination of a threshold point for cellular rejection grade 0 (CR0) using donor-specific cf-DNA and MOMA (without donor genotype information). The last sample obtained from each subject was used for analysis.
**Fig. 32** shows two graphs that depict experimentally determined thresholds (cutpoints) for antibody-mediated rejection (grade 0 vs. grades 1 or 2).
**Fig. 33** is a graph showing the experimental determination of a cutpoint (threshold) for antibody-mediated rejection using MOMA (donor genotype known).
**Fig. 34** is a graph showing the experimental determination of a cutpoint (threshold) for antibody-mediated rejection using MOMA (donor genotype known) and excluding samples from subjects on mechanical support.
**Fig. 35** is a graph showing the experimental determination of a cutpoint (threshold) for antibody-mediated rejection using MOMA (donor genotype known) and the final sample from each subject.
**Fig. 36** is a graph showing the experimental determination of a cutpoint (threshold) for antibody-mediated rejection using MOMA (donor genotype unknown).
**Fig. 37** is a graph showing the experimental determination of a cutpoint (threshold) for antibody-mediated rejection using MOMA (donor genotype unknown) and excluding samples from subjects on mechanical support.
**Fig. 38** is a graph showing the experimental determination of a cutpoint (threshold) for antibody-mediated rejection using MOMA (donor genotype unknown) and the final sample from each subject.
**Fig. 39** shows the experimental determination of cardiac allograft vasculopathy cutpoints (threshold) using donor-specific cell-free DNA (DS cf-DNA) with two different methods (top row).
**Fig. 40** shows the experimental determination of cardiac arrest cutpoints (threshold) using donor-specific cell-free DNA (DS cf-DNA) with two different methods (top row).
**Fig. 41** shows an experimental determination of a threshold for graft vasculopathy using MOMA (with donor genotype information), using 214 samples.
**Fig. 42** shows an experimental determination of a threshold for graft vasculopathy using MOMA (with donor genotype information), excluding samples from subjects on mechanical support.
**Fig. 43** shows an experimental determination of a threshold for graft vasculopathy using MOMA (without donor genotype information), using 214 samples.
**Fig. 44** shows an experimental determination of a threshold for graft vasculopathy using MOMA (without donor genotype information), excluding samples from subjects on mechanical support.
**Fig. 45** shows an experimental determination of a threshold for graft vasculopathy using MOMA (without donor genotype information), using the last sample obtained from each subject (N=79).
**Fig. 46** shows an experimental determination of a threshold for graft vasculopathy using MOMA (without donor genotype information).
**Fig. 47** shows an experimental determination of a threshold for cardiac arrest using donor-specific cf-DNA and MOMA (with donor genotype).
**Fig. 48** shows an experimental determination of a threshold for cardiac arrest using donor-specific cf-DNA and MOMA (with donor genotype). Samples from subjects on mechanical support were excluded from analysis.
**Fig. 49** shows an experimental determination of a threshold for cardiac arrest using donor-specific cf-DNA and MOMA (with donor genotype), using the last sample obtained from each subject.
**Fig. 50** shows an experimental determination of a threshold for cardiac arrest using donor-specific cf-DNA and MOMA (without known donor genotype).
**Fig. 51** shows an experimental determination of a threshold for cardiac arrest using donor-specific cf-DNA and MOMA (without known donor genotype). Samples from subjects on mechanical support were excluded from analysis.
**Fig. 52** shows an experimental determination of a threshold for cardiac arrest using donor-specific cf-DNA and MOMA (without known donor genotype), using the last sample obtained from each subject.
**Fig. 53** shows an experimental determination of a threshold for cardiac arrest using donor-specific cf-DNA and MOMA (without known donor genotype).
**Fig. 54** shows an experimental determination of a threshold for cardiac arrest using donor-specific cf-DNA and MOMA (without known donor genotype). Samples from subjects on mechanical support were excluded from analysis.
**Fig. 55** shows the increase in percent donor-specific fraction (DF) cell-free DNA (cf-DNA) pre-biopsy and post-biopsy.
**Fig. 56** shows the increase in donor genome equivalents (GE) per mL of plasma pre- and post-biopsy.
**Fig. 57** shows the experimental differentiation of CR1/2/3 and CR0 using MOMA (with known donor genotype) in all samples.
**Fig. 58** shows the experimental differentiation of CR1/2/3 and CR0 using MOMA (with known donor genotype). Healthy samples, those with CR0, were those with none of the following: death, cardiac arrest, MCS, treatment for infection, AMR 1&2, graft vasculopathy, and cancer.
**Fig. 59** shows the experimental differentiation of CR1/2/3 and CR0 using MOMA (with known donor genotype) using one sample per subject. The first rejection of the CR1/2/3 group was used as the sample, and the first sample from each member of the "healthy" group were used.
**Fig. 60** shows the experimental differentiation of CR1/2/3 and CR0 using MOMA (with known donor genotype) using plasma samples. Healthy samples, those with CR0, were those with none of the following: death, cardiac arrest, MCS, treatment for infection, AMR 1&2, graft vasculopathy, and cancer.
**Fig. 61** shows the experimental differentiation of CR1/2/3 and CR0 using MOMA (with known donor genotype) using whole blood samples from healthy subjects (those with none of the following: death, cardiac arrest, MCS, treatment for infection, AMR 1&2, graft vasculopathy, and cancer).
**Fig. 62** shows the experimental differentiation of CR1/2/3 and CR0 using MOMA (with unknown donor genotype) in all samples.
**Fig. 63** shows the experimental differentiation of CR1/2/3 and CR0 using MOMA (with unknown donor genotype). Healthy samples, those with CR0, were those with none of the following: death, cardiac arrest, MCS, treatment for infection, AMR 1&2, graft vasculopathy, and cancer.
**Fig. 64** shows the experimental differentiation of CR1/2/3 and CR0 using MOMA (with unknown donor genotype) using one sample per subject. The first rejection of the CR1/2/3 group was used as the sample, and the first sample from each member of the "healthy" group were used.
**Fig. 65** shows the experimental differentiation of CR1/2/3 and CR0 using MOMA (with unknown donor genotype) using plasma samples. Healthy samples, those with CR0, were those with none of the following: death, cardiac arrest, MCS, treatment for infection, AMR 1&2, graft vasculopathy, and cancer.
**Fig. 66** shows the experimental differentiation of CR1/2/3 and CR0 using MOMA (with unknown donor genotype) using whole blood samples. Healthy samples, those with CR0, were those with none of the following: death, cardiac arrest, MCS, treatment for infection, AMR 1&2, graft vasculopathy, and cancer.
**Fig. 67** is a schematic illustrating the data distribution for samples that underwent MOMA (with known donor genotypes). There were 1133 sample data available for the donor fraction.
**Fig. 68** is a schematic illustrating the data distribution for samples that underwent MOMA (with unknown donor genotypes). There were 1204 sample data available for the donor fraction.
**Fig. 69** includes two graphs showing the association of DF cfDNA with cellular rejection (CR) grade (CR0 vs. CR1 or CR2) by Method 1 (with known donor genotype; left graph) and by Method 2 (with unknown donor genotype; right graph) with receiver-operating characteristic (ROC) curves.

### DETAILED DESCRIPTION OF THE INVENTION

It has been found that donor-specific cf-DNA (DS cf-DNA) or donor fraction (DF) cf-DNA is correlated with certain conditions, such as cardiac allograft vasculopathy, cardiac arrest, cellular rejection, transplant organ injury (e.g., cellular injury), and antibody-mediated rejection, each of which, in combination or separately, can be used to assess and/or monitor a subject as a result. Therefore, aspects of the disclosure relate, at least in part, to methods of quantifying DS cf-DNA in a sample in order to assess or determine a grade of cellular rejection, antibody-mediated rejection, transplant organ injury (e.g., cellular injury), cardiac allograft vasculopathy and/or cardiac arrest, or risk associated therewith.

As used herein, "donor-specific nucleic acids" refers to nucleic acids that are from a transplant donor that can be found in a transplant recipient. Such nucleic acids are preferably cell-free DNA. "Cell-free DNA" (or cf-DNA) is DNA that is present outside of a cell, e.g., in the blood, plasma, serum, urine, etc. of a subject. As used herein, the compositions and methods provided herein can be used to determine an amount of cell-free DNA that is specific to a donor, or donor-specific cell-free DNA (DS cf-DNA), that can be found in a transplant recipient. As used herein, "transplant" refers to the moving of an organ or tissue from a donor to a recipient for the purpose of replacing the recipient's damaged or absent organ or tissue. Any one of the methods or compositions provided herein may be used on a sample from a subject that has undergone a transplant of an organ or tissue. In some embodiments, the transplant is a heart transplant.

The subject may be of any age; however, in some embodiments, the subject is less than 24, 22, 20, 18, 16, 14, 12, 10, 9, 8, 7, 6, 5, 4, 3, 2, or 1 year(s) old. In another embodiment, the subject is 24 years or older, for example, 26, 28, 30, 32, 34, 36, 38, 40, 45, 50, 55, 60, 65, 70, 75, or 80 years or older. The subject may be of any weight; however, in some embodiments, the subject is less than 30 kg, for example 28, 26, 24, 22, 20, 19, 18, 17, 16, 15, 14, 13, 12, 11, or 10 kg or fewer. In one embodiment, the subject is less than 16 years of age. In other embodiments, the subject is 30 kg or more, for example, 31, 32, 33, 34, 35, 36, 37, 38, 39, 40, 42, 44, 46, 48, 50, 55, 60, 65, 70, 75, 80, 85, 90, 95, or 100 kg or more. In one embodiment, the subject weighs less than 20 kg.

Importantly, amounts of DS cf-DNA can be used to assess or determine grades of transplant rejection, including even low grades of cellular rejection. As provided herein, any one of the methods can be used to assess a subject that has or is suspected of having a cellular rejection grade of CR2 or lower. Also as provided herein, any one of the methods can be used to assess a subject that has or is suspected of having a cellular rejection grade of CR2 or greater. Amounts of DS cf-DNA can also be used to assess or determine cardiac allograft vasculopathy and/or cardiac arrest, or risk thereof. As provided herein, any one of the methods can be used to assess a subject that has, is suspected of having, has had, or is at risk of having cardiac allograft vasculopathy and/or cardiac arrest. In addition, amounts of DS cf-DNA can be used to assess or determine antibody-mediated rejection. As provided herein, any one of the methods can be used to assess a subject that has or is suspected of having antibody-mediated rejection. Amounts of DS cf-DNA can also be used to assess or determine transplant organ injury, for example cellular injury such as following biopsy. As provided herein, any one of the methods can be used to assess a subject that has or is suspected of having transplant organ injury. As used herein, "suspected of having" refers to a subject whereby a clinician believes there is a likelihood the subject has a specific condition, such as a specific cellular rejection grade, cardiac allograft vasculopathy, antibody-mediated rejection, transplant organ injury (e.g., cellular injury).

In one embodiment of any one of the methods provided herein, the subject may be one that has cellular rejection of any one of the grades provided herein or that a clinician believes there is a likelihood of having any one of the grades of rejection provided herein. Such a subject may be suspected of having a certain grade of cellular rejection based on symptoms (and/or lack thereof) of cellular rejection grades. However, in some embodiments, the subject is one that has been determined to have rejection of a certain grade with one or more other tests, such as with a biopsy.

In one embodiment of any one of the methods provided herein, the subject may be one that has or has had cardiac allograft vasculopathy and/or cardiac arrest or that a clinician believes there is a likelihood of having cardiac allograft vasculopathy and/or cardiac arrest. Such a subject may be suspected of having, or the likelihood may be, based on symptoms (and/or lack thereof) of cardiac allograft vasculopathy and/or cardiac arrest. However, in some embodiments, the foregoing may be based on one or more other tests, such as with a biopsy.

In one embodiment of any one of the methods provided herein, the subject may be one that has antibody-mediated rejection or that a clinician believes there is a likelihood of having antibody-mediated rejection. Such a subject may be suspected of having antibody-mediated rejection based on symptoms (and/or lack thereof) of antibody-mediated rejection. However, in some embodiments, the subject is one that has been determined to have antibody-mediated rejection with one or more other tests, such as with a biopsy.

In one embodiment of any one of the methods provided herein, the subject may be one that has injury to a transplanted organ as provided herein or that a clinician believes there is a likelihood of having an injury to a transplanted organ as provided herein. Such a subject may be suspected of having an injury to the transplant organ based on symptoms (and/or lack thereof) of injury. However, in some embodiments, the subject is one that has been determined to have injury to the transplanted organ with one or more other tests, such as with a biopsy or with an additional biopsy.

In some embodiments, the methods provided herein can be used to confirm such a finding or monitor such a subject for worsening or improving condition.

Cellular rejection can be classified by grade as CR0, CR1, CR2 or CR3 such as according to the International Society for Heart and Lung Transplantation (ISHLT) grading scheme. An exemplary grading scheme is provided below.

### ISHLT-2004 Acute Cellular Rejection Grading Scheme

| **Grade** | **Histopathologic findings** |
|---|---|
| **0R, none** | None |
| **1R, mild** | Interstitial and/or perivascular infiltrate with up to 1 focus of mycocyte damage |
| **2R, moderate** | Two or more foci of infiltrate with associated myocyte damage |
| **3R, severe** | Diffuse infiltrate with multifocal myocyte damage ± edema ± hemorrhage ± vasculitis |

From Stewart et al JHLT, 2005

A subject may be assessed by determining or obtaining one or more amounts of DS cf-DNA. An amount of DS cf-DNA may be determined with experimental techniques, such as those provided elsewhere herein. "Obtaining" as used herein refers to any method by which the respective information or materials can be acquired. Thus, the respective information can be acquired by experimental methods. Respective materials can be created, designed, etc. with various experimental or laboratory methods, in some embodiments. The respective information or materials can also be acquired by being given or provided with the information, such as in a report, or materials. Materials may be given or provided through commercial means (i.e. by purchasing), in some embodiments.

Because of the ability to determine amounts of donor-specific nucleic acids, such as DS cf-DNA, and the correlation with cellular rejection grade, cardiac allograft vasculopathy, cardiac arrest, antibody-mediated rejection, and transplant organ injury (e.g., cellular injury), the methods and compositions provided herein can be used to assess subjects as provided herein. Thus, a risk of improving or worsening rejection condition can be determined in such subjects. A "risk" as provided herein, refers to the presence or absence or progression of any undesirable condition in a subject, or an increased likelihood of the presence or absence or progression of such a condition. As provided herein "increased risk" refers to the presence or progression of any undesirable condition in a subject or an increased likelihood of the presence or progression of such a condition. As provided herein, "decreased risk" refers to the absence of any undesirable condition or progression in a subject or a decreased likelihood of the presence or progression (or increased likelihood of the absence or nonprogression) of such a condition.

As provided herein, early detection or monitoring of conditions, such as cellular rejection, cardiac allograft vasculopathy, cardiac arrest, antibody-mediated rejection, and/or transplant organ injury (e.g., cellular rejection) can facilitate treatment and improve clinical outcomes. As mentioned above, any one of the methods provided can be performed on a subject as provided herein. Such methods can be used to monitor a subject over time, with or without treatment. Further, such methods can aid in the selection, administration and/or monitoring of a treatment or therapy. Accordingly, the methods provided herein can be used to determine a treatment or monitoring regimen.

"Determining a treatment regimen", as used herein, refers to the determination of a course of action for the subject that is undergoing clinically actionable rejection. In one embodiment of any one of the methods provided herein, determining a treatment regimen includes determining an appropriate therapy or providing information regarding an appropriate therapy to provide to a subject. In some embodiments of any one of the methods provided herein, the determining includes providing an appropriate therapy or information regarding an appropriate therapy to a subject. As used herein, information regarding a treatment or therapy or monitoring may be provided in written form or electronic form. In some embodiments, the information may be provided as computer-readable instructions. In some embodiments, the information may be provided orally.

The therapies can be, for example, for treating cellular rejection, such as an anti-rejection therapy. Anti-rejection therapies include, for example, immunosuppressives. Immunosuppressives include, but are not limited to, corticosteroids (e.g., prednisolone or hydrocortisone), glucocorticoids, cytostatics, alkylating agents (e.g., nitrogen mustards (cyclophosphamide), nitrosoureas, platinum compounds, cyclophosphamide (Cytoxan)), antimetabolites (e.g., folic acid analogues, such as methotrexate, purine analogues, such as azathioprine and mercaptopurine, pyrimidine analogues, and protein synthesis inhibitors), cytotoxic antibiotics (e.g., dactinomycin, anthracyclines, mitomycin C, bleomycin, mithramycin), antibodies (e.g., anti-CD20, anti-IL-1, anti-IL-2Ralpha, anti-T-cell or anti-CD-3 monoclonals and polyclonals, such as Atgam, and Thymoglobuline), drugs acting on immunophilins, ciclosporin, tacrolimus, sirolimus, interferons, opiods, TNF-binding proteins, mycophenolate, fingolimod and myriocin. In some embodiments, anti-rejection therapy comprises blood transfer or marrow transplant. Therapies can also include intravenous fluids, antibiotics, surgical drainage, early goal directed therapy (EGDT), vasopressors, steroids, activated protein C, drotrecogin alfa (activated), oxygen and appropriate support for organ dysfunction. This may include hemodialysis in kidney failure, mechanical ventilation in pulmonary dysfunction, transfusion of blood products, and drug and fluid therapy for circulatory failure. Ensuring adequate nutrition-preferably by enteral feeding, but if necessary, by parenteral nutrition-can also be included particularly during prolonged illness. Other associated therapies can include insulin and medication to prevent deep vein thrombosis and gastric ulcers. Other such therapies are known to those of ordinary skill in the art.

The therapies can be, for example, for treating antibody-mediated rejection. Antibody-mediated rejection therapies include, for example, immunosuppressives, plasmapheresis/plasma exchange, intravenous immunoglobulin, corticosteroids, antilymphocyte antibodies, and splenectomy.

Cardiac allograft vasculopathy therapies include, for example, retransplantation, percutaneous coronary interventions (PCI), coronary artery bypass grafting (CABG), transmyocardial laser revascularization and/or heparin-induced/mediated extracorporeal LDL plasmapheresis (HELP), as well as the administration of statins, anti-hypertensive agents, and/or anti-cytomegalovirus (anti-CMV) agents.

Therapies for when cardiac arrest is indicated include, but are not limited to, percutaneous coronary intervention (coronary angioplasty), coronary artery bypass grafting, or addition of an implantable cardioverter defibrillator (ICD). Further therapies include, but are not limited to, anti-arrhythmic agents, involuntary nervous system blockers or blood pressure medications. Further, a subject may be treated with coronary catheterization and/or a cardioverter-defibrillator may be implanted.

The therapies can be, for example, for treating organ injury, and may be used alone or in combination with other therapies related to transplant complications, such as an anti-rejection therapy. Anti-rejection therapies include, for example, immunosuppressives (described above).

Other such therapies are known to those of ordinary skill in the art.

Administration of a treatment or therapy may be accomplished by any method known in the art (see, e.g., Harrison's Principle of Internal Medicine, McGraw Hill Inc.). Preferably, administration of a treatment or therapy occurs in a therapeutically effective amount. Administration may be local or systemic. Administration may be parenteral (e.g., intravenous, subcutaneous, or intradermal) or oral. Compositions for different routes of administration are known in the art (see, e.g., Remington's Pharmaceutical Sciences by E. W. Martin).

The treatment and clinical course may be determined by the subject's cellular rejection grade and/or associated expected outcome. For example, if the amount of DS cf-DNA is equal to or 0.8 or greater, a cellular rejection grade of CR2 or greater may be indicated, and the subject may be treated with, or provided information related thereto, anti-rejection therapies, such as those described above. As another example, if the amount of DS cf-DNA is 0.2 or 0.3 or greater, cardiac allograft vasculopathy and/or cardiac arrest may be indicated, and the subject may be treated with, or provided information related thereto, therapies, such as those described above. In some embodiments, if the amount of DS cf-DNA is 0.2 or greater, antibody-mediated rejection may be indicated, and the subject may be treated with, or provided information related thereto, anti-rejection therapies, such as those described above.

"Determining a monitoring regimen", as used herein, refers to determining a course of action to monitor a condition in the subject over time. In one embodiment of any one of the methods provided herein, determining a monitoring regimen includes determining an appropriate course of action for determining the amount of DS cf-DNA in the subject over time or at a subsequent point in time, or suggesting such monitoring to the subject. This can allow for the measurement of variations in a clinical state and/or permit calculation of normal values or baseline levels (as well as comparisons thereto). In some embodiments of any one of the methods provided herein determining a monitoring regimen includes determining the timing and/or frequency of obtaining samples from the subject and/or determining or obtaining an amount of DS cf-DNA.

In some embodiments of any one of the methods provided herein, the DS cf-DNA may be detected as soon as 4 days after transplant surgery. In other embodiments, the DS cf-DNA may be quantified 5, 6, 7 or 8 or more days after transplant. In order to monitor the subject's DS cf-DNA levels, samples may be taken at monthly, bimonthly, or at more frequent intervals for up to 6 months, up to 8 months, up to 10 months, up to 12 months, or longer.

In the case of transplant organ injury, in some embodiments of any one of the methods provided herein, the DS cf-DNA level may be detected as soon as one minute after the injury. In other embodiments, the DS cf-DNA may be quantified 5, 10, 15, 20, 25, 30, 35, 40, 45, 50, 55, or 60 minutes after the injury. In another embodiment, the DS cf-DNA may be quantified 1, 2, 3, 4, 5, 6, 7, 8, 9, or 10 hours after the injury. In order to monitor the subject's DS cf-DNA levels, samples may be taken at monthly, bimonthly, or at more frequent intervals for up to 6 months, up to 8 months, up to 10 months, up to 12 months, or longer.

As increasing levels of DS cf-DNA have been found to correlate with increased risk, a clinician may determine that a subject should undergo more frequent sampling if the subject's DS cf-DNA is found to increase between time points. If a subject is found to have decreasing levels of DS cf-DNA between time points, a clinician may determine that less frequent sampling is sufficient. Timing and/or frequency of monitoring may also be determined by a comparison to one or more threshold values. For example, if the amount of DS cf-DNA is equal to or greater than 0.1, 0.2 or 0.3 (or any one of the thresholds provided herein) and/or is increasing, more frequent sampling may be needed, whereas, if the amount of DS cf-DNA is less than 0.1, 0.2 or 0.3 (or any one of the thresholds provided herein), and/or is not increasing, less frequent sampling may be required. Generally, subjects with higher or increasing amounts of DS cf-DNA require closer monitoring and more frequent sampling. In some embodiments of any one of the methods provided herein, each amount and time point may be recorded in a report or in a database.

Reports with any one or more of the values as provided herein are also provided in an aspect. Reports may be in oral, written (or hard copy) or electronic form, such as in a form that can be visualized or displayed. Preferably, the report provides the amount of donor-specific nucleic acids in a sample. In some embodiments, the report provides amounts of donor-specific nucleic acids in samples from a subject over time.

In some embodiments, the amounts are in or entered into a database. In one aspect, a database with such values is provided. From the amount(s), a clinician may assess the need for a treatment or monitoring of a subject. Accordingly, in any one of the methods provided herein, the method can include assessing the amount of nucleic acids in the subject at more than one point in time. Such assessing can be performed with any one of the methods or compositions provided herein.

As used herein, "amount" refers to any quantitative value for the measurement of nucleic acids and can be given in an absolute or relative amount. Further, the amount can be a total amount, frequency, ratio, percentage, etc. As used herein, the term "level" can be used instead of "amount" but is intended to refer to the same types of values. Generally, unless otherwise provided, the amounts provided herein represent the ratio or percentage of DS cf-DNA in a sample relative to the total.

In some embodiments, any one of the methods provided herein can comprise comparing an amount of donor-specific nucleic acids to a threshold value, or to one or more prior amounts, to identify a subject at increased or decreased risk. In some embodiments of any one of the methods provided herein, a subject having an increased amount of donor-specific nucleic acids compared to a threshold value, or to one or more prior amounts, is identified as being at increased risk. In some embodiments of any one of the methods provided herein, a subject having a decreased or similar amount of donor-specific nucleic acids compared to a threshold value, or to one or more prior amounts, is identified as being at decreased or not increased risk.

"Threshold" or "threshold value" or "cutpoint", as used herein, refers to any predetermined level or range of levels that is indicative of the presence or absence of a condition or the presence or absence of a risk. The threshold value can take a variety of forms. It can be single cut-off value, such as a median or mean. It can be established based upon comparative groups, such as where the risk in one defined group is double the risk in another defined group. It can be a range, for example, where the tested population is divided equally (or unequally) into groups, such as a low-risk group, a medium-risk group and a high-risk group, or into quadrants, the lowest quadrant being subjects with the lowest risk and the highest quadrant being subjects with the highest risk. The threshold value can depend upon the particular population selected. For example, an apparently healthy population will have a different 'normal' range. As another example, a threshold value can be determined from baseline values before the presence of a condition or risk or after a course of treatment. Such a baseline can be indicative of a normal or other state in the subject not correlated with the risk or condition that is being tested for. In some embodiments, the threshold value can be a baseline value of the subject being tested. Accordingly, the predetermined values selected may take into account the category in which the subject falls. Appropriate ranges and categories can be selected with no more than routine experimentation by those of ordinary skill in the art. The threshold value of any one of the methods provided herein, can be any one of the threshold values provided herein, such as in the **Examples** or **Figures.**

The threshold values provided herein can be used to determine or assign a cellular rejection grade to a subject. Accordingly, if the amount of DS cf-DNA measured is less than 0.2, the subject may be assigned a cellular rejection grade of CR0. If the amount of DS cf-DNA measured is less than 0.15, the subject may be assigned a cellular rejection grade of CR0. If the amount is between 0.15 and 0.8, the subject may be assigned a cellular rejection grade of CR1. If the amount is between 0.15 but less than 0.8, the subject may be assigned a cellular rejection grade of CR1. If the amount is between 0.2 and 0.8, the subject may be assigned a cellular rejection grade of CR1. If the amount is between 0.2 but less than 0.8, the subject may be assigned a cellular rejection grade of CR1. If the amount is between 0.25 or 0.3 but less than 0.75 or 0.8, the subject may be assigned a cellular rejection grade of CR1. If the amount is equal to or greater than 0.8 or 0.9, then the subject may be assigned a cellular rejection grade of CR2 or greater. The assigning of a rejection grade can be done based on any one of the comparisons as provided herein with or without other indicators of such a grade.

The threshold values provided herein can also be used to determine cardiac allograft vasculopathy and/or cardiac arrest in a subject. Accordingly, if the amount of DS cf-DNA measured is equal to or greater than 0.2, 0.3, 0.4, or 0.5 cardiac allograft vasculopathy and/or cardiac arrest may be indicated. The assessment or determination can be done based on any one of the comparisons as provided herein with or without other indicators of cardiac allograft vasculopathy and/or cardiac arrest.

The threshold values provided herein can also be used to determine the presence or absence of antibody-mediated rejection, or risk associated therewith, in the subject. Accordingly, if the amount of DS cf-DNA measured is less than 0.2, the subject may not have antibody-mediated rejection. If the amount is equal to or greater than 0.2, then the subject may have antibody-mediated rejection. The determination of the presence or absence of antibody-mediated rejection can be done based on any one of the comparisons as provided herein with or without other indicators of such a condition.

The threshold values provided herein can be used to determine whether a subject has transplant organ injury. Accordingly, if the amount of DS cf-DNA measured is less than a threshold value, the subject may not have transplant organ injury. If the amount is greater than a threshold value, the subject may have transplant organ injury. Also, injury may be measured by examining the fold increase of DS cf-DNA in a subject, such as relative to a baseline in some embodiments. For this analysis, a first sample may be taken at a time point prior to the injury, and a second sample may be taken within 1, 2, 3, 4, 5, 10, 15, 20, 25, 30, 35, 40, 45, 50, 55, 60 minutes, or more after the injury. The fold increase between the two samples can then be determined. If the fold increase is above a threshold value, it may indicate that the subject has, or is at risk of having, transplant organ injury; if the fold increase is lower than a threshold value, it may indicate that the subject does not have, or is not at risk of having, transplant organ injury.

The threshold values can also be used for comparisons to make treatment and/or monitoring decisions. For example, if the amount of DS cf-DNA is equal to or greater than 0.1, 0.2 or 0.3 and/or increasing over time, further monitoring may be indicated. As a further example, if the amount is equal to 0.8 or greater, treatment of the subject may be indicated. If the amount is 0.3-0.5, 0.3-0.6 or 0.3-0.7, for example, additional testing of the subject, such as with a biopsy may be indicated. If the amount is equal to or greater than any one of the thresholds provided herein, additional testing of the subject, such as with a biopsy may be indicated.

Accordingly, any one of the methods provided herein may further include an additional test(s) for assessing the subject, or a step of suggesting such further testing to the subject (or providing information about such further testing). The additional test(s) may be any one of the methods provided herein. The additional test(s) may be any one of the other methods provided herein or otherwise known in the art as appropriate.

Exemplary additional tests for subjects, include, but are not limited to, echocardiogram, coronary angiography, intravascular ultrasound (IVUS), biopsy (e.g., endomycardial biopsy), stress echocardiography, CT coronary angiography, coronary flow reserve assessment (contrast-enhanced echocardiography), stress myocardial perfusion scintigraphy, positron emission tomography (PET) scanning, and measurement of serum biomarkers, such as BNP and/or troponin. The type of additional test(s) will depend upon the severity of the condition of the subject and/or is well within the determination of the skilled artisan.

Exemplary additional tests include, but are not limited to, presence of donor-specific antibody (HLA antibodies), positive C4d staining on biopsy (e.g., renal biopsy, endomycardial biopsy), and histopathological evidence of antibody-mediated injury (e.g., glomerulitis, peritubular capillaritis, arteritis). In some embodiments of any one of the methods provided herein, the other test is a determination of the level of BNP and/or troponin in the transplant recipient. In other embodiments of any one of the methods provided herein, the other test in addition to the level of BNP and/or troponin or in place thereof is an echocardiogram. The type of additional test(s) will depend upon the condition, suspected condition, or severity thereof and/or is well within the determination of the skilled artisan.

The amount of DS cf-DNA may be determined by a number of methods. In some embodiments such a method is a sequencing-based method. For example, the DS cf-DNA may be measured by analyzing the DNA of a sample to identify multiple loci, an allele of each of the loci may be determined, and informative loci may be selected based on the determined alleles. As used herein, "loci" refer to nucleotide positions in a nucleic acid, e.g., a nucleotide position on a chromosome or in a gene. As used herein, "informative loci" refers to a locus where the genotype of the subject is homozygous for the major allele, while the genotype of the donor is homozygous or heterozygous for the minor allele. As used herein, "minor allele" refers to the allele that is less frequent in the population of nucleic acids for a locus. In some embodiments, the minor allele is the nucleotide identity at the locus in the nucleic acid of the donor. A "major allele", on the other hand, refers to the more frequent allele in a population. In some embodiments, the major allele is the nucleotide identity at the locus in the nucleic acid of the subject.

In some embodiments, the informative loci and alleles can be determined based on prior genotyping of the nucleic acids of the subject and the nucleic acids of the donor. For example, the genotype of the recipient and donor can be compared, and informative loci can be identified as those loci where the recipient is homozygous for a nucleotide identity and the donor is heterozygous or homozygous for a different nucleotide identity. Methods for genotyping are well known in the art and further described herein. In this example, the minor and major allele may be identified by determining the relative quantities of each allele at the informative locus and/or may be identified as the nucleotide identity at the informative locus in the donor DNA (minor allele) and the recipient DNA (major allele). Accordingly, the methods provided can further include a step of genotyping the recipient and donor, or obtaining or being provided with such genotypes.

An estimated allele frequency, such as the estimated minor allele frequency, at the informative loci may then be calculated in a suitable manner. In some embodiments, the estimated allele frequency may be calculated based on modeling the number of counts of the allele, such as the minor allele, at the informative loci using a statistical distribution. For example, the estimated allele frequency can be calculated by modeling allele read counts using a binomial distribution. In some embodiments, the peak of such a distribution is determined and is indicative of the percent donor-specific cf-DNA. A frequency of the minor allele at the informative loci may also be calculated using a maximum likelihood method. In some embodiments, the minor allele frequency (MAF) may be calculated with genotypes from plasma DNA of the subject, and donor genotypes for informative loci may be inferred using expectation maximization. In some embodiments, the read counts for the major and/or minor allele(s) can be corrected prior to estimating the allele frequency.

The DNA may be analyzed using any suitable next generation or high-throughput sequencing and/or genotyping technique. Examples of next generation and high-throughput sequencing and/or genotyping techniques include, but are not limited to, massively parallel signature sequencing, polony sequencing, 454 pyrosequencing, Illumina (Solexa) sequencing, SOLiD sequencing, ion semiconductor sequencing, DNA nanoball sequencing, heliscope single molecule sequencing, single molecule real time (SMRT) sequencing, MassARRAY^{®}, and Digital Analysis of Selected Regions (DANSR^{™}) (see, e.g., Stein RA (1 September 2008). "Next-Generation Sequencing Update". Genetic Engineering & Biotechnology News 28 (15); Quail, Michael; Smith, Miriam E; Coupland, Paul; Otto, Thomas D; Harris, Simon R; Connor, Thomas R; Bertoni, Anna; Swerdlow, Harold P; Gu, Yong (1 January 2012). "A tale of three next generation sequencing platforms: comparison of Ion torrent, pacific biosciences and illumina MiSeq sequencers". BMC Genomics 13 (1): 341; Liu, Lin; Li, Yinhu; Li, Siliang; Hu, Ni; He, Yimin; Pong, Ray; Lin, Danni; Lu, Lihua; Law, Maggie (1 January 2012). "Comparison of Next-Generation Sequencing Systems". Journal of Biomedicine and Biotechnology 2012: 1-11; Qualitative and quantitative genotyping using single base primer extension coupled with matrix-assisted laser desorption/ionization time-of-flight mass spectrometry (MassARRAY®). Methods Mol Biol. 2009;578:307-43; Chu T, Bunce K, Hogge WA, Peters DG. A novel approach toward the challenge of accurately quantifying fetal DNA in maternal plasma. Prenat Diagn 2010;30:1226-9; and Suzuki N, Kamataki A, Yamaki J, Homma Y. Characterization of circulating DNA in healthy human plasma. Clinica chimica acta; International Journal of Clinical Chemistry 2008;387:55-8).

In one embodiment, any one of the methods for determining DS cf-DNA may be any one of the methods of U.S. Publication No. 2015-0086477-A1.

An amount of DS cf-DNA may also be determined by a MOMA assay. In one embodiment, any one of the methods for determining DS cf-DNA may be any one of the methods of PCT Publication No. WO 2016/176662 A1.

The DS cf-DNA may be determined using differences in sequence identity between the subject and donor genotype. Such differences may be single nucleotide variants (SNVs); however, wherever a SNV is referred to herein, any difference in sequence identity between recipient and donor-specific nucleic acids is intended to also be applicable. Thus, any one of the methods or compositions provided herein may be applied to recipient versus donor-specific nucleic acids where there is a difference in sequence identity. As used herein, "single nucleotide variant" refers to a nucleic acid sequence within which there is sequence variability, preferably in some embodiments at a single nucleotide. These SNVs include any mutations specific to or that can identify DS cf-DNA. Primers can be prepared as provided herein for any one or more of the SNVs.

The nucleic acid sequence within which there is sequence identity variability is generally referred to as a "target". As used herein, a "SNV target" refers to a nucleic acid sequence within which there is sequence variability, such as at a single nucleotide. The SNV target has more than one allele, and in preferred embodiments, the SNV target is biallelic. Donor-specific nucleic acids can be quantified even at extremely low levels by performing amplification-based quantification assays, such as quantitative PCR assays, with primers specific for SNV targets. In some embodiments, the amount of donor-specific nucleic acids is determined by attempting an amplification-based quantification assay, such as quantitative PCR, with primers for a plurality of SNV targets. A "plurality of SNV targets" refers to more than one SNV target where for each target there are at least two alleles. Preferably, in some embodiments, each SNV target is expected to be biallelic and a primer pair specific to each allele of the SNV target is used to specifically amplify nucleic acids of each allele, where amplification occurs if the nucleic acid of the specific allele is present in the sample. As used herein, one allele may be the donor-specific version of a target sequence and another allele is the recipient-specific version of the sequence.

In an embodiment of any one of the methods or compositions provided herein, one or more primer pairs for SNV target(s) can be pre-selected based on knowledge that the SNV targets will be informative, such as with knowledge of genotype, such as of the donor. In such embodiments, the genotype of the donor is known or can be determined. Thus, any one of the methods provided herein, can include a step of genotyping the donor or obtaining the donor genotype.

In other embodiments of any one of the methods provided herein, the genotype of the donor is unknown. In an embodiment of such cases, the donor genotype may be inferred with an expectation maximization method. As an example, using the known recipient genotype, targets known to be homozygous in the recipient can be selected. Any contaminants can be attributed to donor-specific nucleic acids, and the resulting assay collection will consist of a tri-modal distribution: non-, half-, and fully-informative assays. With a sufficient number of recipient homozygous assays, the presence of donor fully-informative assays can be inferred.

For example, if a recipient genotype is homozygous and known, then measurements not associated with the recipient genotype (those that are truly donor-homozygous) will have the highest cluster, and equal the guess (fully-informative), as compared to those that are donor-heterozygous, which will only be at half the guess (half-informative). Then, a probability distribution can be plotted and an expectation maximization algorithm (EM) can be used to infer donor genotype. The EM algorithm can be used to infer the donor genotype frequency in any one of the methods provided herein. Accordingly, an EM algorithm may be used to infer the most likely donor genotypes at all assayed SNV targets. Using inferred donor genotypes, quantification may proceed as in the full-information scenario discussed above. EM may begin on the assumption that the minor allele ratio found at an assay follows a tri-modal distribution (one for each combination of recipient (A) and donor (B), i.e., AA, AB, and BB). With all donor genotypes unknown, it is possible to bootstrap from the knowledge that any assays exhibiting nearly zero minor allele are donor AA (i.e., recipient alleles), and the highest is donor BB. Initial guesses for all donor genotypes may then be recorded, and the mean of each cluster can be calculated. Enforcing that the donor BB assays' mean is twice that of the donor AB restricts the search. The algorithm then reassigns guessed donor genotypes based on the clusters and built-in assumptions. The process is iterative until no more changes are made. The final result is a set of the most likely donor genotypes given their measured divergence from the background. Generally, every target falls into the model; a result may be excluded if between groups after maximization.

In another embodiment of any one of the methods or compositions provided herein, primer pairs for a plurality of SNV targets can be selected for the likelihood at least one (or more) may be informative. In such embodiments, primer pairs for a panel of SNV targets are used in any one of the methods provided herein. In some embodiments, the panel of SNV targets is a panel of at least 30, 35, 40, 45, 50, 55, 60, 65, 70, 75, 80, 85, 90, 95 or more possible targets.

As used herein, "an informative SNV target" is one in which amplification with primers as provided herein occurs, and the results of which are informative. "Informative results" as provided herein are the results that can be used to quantify the level of donor-specific nucleic acids in a sample. In some embodiments, informative results exclude results that are considered "no call" or erroneous call results. From the informative results, allele percentages can be calculated using standard curves, in some embodiments of any one of the methods provided. In some embodiments of any one of the methods provided, the amount of donor-specific nucleic acids represents an average across informative results for the donor-specific nucleic acids, respectively.

The amount of donor-specific nucleic acids may be determined with the quantities of the major and minor alleles as well as the genotype of the recipient in some embodiments. In some embodiments of any one of the methods provided herein, the alleles can be determined based on prior genotyping of the subject. Methods for genotyping are well known in the art. Such methods include sequencing, such as next generation, hybridization, microarray, other separation technologies or PCR assays. Any one of the methods provided herein can include steps of obtaining such genotypes.

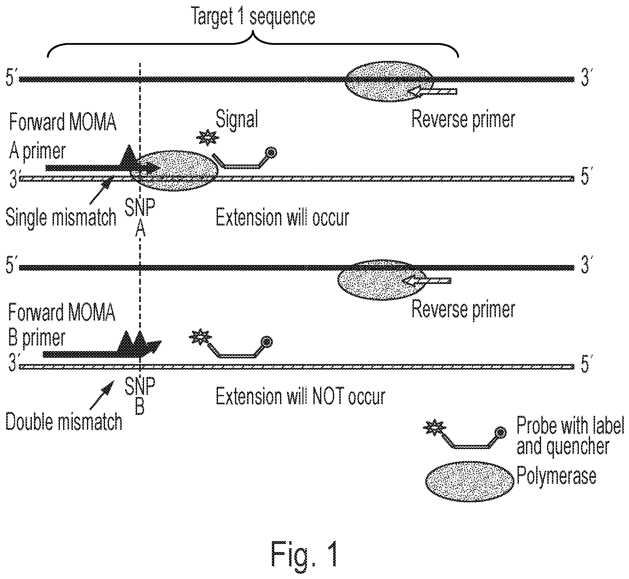

Primers for use in MOMA assays may be obtained, and any one of the methods provided herein can include a step of obtaining one or more primer pairs for performing the amplification-based quantification assays, such as PCR assays. Generally, the primers possess unique properties that facilitate their use in quantifying amounts of nucleic acids. For example, a forward primer of a primer pair can be mismatched at a 3' nucleotide (e.g., penultimate 3' nucleotide). In some embodiments of any one of the methods or compositions provided, this mismatch is at a 3' nucleotide but adjacent to the SNV position. In some embodiments of any one of the methods or composition provided, the mismatch positioning of the primer relative to a SNV position is as shown in **Fig. 1****.** Generally, such a forward primer, even with the 3' mismatch, will produce an amplification product (in conjunction with a suitable reverse primer) in an amplification reaction, such as a PCR reaction, thus allowing for the amplification and resulting detection of a nucleic acid with the respective SNV. If the particular SNV is not present, and there is a double mismatch with respect to the other allele of the SNV target, an amplification product will generally not be produced. Preferably, in some embodiments of any one of the methods or compositions provided herein, for each SNV target, a primer pair is obtained whereby specific amplification of each allele can occur without amplification of the other allele(s). "Specific amplification" refers to the amplification of a specific allele of a target without substantial amplification of another nucleic acid or without amplification of another nucleic acid sequence above background or noise. In some embodiments, specific amplification results only in the amplification of the specific allele.

In some embodiments of any one of the methods or compositions provided herein, for each SNV target that is biallelic, there are two primer pairs, each specific to one of the two alleles and thus have a single mismatch with respect to the allele it is to amplify and a double mismatch with respect to the allele it is not to amplify (if nucleic acids of these alleles are present). In some embodiments of any one of the methods or compositions provided herein, the mismatch primer is the forward primer. In some embodiments of any one of the methods or compositions provided herein, the reverse primer of the two primer pairs for each SNV target is the same.

These concepts can be used in the design of primer pairs for any one of the methods and compositions provided herein. It should be appreciated that the forward and reverse primers are designed to bind opposite strands (e.g., a sense strand and an antisense strand) in order to amplify a fragment of a specific locus of the template. The forward and reverse primers of a primer pair may be designed to amplify a nucleic acid fragment of any suitable size to detect the presence of, for example, an allele of a SNV target according to the disclosure. Any one of the methods provided herein can include one or more steps for obtaining one or more primer pairs as described herein.

It should be appreciated that the primer pairs described herein may be used in a multiplex amplification-based quantification assay, such as a PCR assay. Accordingly, in some embodiments of any one of the methods or compositions provided herein, the primer pairs are designed to be compatible with other primer pairs in a PCR reaction. For example, the primer pairs may be designed to be compatible with at least 1, at least 2, at least 3, at least 4, at least 5, etc. other primer pairs in a PCR reaction. As used herein, primer pairs in a PCR reaction are "compatible" if they are capable of amplifying their target in the same PCR reaction. In some embodiments, primer pairs are compatible if the primer pairs are inhibited from amplifying their target DNA by no more than 1%, no more than 2%, no more than 3%, no more than 4%, no more than 5%, no more than 10%, no more than 15%, no more than 20%, no more than 25%, no more than 30%, no more than 35%, no more than 40%, no more than 45%, no more than 50%, or no more than 60% when multiplexed in the same PCR reaction. Primer pairs may not be compatible for a number of reasons including, but not limited to, the formation of primer dimers and binding to off-target sites on a template that may interfere with another primer pair. Accordingly, the primer pairs of the disclosure may be designed to prevent the formation of dimers with other primer pairs or limit the number of off-target binding sites. Exemplary methods for designing primers for use in a multiplex PCR assay are known in the art or otherwise described herein.

In some embodiments, the primer pairs described herein are used in a multiplex amplification-based quantification assay, such as a PCR assay, to quantify an amount of donor-specific nucleic acids. Accordingly, in some embodiments of any one of the methods or compositions provided herein, the primer pairs are designed to detect genomic regions that are diploid, excluding primer pairs that are designed to detect genomic regions that are potentially non-diploid. In some embodiments of any one of the methods or compositions provided herein, the primer pairs used in accordance with the disclosure do not detect repeat-masked regions, known copy-number variable regions, or other genomic regions that may be non-diploid.

In some embodiments of any one of the methods provided herein, the amplification-based quantitative assay is any quantitative assay, such as whereby nucleic acids are amplified and the amounts of the nucleic acids can be determined. Such assays include those whereby nucleic acids are amplified with the MOMA primers as described herein and quantified. Such assays include simple amplification and detection, hybridization techniques, separation technologies, such as electrophoresis, next generation sequencing and the like.

In some embodiments of any one of the methods provided herein the PCR is quantitative PCR meaning that amounts of nucleic acids can be determined. Quantitative PCR include real-time PCR, digital PCR, TAQMAN^{™}, etc. In some embodiments of any one of the methods provided herein the PCR is "real-time PCR". Such PCR refers to a PCR reaction where the reaction kinetics can be monitored in the liquid phase while the amplification process is still proceeding. In contrast to conventional PCR, real-time PCR offers the ability to simultaneously detect or quantify in an amplification reaction in real time. Based on the increase of the fluorescence intensity from a specific dye, the concentration of the target can be determined even before the amplification reaches its plateau.

The use of multiple probes can expand the capability of single-probe real-time PCR. Multiplex real-time PCR uses multiple probe-based assays, in which each assay can have a specific probe labeled with a unique fluorescent dye, resulting in different observed colors for each assay. Real-time PCR instruments can discriminate between the fluorescence generated from different dyes. Different probes can be labeled with different dyes that each have unique emission spectra. Spectral signals are collected with discrete optics, passed through a series of filter sets, and collected by an array of detectors. Spectral overlap between dyes may be corrected by using pure dye spectra to deconvolute the experimental data by matrix algebra.

A probe may be useful for methods of the present disclosure, particularly for those methods that include a quantification step. Any one of the methods provided herein can include the use of a probe in the performance of the PCR assay(s), while any one of the compositions or kits provided herein can include one or more probes. Importantly, in some embodiments of any one or more of the methods provided herein, the probe in one or more or all of the PCR quantification assays is on the same strand as the mismatch primer and not on the opposite strand. It has been found that in so incorporating the probe in a PCR reaction, additional allele specific discrimination can be provided.

As an example, a TAQMAN^{™} probe is a hydrolysis probe that has a FAM^{™} or VIC^{®} dye label on the 5' end, and minor groove binder (MGB) non-fluorescent quencher (NFQ) on the 3' end. The TAQMAN^{™} probe principle generally relies on the 5'-3' exonuclease activity of Taq^{®} polymerase to cleave the dual-labeled TAQMAN^{™} probe during hybridization to a complementary probe-binding region and fluorophore-based detection. TAQMAN^{™} probes can increase the specificity of detection in quantitative measurements during the exponential stages of a quantitative PCR reaction.

PCR systems generally rely upon the detection and quantitation of fluorescent dyes or reporters, the signal of which increase in direct proportion to the amount of PCR product in a reaction. For example, in the simplest and most economical format, that reporter can be the double-stranded DNA-specific dye SYBR^{®} Green (Molecular Probes). SYBR^{®} Green is a dye that binds the minor groove of double-stranded DNA. When SYBR^{®} Green dye binds to a double-stranded DNA, the fluorescence intensity increases. As more double-stranded amplicons are produced, SYBR^{®} Green dye signal will increase.

It should be appreciated that the PCR conditions provided herein may be modified or optimized to work in accordance with any one of the methods described herein. Typically, the PCR conditions are based on the enzyme used, the target template, and/or the primers. In some embodiments, one or more components of the PCR reaction is modified or optimized. Non-limiting examples of the components of a PCR reaction that may be optimized include the template DNA, the primers (e.g., forward primers and reverse primers), the deoxynucleotides (dNTPs), the polymerase, the magnesium concentration, the buffer, the probe (e.g., when performing real-time PCR), the buffer, and the reaction volume.

In any of the foregoing embodiments, any DNA polymerase (enzyme that catalyzes polymerization of DNA nucleotides into a DNA strand) may be utilized, including thermostable polymerases. Suitable polymerase enzymes will be known to those skilled in the art, and include E. coli DNA polymerase, Klenow fragment of E. coli DNA polymerase I, T7 DNA polymerase, T4 DNA polymerase, T5 DNA polymerase, Klenow class polymerases, Taq polymerase, Pfu DNA polymerase, Vent polymerase, bacteriophage 29, REDTaq^{™} Genomic DNA polymerase, or sequenase. Exemplary polymerases include, but are not limited to Bacillus stearothermophilus pol I, Thermus aquaticus (Taq) pol I, Pyrccoccus furiosus (Pfu), Pyrococcus woesei (Pwo), Thermus flavus (Tfl), Thermus thermophilus (Tth), Thermus litoris (Tli) and Thermotoga maritime (Tma). These enzymes, modified versions of these enzymes, and combination of enzymes, are commercially available from vendors including Roche, Invitrogen, Qiagen, Stratagene, and Applied Biosystems. Representative enzymes include PHUSION^{®} (New England Biolabs, Ipswich, MA), Hot MasterTaq^{™} (Eppendorf), PHUSION^{®} Mpx (Finnzymes). PyroStart^{®} (Fermentas), KOD (EMD Biosciences), Z-Taq (TAKARA), and CS3AC/LA (KlenTaq, University City, MO).

Salts and buffers include those familiar to those skilled in the art, including those comprising MgCl₂, and Tris-HCl and KCl, respectively. Typically, 1.5-2.0nM of magnesium is optimal for Taq DNA polymerase, however, the optimal magnesium concentration may depend on template, buffer, DNA and dNTPs as each has the potential to chelate magnesium. If the concentration of magnesium [Mg²⁺] is too low, a PCR product may not form. If the concentration of magnesium [Mg²⁺] is too high, undesired PCR products may be seen. In some embodiments the magnesium concentration may be optimized by supplementing magnesium concentration in 0.1mM or 0.5mM increments up to about 5 mM.

Buffers used in accordance with the disclosure may contain additives such as surfactants, dimethyl sulfoxide (DMSO), glycerol, bovine serum albumin (BSA) and polyethylene glycol (PEG), as well as others familiar to those skilled in the art. Nucleotides are generally deoxyribonucleoside triphosphates, such as deoxyadenosine triphosphate (dATP), deoxycytidine triphosphate (dCTP), deoxyguanosine triphosphate (dGTP), and deoxythymidine triphosphate (dTTP), which are also added to a reaction adequate amount for amplification of the target nucleic acid. In some embodiments, the concentration of one or more dNTPs (e.g., dATP, dCTP, dGTP, dTTP) is from about 10 µM to about 500µM which may depend on the length and number of PCR products produced in a PCR reaction.

In some embodiments, the concentration of primers used in the PCR reaction may be modified or optimized. In some embodiments, the concentration of a primer (e.g., a forward or reverse primer) in a PCR reaction may be, for example, about 0.05 µM to about 1 µM. In particular embodiments, the concentration of each primer is about 1 nM to about 1 µM. It should be appreciated that the primers in accordance with the disclosure may be used at the same or different concentrations in a PCR reaction. For example, the forward primer of a primer pair may be used at a concentration of 0.5 µM and the reverse primer of the primer pair may be used at 0.1 uM. The concentration of the primer may be based on factors including, but not limited to, primer length, GC content, purity, mismatches with the target DNA or likelihood of forming primer dimers.

In some embodiments, the thermal profile of the PCR reaction is modified or optimized. Non-limiting examples of PCR thermal profile modifications include denaturation temperature and duration, annealing temperature and duration and extension time.

The temperature of the PCR reaction solutions may be sequentially cycled between a denaturing state, an annealing state, and an extension state for a predetermined number of cycles. The actual times and temperatures can be enzyme, primer, and target dependent. For any given reaction, denaturing states can range in certain embodiments from about 70 °C to about 100 °C. In addition, the annealing temperature and time can influence the specificity and efficiency of primer binding to a particular locus within a target nucleic acid and may be important for particular PCR reactions. For any given reaction, annealing states can range in certain embodiments from about 20 °C to about 75 °C. In some embodiments, the annealing state can be from about 46 °C to 64°C. In certain embodiments, the annealing state can be performed at room temperature (e.g., from about 20 °C to about 25 °C).

Extension temperature and time may also impact the allele product yield. For a given enzyme, extension states can range in certain embodiments from about 60 °C to about 75 °C.

Quantification of the amounts of the alleles from a PCR assay can be performed as provided herein or as otherwise would be apparent to one of ordinary skill in the art. As an example, amplification traces are analyzed for consistency and robust quantification. Internal standards may be used to translate the cycle threshold to amount of input nucleic acids (e.g., DNA). The amounts of alleles can be computed as the mean of performant assays and can be adjusted for genotype.

Any one of the methods provided herein can comprise extracting nucleic acids, such as cell-free DNA (e.g., donor-specific cell-free DNA), from a sample obtained from a subject. Such extraction can be done using any method known in the art or as otherwise provided herein (see, e.g., Current Protocols in Molecular Biology, latest edition, or the QIAamp circulating nucleic acid kit or other appropriate commercially available kits). An exemplary method for isolating cell-free DNA from blood is described. Blood containing an anticoagulant such as EDTA or DTA is collected from a subject. The plasma, which contains cf-DNA, is separated from cells present in the blood (e.g., by centrifugation or filtering). An optional secondary separation may be performed to remove any remaining cells from the plasma (e.g., a second centrifugation or filtering step). The cf-DNA can then be extracted using any method known in the art, e.g., using a commercial kit such as those produced by Qiagen. Other exemplary methods for extracting cf-DNA are also known in the art (see, e.g., Cell-Free Plasma DNA as a Predictor of Outcome in Severe Sepsis and Septic Shock. Clin. Chem. 2008, v. 54, p. 1000-1007; Prediction of MYCN Amplification in Neuroblastoma Using Serum DNA and Real-Time Quantitative Polymerase Chain Reaction. JCO 2005, v. 23, p.5205-5210; Circulating Nucleic Acids in Blood of Healthy Male and Female Donors. Clin. Chem. 2005, v. 51, p.1317-1319; Use of Magnetic Beads for Plasma Cell-free DNA Extraction: Toward Automation of Plasma DNA Analysis for Molecular Diagnostics. Clin. Chem. 2003, v. 49, p. 1953-1955; Chiu RWK, Poon LLM, Lau TK, Leung TN, Wong EMC, Lo YMD. Effects of blood-processing protocols on fetal and total DNA quantification in maternal plasma. Clin Chem 2001;47:1607-1613; and Swinkels et al. Effects of Blood-Processing Protocols on Cell-free DNA Quantification in Plasma. Clinical Chemistry, 2003, vol. 49, no. 3, 525-526).

In some embodiments of any one of the methods provided herein, a pre-amplification step is performed. An exemplary method of such an amplification is as follows, and such a method can be included in any one of the methods provided herein. Approximately 15 ng of cell-free plasma DNA is amplified in a PCR using Q5 DNA polymerase with approximately 13 targets where pooled primers were at 4uM total. Samples undergo approximately 25 cycles. Reactions are in 25 ul total. After amplification, samples can be cleaned up using several approaches including AMPURE bead cleanup, bead purification, or simply ExoSAP-IT^{™}, or Zymo.

As used herein, the sample from a subject can be a biological sample. Examples of such biological samples include whole blood, plasma, serum, urine, etc. In some embodiments, addition of further nucleic acids, e.g., a standard, to the sample can be performed.

In another aspect, compositions and kits comprising one or more primer pairs as provided herein are provided. Other reagents for performing an assay, such as a PCR assay, may also be included in the composition or kit.

Various aspects of the present invention may be used alone, in combination, or in a variety of arrangements not specifically discussed in the embodiments described in the foregoing and are therefore not limited in their application to the details and arrangement of components set forth in the foregoing description or illustrated in the drawings. For example, aspects described in one embodiment may be combined in any manner with aspects described in other embodiments.

Also, embodiments of the invention may be implemented as one or more methods, of which an example has been provided. The acts performed as part of the method(s) may be ordered in any suitable way. Accordingly, embodiments may be constructed in which acts are performed in an order different from illustrated, which may include performing some acts simultaneously, even though shown as sequential acts in illustrative embodiments.

Use of ordinal terms such as "first," "second," "third," etc., in the claims to modify a claim element does not by itself connote any priority, precedence, or order of one claim element over another or the temporal order in which acts of a method are performed. Such terms are used merely as labels to distinguish one claim element having a certain name from another element having a same name (but for use of the ordinal term).

The phraseology and terminology used herein is for the purpose of description and should not be regarded as limiting. The use of "including," "comprising," "having," "containing", "involving", and variations thereof, is meant to encompass the items listed thereafter and additional items.

The following description provides examples of the methods provided herein.

### EXAMPLES

### Example 1 - MOMA Assay With Recipient and Donor Genotype Information

### SNV Target Selection

Identification of targets for multiplexing in accordance with the disclosure may include one or more of the following steps. First, highly heterozygous SNPs were screened on several ethnic control populations (Hardy-Weinberg p > 0.25), excluding known difficult regions. Difficult regions include syndromic regions likely to be abnormal in patients and regions of low complexity, including centromeres and telomeres of chromosomes. Target fragments of desired lengths were then designed *in silico.* Specifically, two 20-26 bp primers spanning each SNP's 70 bp window were designed. All candidate primers were then queried to GCRh37 using BLAST. Those primers that were found to be sufficiently specific were retained, and monitored for off-target hits, particularly at the 3' end of the fragment. The off-target candidate hits were analyzed for pairwise fragment generation that would survive size selection. Selected primers were then subjected to an *in silico* multiplexing evaluation. The primers' computed melting temperatures and guanine-cytosine percentages (GC%) were used to filter for moderate range sequences. An iterated genetic algorithm and simulated annealing were used to select candidate primers compatible for 400 targets, ultimately resulting in the selection of 800 primers. The 800 primers were generated and physically tested for multiplex capabilities at a common melting temperature in a common solution. Specifically, primers were filtered based on even amplification in the multiplex screen and moderate read depth window. Forty-eight assays were designed for MOMA using the top performing multiplexed SNPs. Each SNP had a probe designed in WT/MUT at four mismatch choices; there were eight probes per assay. The new nested primers were designed within the 70 bp enriched fragments. Finally, the primers were experimentally amplified with known heterozygous individuals to evaluate amplification efficiency (8 probes x 48 assays in triplicate, using TAQMAN^{™}).

### A priori Genotyping Informativeness of each Assay

Using the known recipient and donor genotypes at each assayed SNP, a subset of informative assays was selected. Note that recipient homozygous sites can be used where the donor is any other genotype. Additionally, if the donor genotype is not known, it can be inferred, such as by using plasma data discrepancies. Genotypes may also be learned through sequencing, SNP microarray, or application of a MOMA assay on known 0% (clean recipient) samples.

### Post Processing Analysis of Multiplex Assay Performance

Patient-specific MOMA probe biases were estimated across the experimental cohort. Selection iteratively was refined to make the final donor percent call. Further, automatic outlier detection provided patient-specific anomalous genomic regions.

### Reconstruction Experiment

The sensitivity and precision of the assay were evaluated using reconstructed plasma samples with known mixing ratios. Specifically, the ratios of 1:10, 1:20, 1:100, 1:200, and 1:1000 were evaluated.

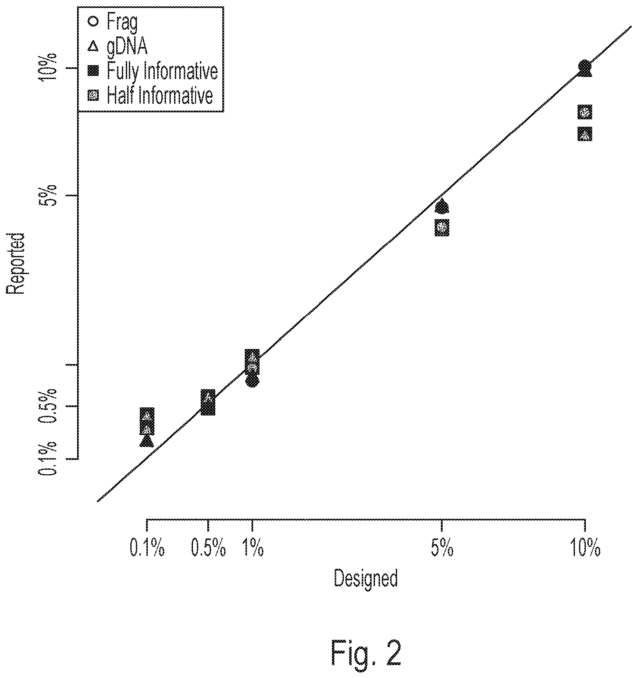

Results of the reconstruction experiment are shown in **Fig. 2****.** One target is fully informative where there is a homozygous donor against a homozygous recipient (shaded data points). The other target is half informative where there is a heterozygous donor against a homozygous recipient (open data points).

### Example 2 - MOMA Assay with Recipient but not Donor Genotype Information

To work without donor genotype information, the following procedure may be performed to infer informative assays and allow for quantification of donor-specific cell-free DNA in plasma samples. All assays were evaluated for performance in the full information scenario. This procedure thus assumed clean AA/AB/BB genotypes at each assay and unbiased behavior of each quantification. With recipient genotype, assays known to be homozygous in the recipient were selected. Any contamination was attributed to the donor nucleic acids, and the assay collection created a tri-modal distribution with three clusters of assays corresponding to the non-, half, and fully-informative assays. With sufficient numbers of recipient homozygous assays, the presence of donor fully informative assays can be assumed.

If the recipient genotype is homozygous and known, then if a measurement that is not the recipient genotype is observed, the probes which are truly donor-homozygous will have the highest cluster and equal the guess whereas those that are donor heterozygous will be at half the guess. A probability distribution can be plotted and an expectation maximization algorithm (EM) can be employed to infer donor genotype. Such can be used to infer the donor genotype frequency in any one of the methods provided herein. Accordingly, an EM algorithm was used to infer the most likely donor genotypes at all assayed SNV targets. With inferred donor genotypes, quantification may proceed as in the full-information scenario. EM can begin with the assumption that the minor allele ratio found at an assay follows a tri-modal distribution, one for each combination of recipient and donor, given all assays are "AA" in the recipient (or flipped from "BB" without loss of generality). With all donor genotypes unknown, it is possible to bootstrap from the knowledge that any assays exhibiting nearly zero minor allele are donor AA, and the highest is donor BB. Initial guesses for all donor genotypes were recorded, and the mean of each cluster calculated. Enforcing that the donor BB assays' mean is twice that of the donor AB restricts the search. The algorithm then reassigns guessed donor genotypes based on the clusters and built-in assumptions. The process was iterative until no more changes were made. The final result is a set of the most likely donor genotypes given their measured divergence from the background. Generally, every target falls into the model; a result may be tossed if between groups after maximization.

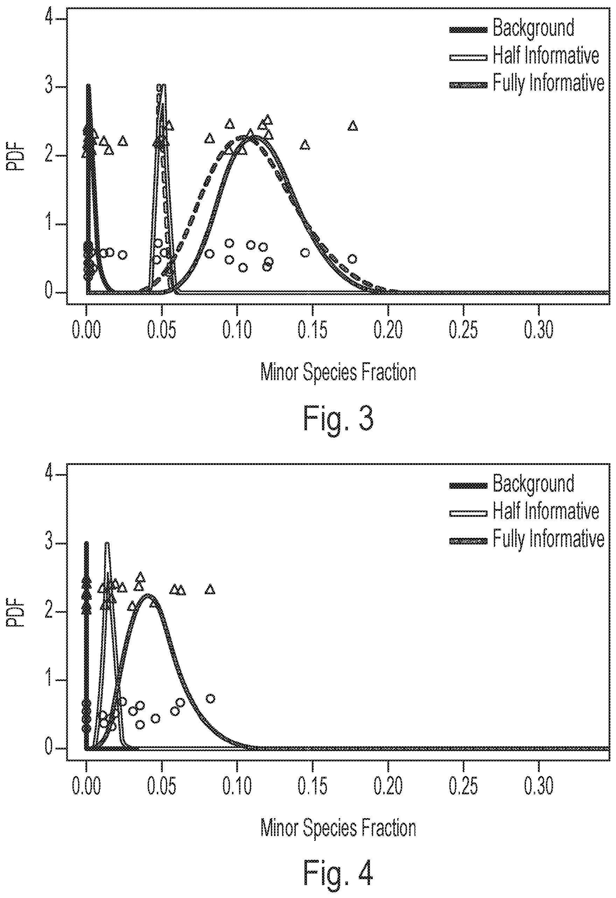

**Figs. 3** and **4** show exemplary results from plasma samples handled in this manner. The x-axis is the donor% for any assay found recipient homozygous. The rows of points represent individual PCR assay results. The bottom-most row of circles represents the initial guess of donor genotypes, some AA, some A/B and some BB. Then the solid curves were drawn representing beta distributions centered on the initial assays, spotted for homozygous (fully informative) and white for heterozygous (half informative) with black curves representing the distribution of non-informative assays or background noise. The assays were re-assigned updated guesses in the second row. The second row's curves use dashed lines. The top row is the final estimate because no change occurred. Double the peak of the white dashed curve corresponds to the maximum likelihood donor% call, at around 10%, or equal to the mean of the dotted curve.

A reconstruction experiment (Recon1) using DNA from two individuals was created at 10%, 5%, 1%, 0.5%, and 0.1%. All mixes were amplified with a multiplex library of targets, cleaned, then quantitatively genotyped using a MOMA method. The analysis was performed with genotyping each individual in order to know their true genotypes. Informative targets were determined using prior knowledge of the genotype of the major individual (looking for homozygous sites), and where the second individual was different, and used to calculate fractions (percentage) using informative targets. The fractions were then calculated (depicted in black to denote "With Genotype" information).

A second reconstruction experiment (Recon2), beginning with two individuals, major and minor, was also created at 10%, 5%, 1%, 0.5%, and 0.1%. All mixes were amplified with the multiplex library of targets, cleaned, and then quantitatively genotyped using a MOMA method. The analysis was performed by genotyping each individual in order to know their true genotypes. Informative targets were determined using prior knowledge of the genotype of the second individual as described above. The fractions were then calculated (depicted in black to denote "With Genotype" information).

These reconstructions were run again the next day (Recon3).

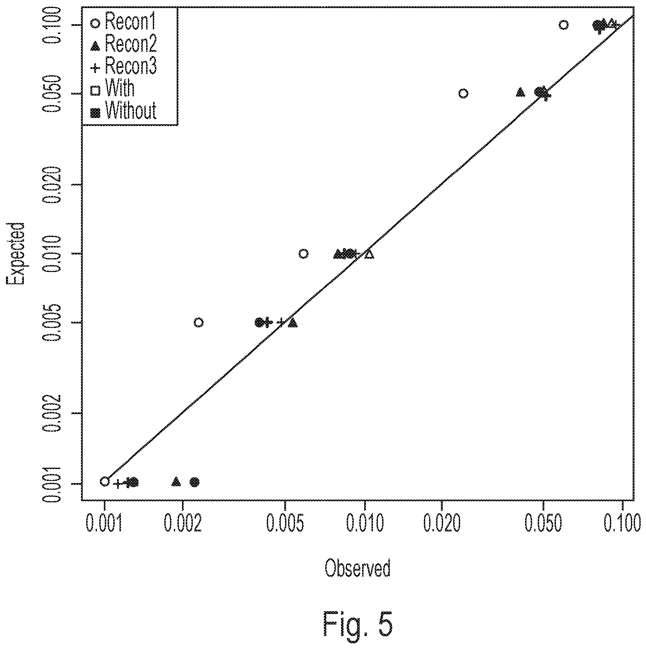

The same reconstruction samples (Recon 1,2,3) were then analyzed again only using the genotyping information available for the first individual (major DNA contributor). Genotyping information from the second individual (minor DNA contributor) was not used. Approximately 38-40 targets were used to calculate fractions without genotyping (simulating without donor); they are presented as shaded points **(****Fig. 5****).** It was found that each target that was recipient homozygous was generally useful. The circles show a first estimate, a thresholding; those on the right were thought to be fully informative and those on the left, not. The triangles along the top were the same targets, but for the final informativity decisions they were recolored.

### Example 3 - MOMA cf-DNA Assay

### Principles and Procedures of a MOMA cfDNA Assay

This exemplary assay is designed to determine the percentage of DS cf-DNA present in a transplant recipient's blood sample. In this embodiment, the recipient's blood sample is collected in an EDTA tube and centrifuged to separate the plasma and buffy coat. The plasma and buffy coat can be aliquoted into two separate 15 mL conical tubes and frozen. The plasma sample can be used for quantitative genotyping (qGT), while the buffy coat can be used for basic genotyping (bGT) of the recipient. In addition to the transplant recipient's blood sample, a small piece of discarded tissue or blood sample from the donor can be used for basic genotyping.

The first step in the process can be to extract cell free DNA from the plasma sample (used for qGT) and genomic DNA (gDNA) from the buffy coat, whole blood, or tissue sample (used for bGT). The total amount of cfDNA can be determined by qPCR and normalized to a target concentration. This process is known as a cfDNA Quantification. gDNA can be quantified using UV-spectrophotometry and normalized. Fifteen ng of DNA generally provides accurate and valid results.

The normalized patient DNA can be used as an input into a highly-multiplexed library PCR amplification reaction containing, for example, 96 primer pairs, each of which amplify a region including one of the MOMA target sites. The resulting library can be used as the input for either the bGT or qGT assay as it consists of PCR amplicons having the MOMA target primer and probe sites. This step can improve the sensitivity of the overall assay by increasing the copy number of each target prior to the highly-specific qPCR amplification. Controls and calibrators/standards can be amplified with the multiplex library alongside patient samples. Following the library amplification, an enzymatic cleanup can be performed to remove excess primers and unincorporated deoxynucleotide triphosphates (dNTPs) to prevent interference with the downstream amplification.

In a parallel workflow the master mixes can be prepared and transferred to a 384-well PCR plate. The amplified samples, controls, and calibrators/standards can then be diluted with the library dilution buffer to a predetermined volume and concentration. The diluted samples and controls can be aliquoted to a 6-well reservoir plate and transferred to the 384-well PCR plate using an acoustic liquid handler. The plate can then be sealed and moved to a real-time PCR amplification and detection system.

MOMA can perform both the basic and quantitative genotyping analyses by targeting biallelic SNPs that are likely to be distinct between a transplant donor and recipient making them highly informative. The basic genotyping analysis can label the recipient and donor with three possible genotypes at each target (e.g. homozygous REF, heterozygous REF and VAR, and homozygous VAR). This information can be used for the quantitative genotyping analysis, along with standard curves, to quantitate to the allele ratio for each target, known as a minor-species proportion. The median of all informative and quality-control passed allele ratios can be used to determine the % of DS cfDNA.

### Example 4 - Examples of Computer-Implemented Embodiments

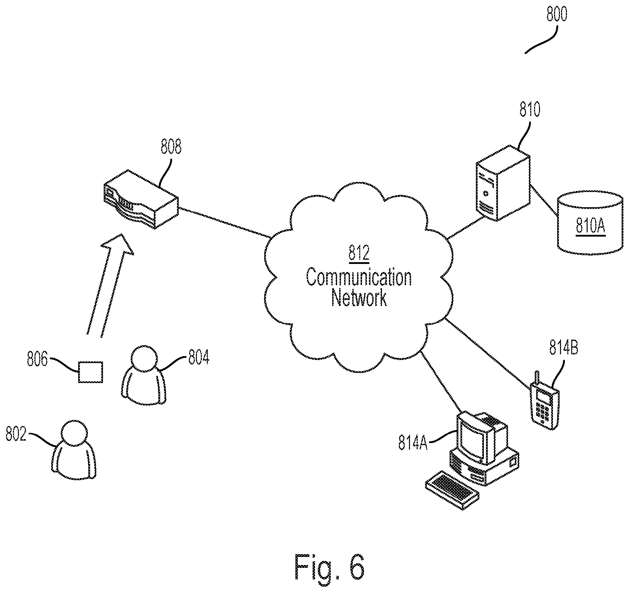

In some embodiments, the diagnostic techniques described above may be implemented via one or more computing devices executing one or more software facilities to analyze samples for a subject, such as over time, measure nucleic acids (such as cell-free DNA) in the samples, and produce a result, such as a diagnostic result, based on one or more of the samples. **Fig. 6** illustrates an example of a computer system with which some embodiments may operate, though it should be appreciated that embodiments are not limited to operating with a system of the type illustrated in **Fig. 6****.**

The computer system of Fig. **6** includes a subject 802 and a clinician 804 that may obtain a sample 806 from the subject 806. As should be appreciated from the foregoing, the sample 806 may be any suitable sample of biological material for the subject 802 that may be used to measure the presence of nucleic acids (such as cell-free DNA) in the subject 802, including a blood sample. The sample 806 may be provided to an analysis device 808, which one of ordinary skill will appreciate from the foregoing will analyze the sample 808 so as to determine (including estimate) amounts of nucleic acids (such as cell-free DNA), including amounts of DS nucleic acids (such as DS cell-free DNA) in the sample 806 and/or the subject 802. For ease of illustration, the analysis device 808 is depicted as single device, but it should be appreciated that analysis device 808 may take any suitable form and may, in some embodiments, be implemented as multiple devices. To determine the amounts of nucleic acids (such as cell-free DNA) in the sample 806 and/or subject 802, the analysis device 808 may perform any of the techniques described above, and is not limited to performing any particular analysis. The analysis device 808 may include one or more processors to execute an analysis facility implemented in software, which may drive the processor(s) to operate other hardware and receive the results of tasks performed by the other hardware to determine on overall result of the analysis, which may be the amounts of nucleic acids (such as cell-free DNA) in the sample 806 and/or the subject 802. The analysis facility may be stored in one or more computer-readable storage media, such as a memory of the device 808. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

In some embodiments, the clinician 804 may directly provide the sample 806 to the analysis device 808 and may operate the device 808 in addition to obtaining the sample 806 from the subject 802, while in other embodiments the device 808 may be located geographically remote from the clinician 804 and subject 802 and the sample 806 may need to be shipped or otherwise transferred to a location of the analysis device 808. The sample 806 may in some embodiments be provided to the analysis device 808 together with (e.g., input via any suitable interface) an identifier for the sample 806 and/or the subject 802, for a date and/or time at which the sample 806 was obtained, or other information describing or identifying the sample 806.

The analysis device 808 may in some embodiments be configured to provide a result of the analysis performed on the sample 806 to a computing device 810, which may include a data store 810A that may be implemented as a database or other suitable data store. The computing device 810 may in some embodiments be implemented as one or more servers, including as one or more physical and/or virtual machines of a distributed computing platform such as a cloud service provider. In other embodiments, the device 810 may be implemented as a desktop or laptop personal computer, a smart mobile phone, a tablet computer, a special-purpose hardware device, or other computing device.

In some embodiments, the analysis device 808 may communicate the result of its analysis to the device 810 via one or more wired and/or wireless, local and/or wide-area computer communication networks, including the Internet. The result of the analysis may be communicated using any suitable protocol and may be communicated together with the information describing or identifying the sample 806, such as an identifier for the sample 806 and/or subject 802 or a date and/or time the sample 806 was obtained.

The computing device 810 may include one or more processors to execute a diagnostic facility implemented in software, which may drive the processor(s) to perform diagnostic techniques described herein. The diagnostic facility may be stored in one or more computer-readable storage media, such as a memory of the device 810. In other embodiments, techniques described herein for analyzing a sample may be partially or entirely implemented in one or more special-purpose computer components such as Application Specific Integrated Circuits (ASICs), or through any other suitable form of computer component that may take the place of a software implementation.

The diagnostic facility may receive the result of the analysis and the information describing or identifying the sample 806 and may store that information in the data store 810A. The information may be stored in the data store 810A in association with other information for the subject 802, such as in a case that information regarding prior samples for the subject 802 was previously received and stored by the diagnostic facility. The information regarding multiple samples may be associated using a common identifier, such as an identifier for the subject 802. In some cases, the data store 810A may include information for multiple different subjects.

The diagnostic facility may also be operated to analyze results of the analysis of one or more samples 806 for a particular subject 802, identified by user input, so as to determine a diagnosis for the subject 802. The diagnosis may be a conclusion of a risk that the subject 802 has, may have, or may in the future develop a particular condition. The diagnostic facility may determine the diagnosis using any of the various examples described above, including by comparing the amounts of nucleic acids (such as cell-free DNA) determined for a particular sample 806 to one or more thresholds or by comparing a change over time in the amounts of nucleic acids (such as cell-free DNA) determined for samples 806 over time, such as to one or more thresholds. For example, the diagnostic facility may determine a risk to the subject 802 of a condition by comparing an amount of nucleic acids (such as cell-free DNA) for one or more samples 806 to one threshold and comparing an amount of nucleic acids (such as cell-free DNA) for the same sample(s) 806 to another threshold. Based on the comparisons to the thresholds, the diagnostic facility may produce an output indicative of a risk to the subject 802 of a condition.

As should be appreciated from the foregoing, in some embodiments, the diagnostic facility may be configured with different thresholds to which amounts of nucleic acids (such as cell-free DNA) may be compared. The different thresholds may, for example, correspond to different demographic groups (age, gender, race, economic class, presence or absence of a particular procedure/condition/other in medical history, or other demographic categories), different conditions, and/or other parameters or combinations of parameters. In such embodiments, the diagnostic facility may be configured to select thresholds against which amounts of nucleic acids (such as cell-free DNA) are to be compared, with different thresholds stored in memory of the computing device 810. The selection may thus be based on demographic information for the subject 802 in embodiments in which thresholds differ based on demographic group, and in these cases demographic information for the subject 802 may be provided to the diagnostic facility or retrieved (from another computing device, or a data store that may be the same or different from the data store 810A, or from any other suitable source) by the diagnostic facility using an identifier for the subject 802. The selection may additionally or alternatively be based on the condition for which a risk is to be determined, and the diagnostic facility may prior to determining the risk receive as input a condition and use the condition to select the thresholds on which to base the determination of risk. It should be appreciated that the diagnostic facility is not limited to selecting thresholds in any particular manner, in embodiments in which multiple thresholds are supported.

In some embodiments, the diagnostic facility may be configured to output for presentation to a user a user interface that includes a diagnosis of a risk and/or a basis for the diagnosis for a subject 802. The basis for the diagnosis may include, for example, amounts of nucleic acids (such as cell-free DNA) detected in one or more samples 806 for a subject 802. In some embodiments, user interfaces may include any of the examples of results, values, amounts, graphs, etc. discussed above. They can include results, values, amounts, etc. over time. For example, in some embodiments, a user interface may incorporate a graph similar to that shown in any one of the figures provided herein. In such a case, in some cases the graph may be annotated to indicate to a user how different regions of the graph may correspond to different diagnoses that may be produced from an analysis of data displayed in the graph. For example, thresholds against which the graphed data may be compared to determine the analysis may be imposed on the graph(s).

A user interface including a graph, particularly with the lines and/or shading, may provide a user with a far more intuitive and faster-to-review interface to determine a risk of the subject 802 based on amounts of nucleic acids (such as cell-free DNA), than may be provided through other user interfaces. It should be appreciated, however, that embodiments are not limited to being implemented with any particular user interface.

In some embodiments, the diagnostic facility may output the diagnosis or a user interface to one or more other computing devices 814 (including devices 814A, 814B) that may be operated by the subject 802 and/or a clinician, which may be the clinician 804 or another clinician. The diagnostic facility may transmit the diagnosis and/or user interface to the device 814 via the network(s) 812.

Techniques operating according to the principles described herein may be implemented in any suitable manner. Included in the discussion above are a series of flow charts showing the steps and acts of various processes that determine a risk of a condition based on an analysis of amounts of nucleic acids (such as cell-free DNA). The processing and decision blocks discussed above represent steps and acts that may be included in algorithms that carry out these various processes. Algorithms derived from these processes may be implemented as software integrated with and directing the operation of one or more single- or multi-purpose processors, may be implemented as functionally-equivalent circuits such as a Digital Signal Processing (DSP) circuit or an Application-Specific Integrated Circuit (ASIC), or may be implemented in any other suitable manner. It should be appreciated that embodiments are not limited to any particular syntax or operation of any particular circuit or of any particular programming language or type of programming language. Rather, one skilled in the art may use the description above to fabricate circuits or to implement computer software algorithms to perform the processing of a particular apparatus carrying out the types of techniques described herein. It should also be appreciated that, unless otherwise indicated herein, the particular sequence of steps and/or acts described above is merely illustrative of the algorithms that may be implemented and can be varied in implementations and embodiments of the principles described herein.

Accordingly, in some embodiments, the techniques described herein may be embodied in computer-executable instructions implemented as software, including as application software, system software, firmware, middleware, embedded code, or any other suitable type of computer code. Such computer-executable instructions may be written using any of a number of suitable programming languages and/or programming or scripting tools, and also may be compiled as executable machine language code or intermediate code that is executed on a framework or virtual machine.

When techniques described herein are embodied as computer-executable instructions, these computer-executable instructions may be implemented in any suitable manner, including as a number of functional facilities, each providing one or more operations to complete execution of algorithms operating according to these techniques. A "functional facility," however instantiated, is a structural component of a computer system that, when integrated with and executed by one or more computers, causes the one or more computers to perform a specific operational role. A functional facility may be a portion of or an entire software element. For example, a functional facility may be implemented as a function of a process, or as a discrete process, or as any other suitable unit of processing. If techniques described herein are implemented as multiple functional facilities, each functional facility may be implemented in its own way; all need not be implemented the same way. Additionally, these functional facilities may be executed in parallel and/or serially, as appropriate, and may pass information between one another using a shared memory on the computer(s) on which they are executing, using a message passing protocol, or in any other suitable way.

Generally, functional facilities include routines, programs, objects, components, data structures, etc. that perform particular tasks or implement particular abstract data types. Typically, the functionality of the functional facilities may be combined or distributed as desired in the systems in which they operate. In some implementations, one or more functional facilities carrying out techniques herein may together form a complete software package. These functional facilities may, in alternative embodiments, be adapted to interact with other, unrelated functional facilities and/or processes, to implement a software program application.

Some exemplary functional facilities have been described herein for carrying out one or more tasks. It should be appreciated, though, that the functional facilities and division of tasks described is merely illustrative of the type of functional facilities that may implement the exemplary techniques described herein, and that embodiments are not limited to being implemented in any specific number, division, or type of functional facilities. In some implementations, all functionality may be implemented in a single functional facility. It should also be appreciated that, in some implementations, some of the functional facilities described herein may be implemented together with or separately from others (i.e., as a single unit or separate units), or some of these functional facilities may not be implemented.

Computer-executable instructions implementing the techniques described herein (when implemented as one or more functional facilities or in any other manner) may, in some embodiments, be encoded on one or more computer-readable media to provide functionality to the media. Computer-readable media include magnetic media such as a hard disk drive, optical media such as a Compact Disk (CD) or a Digital Versatile Disk (DVD), a persistent or non-persistent solid-state memory (e.g., Flash memory, Magnetic RAM, etc.), or any other suitable storage media. Such a computer-readable medium may be implemented in any suitable manner, including as a portion of a computing device or as a stand-alone, separate storage medium. As used herein, "computer-readable media" (also called "computer-readable storage media") refers to tangible storage media. Tangible storage media are non-transitory and have at least one physical, structural component. In a "computer-readable medium," as used herein, at least one physical, structural component has at least one physical property that may be altered in some way during a process of creating the medium with embedded information, a process of recording information thereon, or any other process of encoding the medium with information. For example, a magnetization state of a portion of a physical structure of a computer-readable medium may be altered during a recording process.

In some, but not all, implementations in which the techniques may be embodied as computer-executable instructions, these instructions may be executed on one or more suitable computing device(s) operating in any suitable computer system, including the exemplary computer system of **Fig. 6****,** or one or more computing devices (or one or more processors of one or more computing devices) may be programmed to execute the computer-executable instructions. A computing device or processor may be programmed to execute instructions when the instructions are stored in a manner accessible to the computing device or processor, such as in a data store (e.g., an on-chip cache or instruction register, a computer-readable storage medium accessible via a bus, etc.). Functional facilities comprising these computer-executable instructions may be integrated with and direct the operation of a single multi-purpose programmable digital computing device, a coordinated system of two or more multi-purpose computing device sharing processing power and jointly carrying out the techniques described herein, a single computing device or coordinated system of computing device (co-located or geographically distributed) dedicated to executing the techniques described herein, one or more Field-Programmable Gate Arrays (FPGAs) for carrying out the techniques described herein, or any other suitable system.

Embodiments have been described where the techniques are implemented in circuitry and/or computer-executable instructions. It should be appreciated that some embodiments may be in the form of a method, of which at least one example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments. Any one of the aforementioned, including the aforementioned devices, systems, embodiments, methods, techniques, algorithms, media, hardware, software, interfaces, processors, displays, networks, inputs, outputs or any combination thereof are provided herein in other aspects.

### Example 5 - Donor-specific Cell-free DNA (DS cf-DNA) Correlation with Cellular Rejection Grade

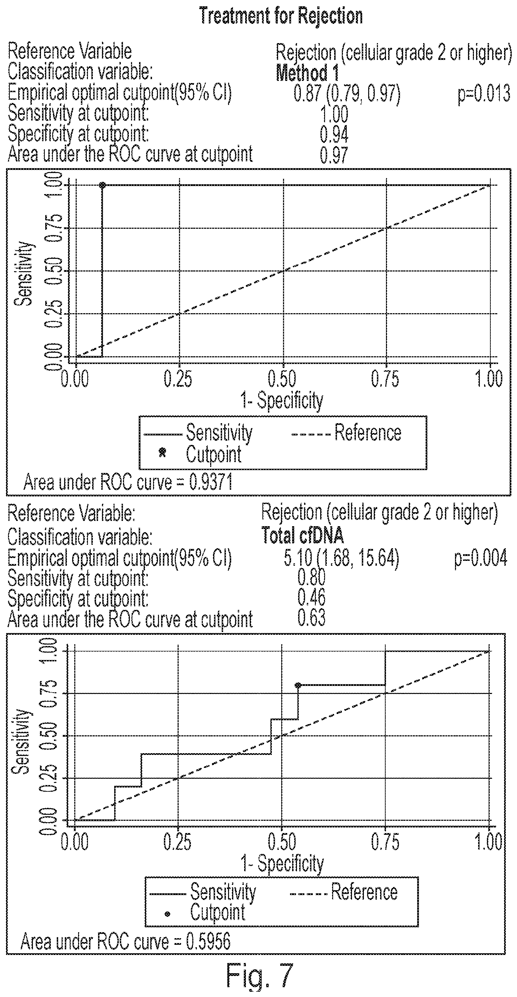

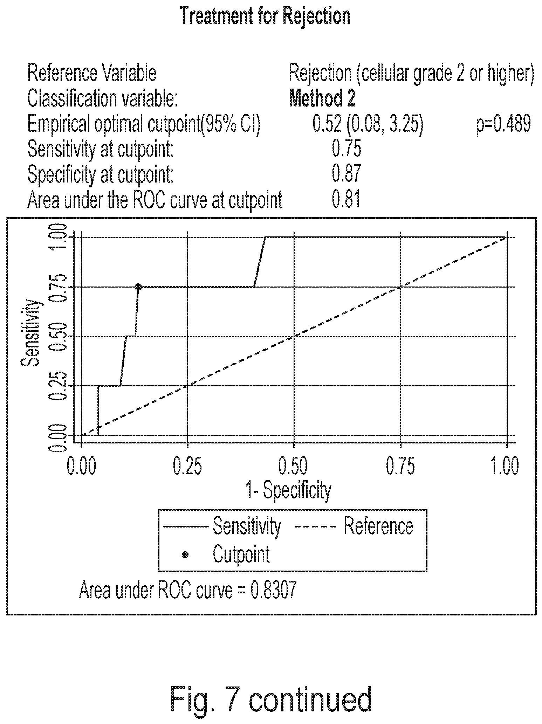

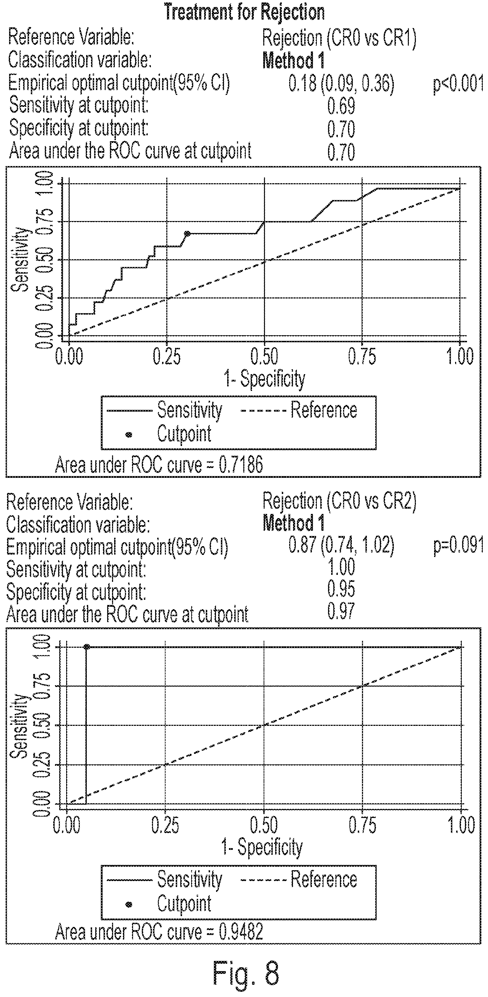

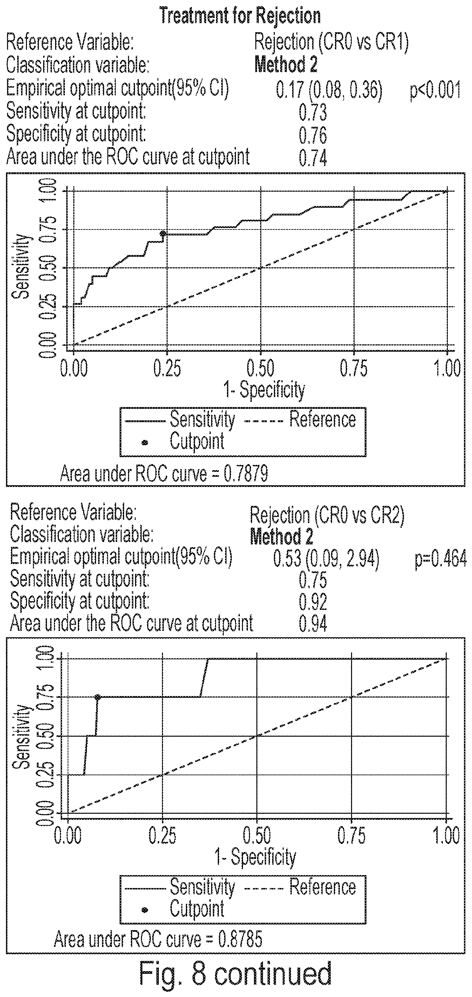

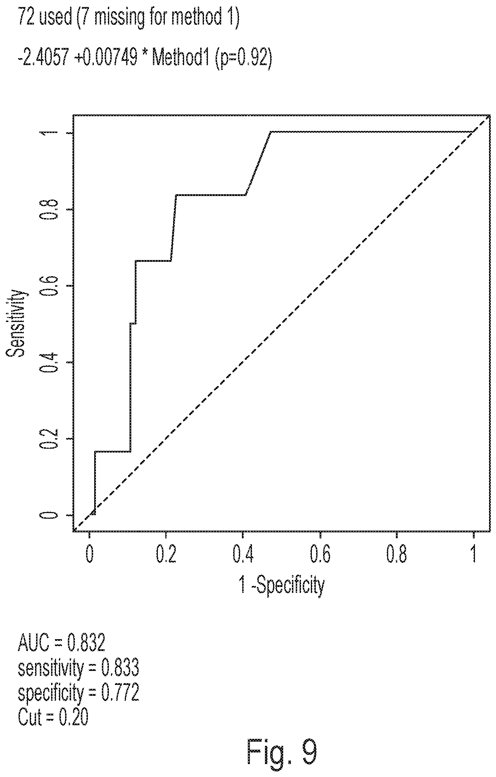

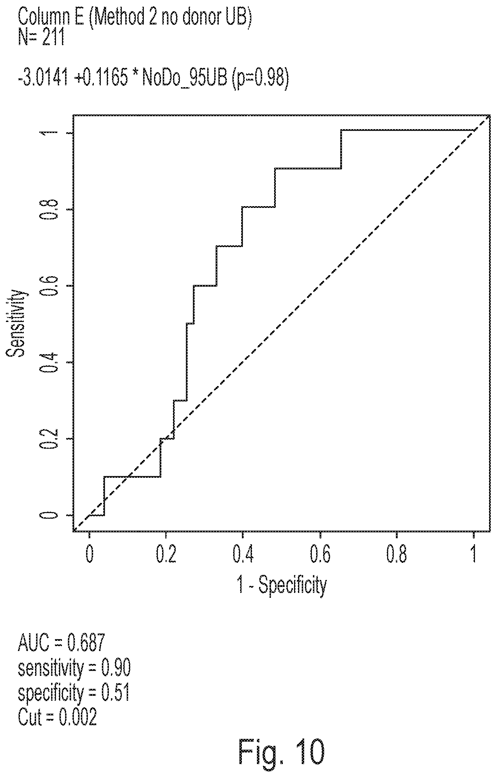

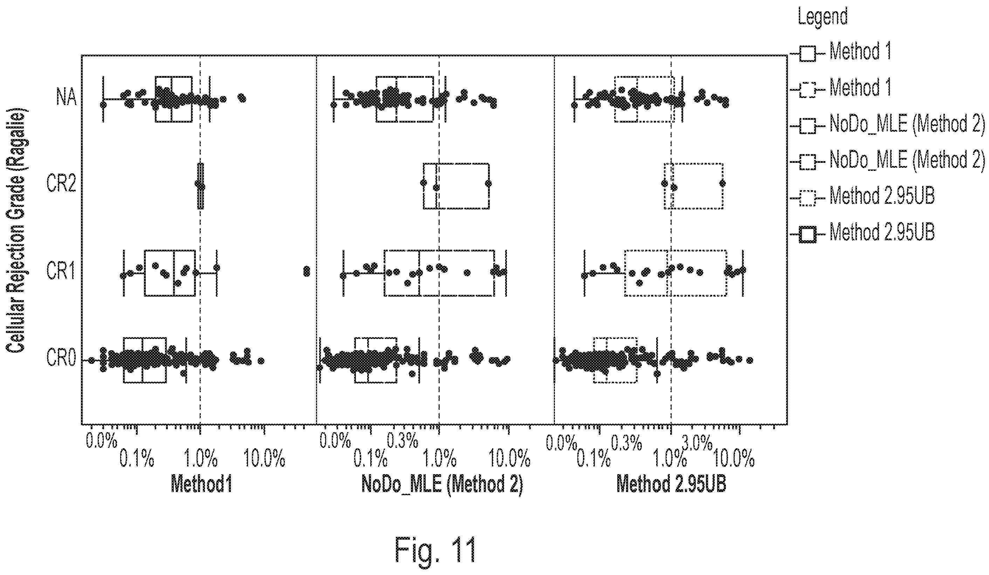

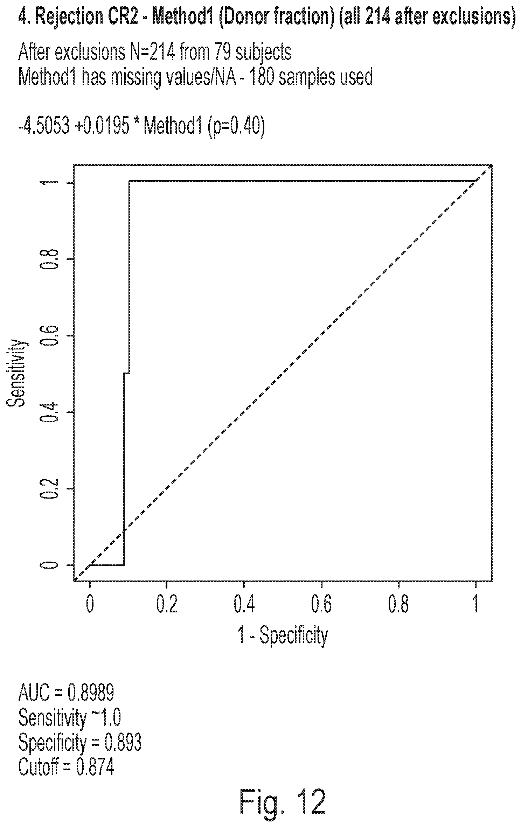

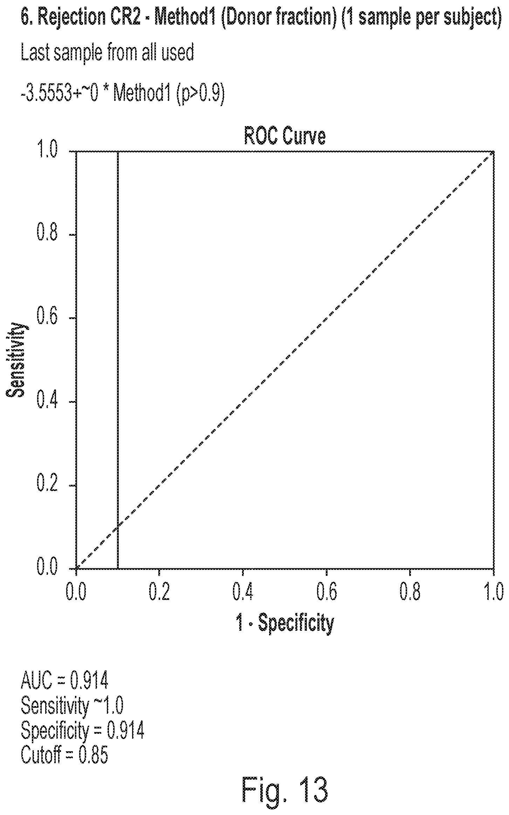

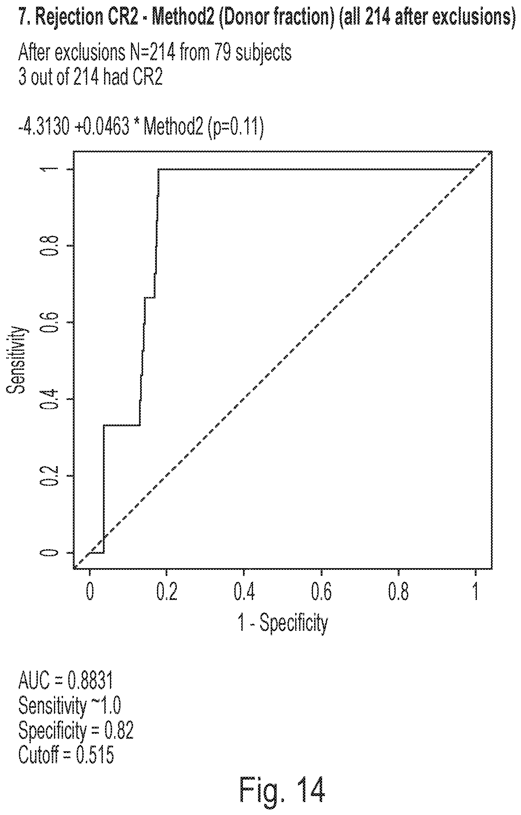

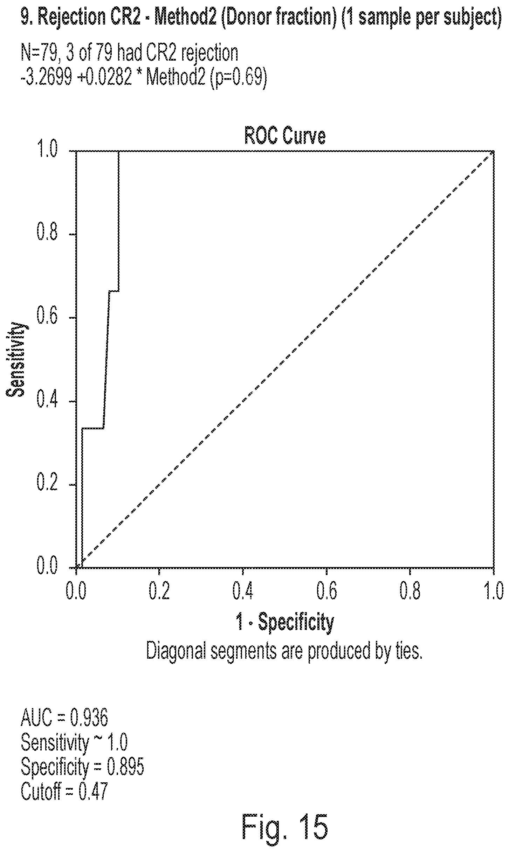

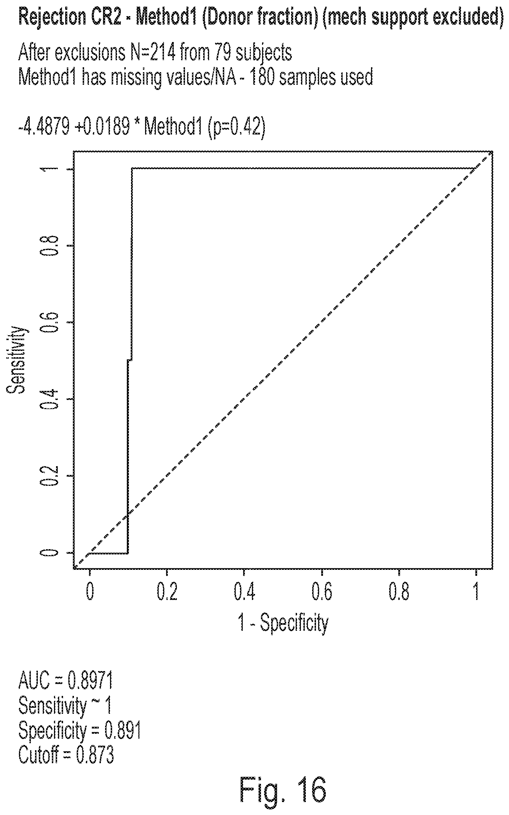

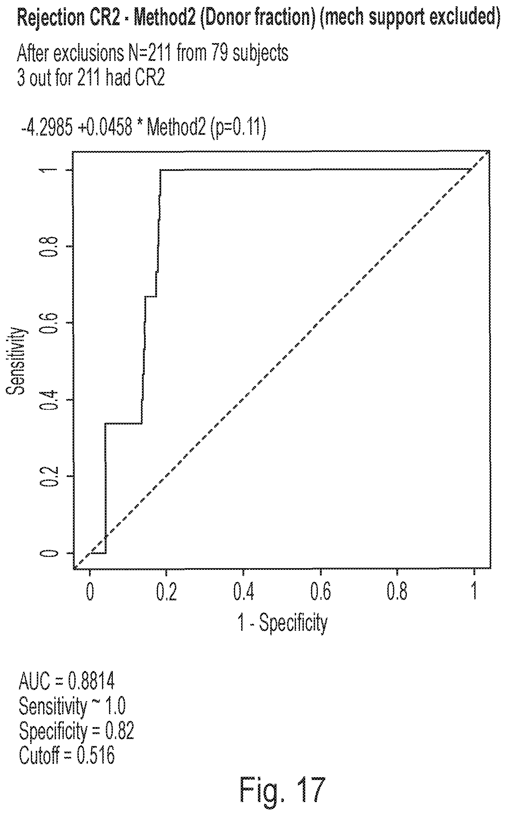

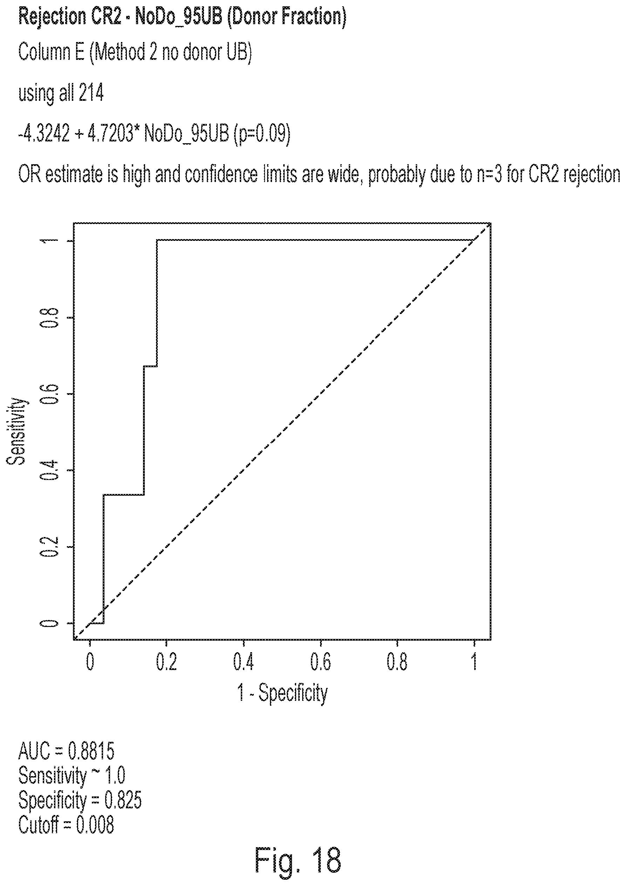

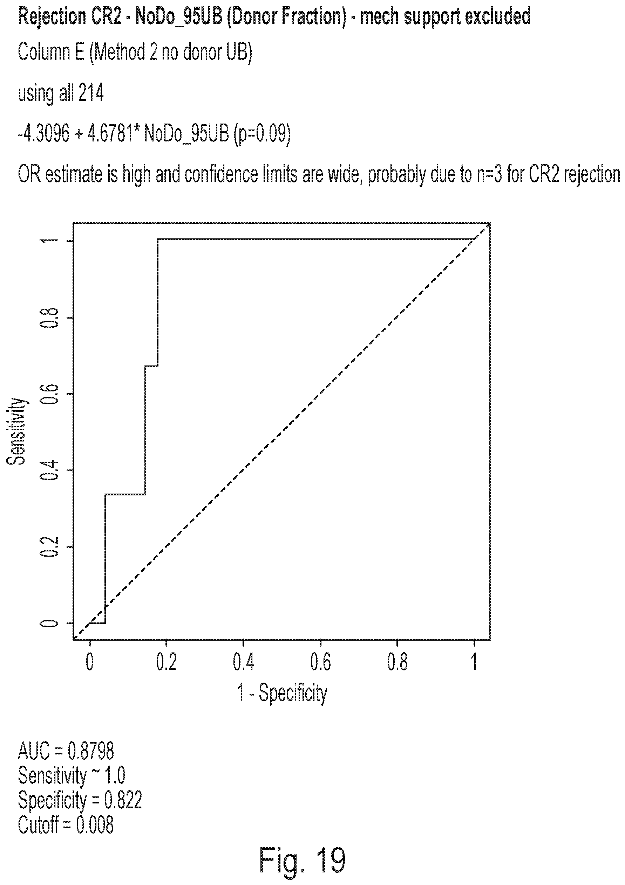

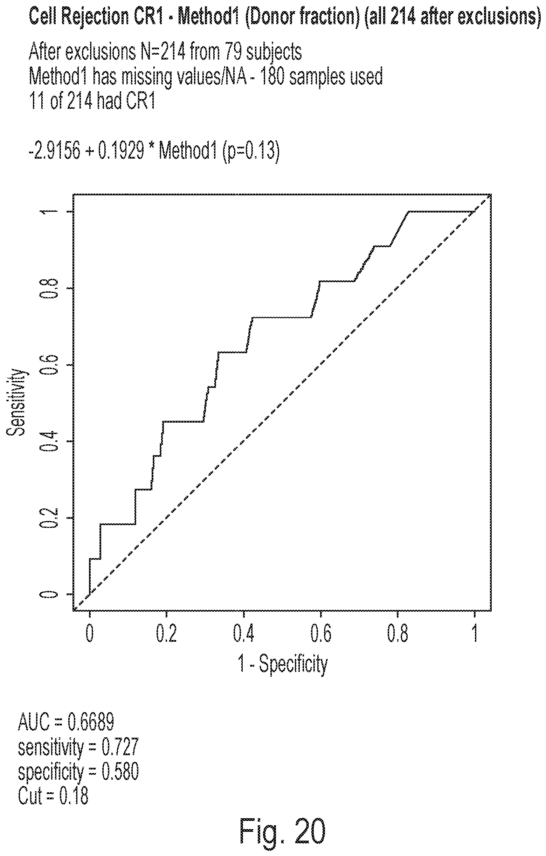

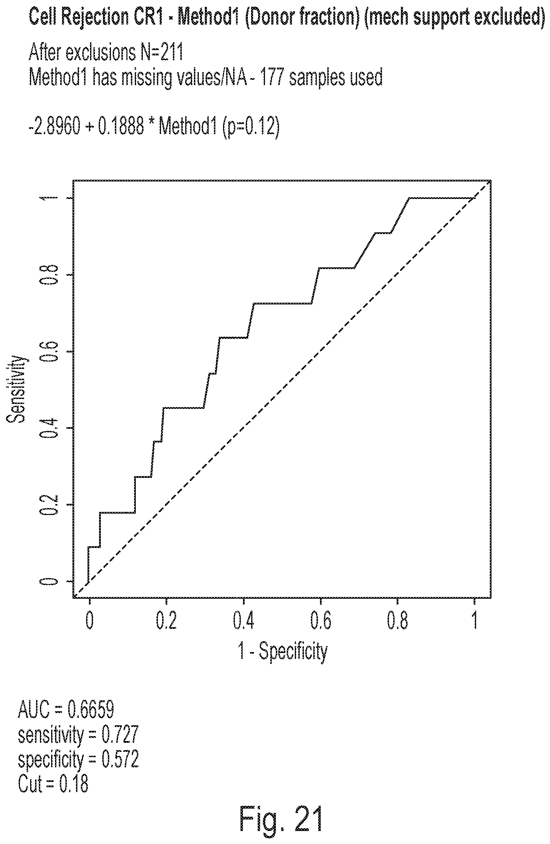

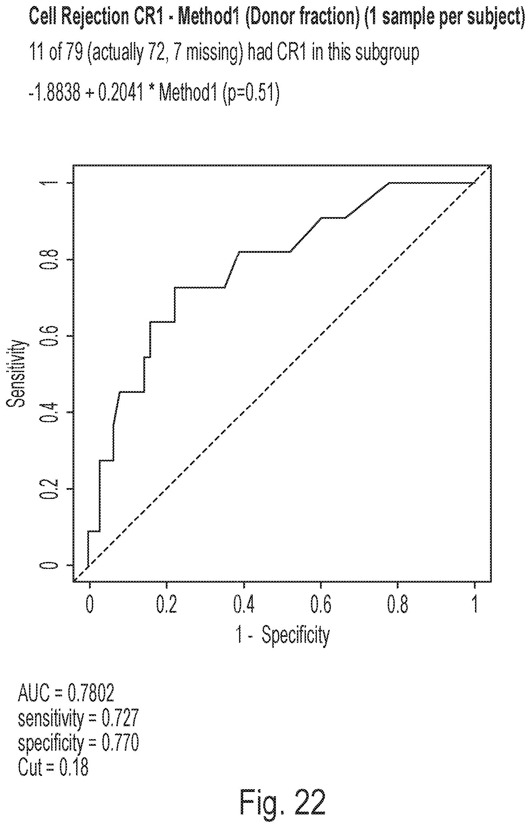

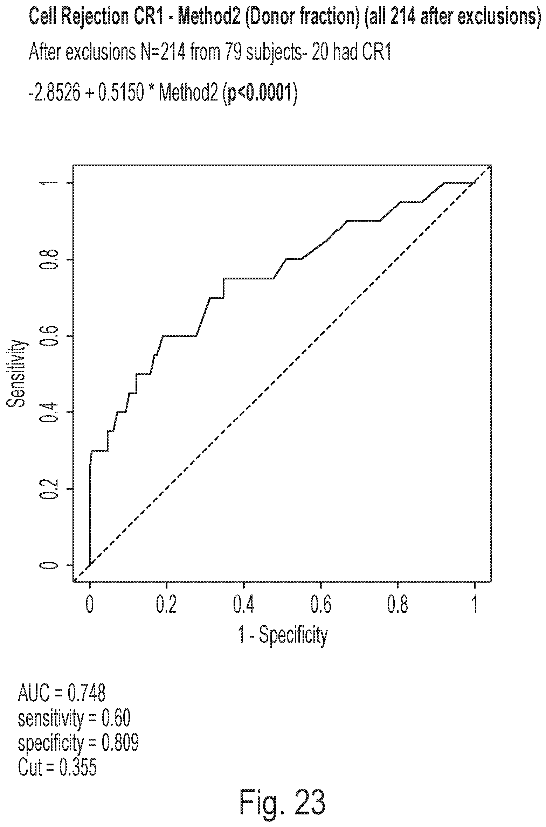

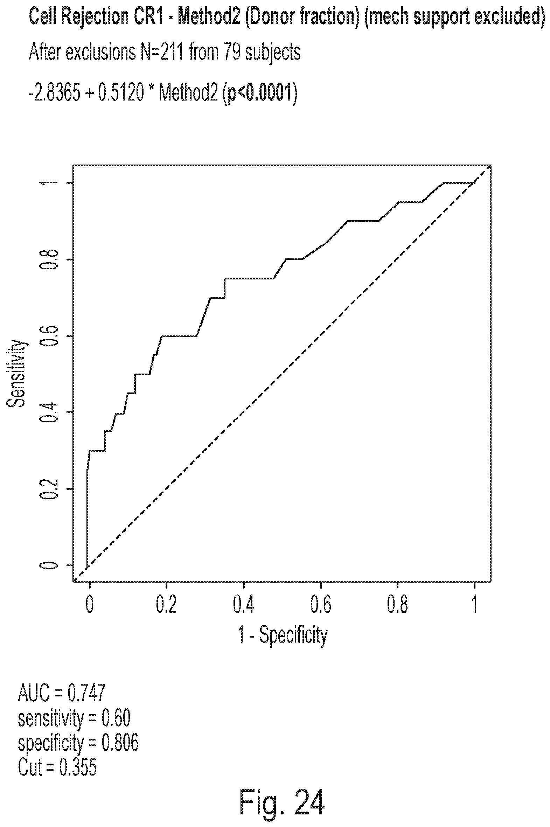

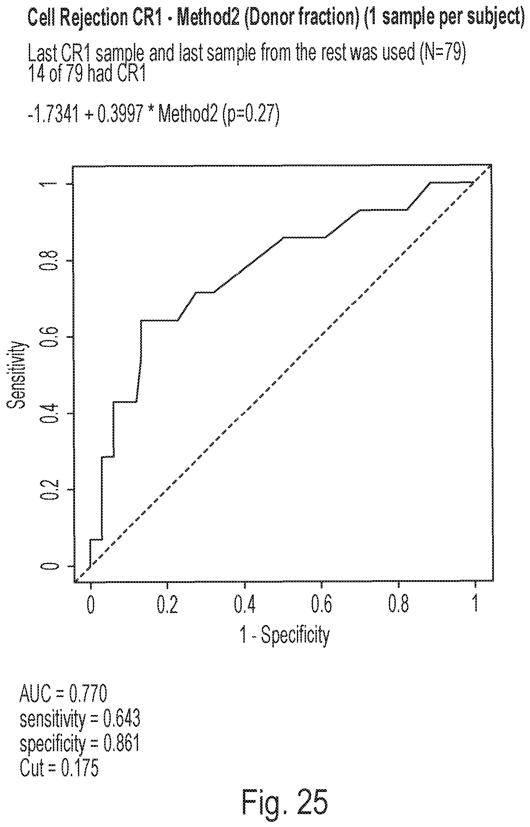

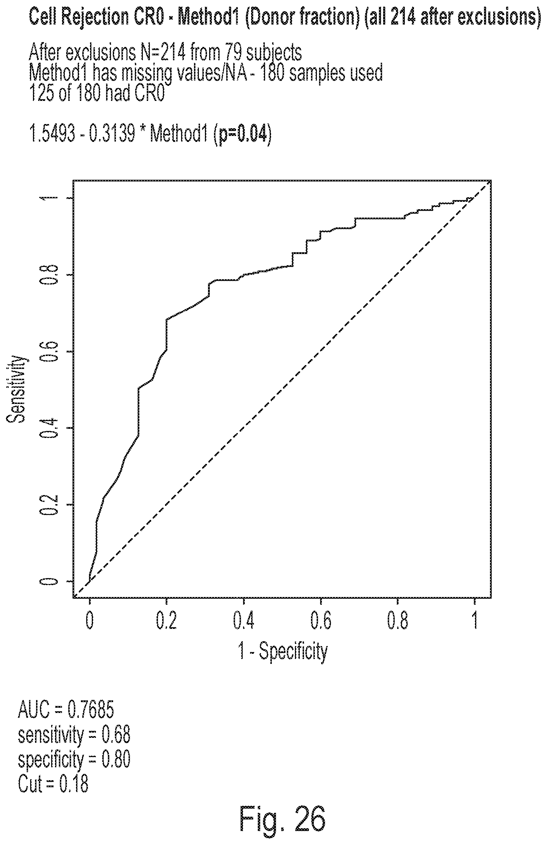

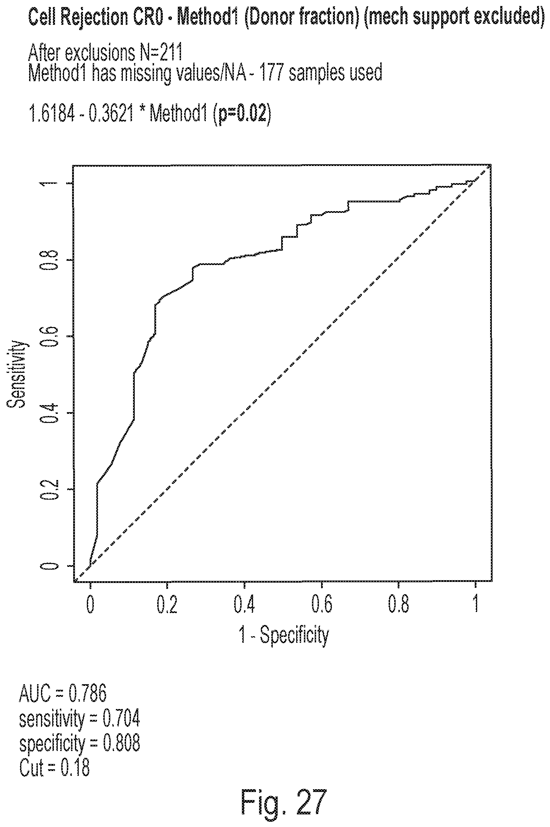

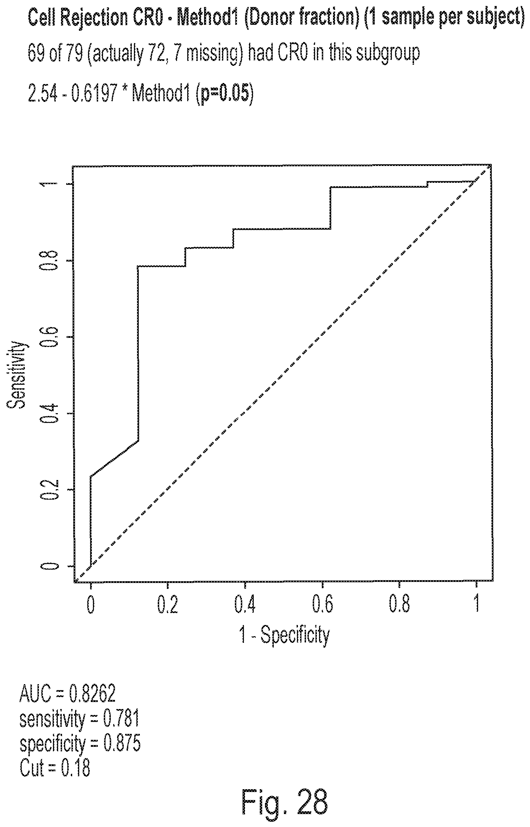

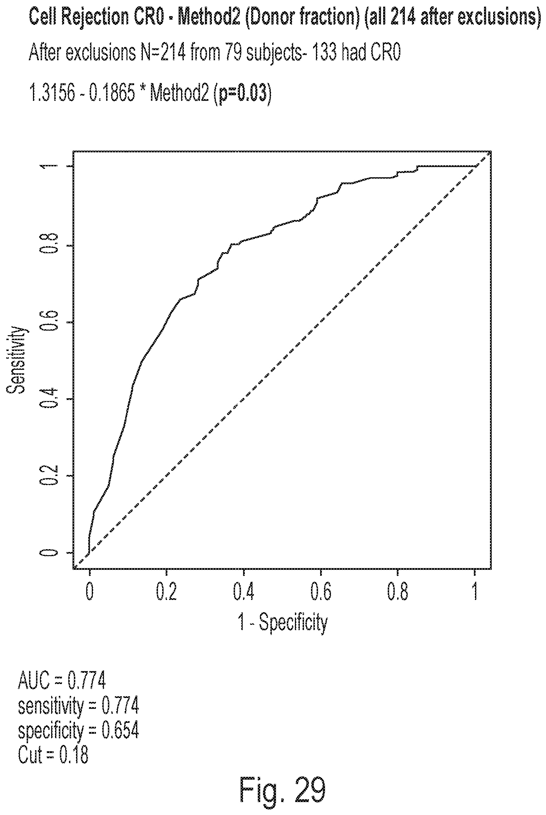

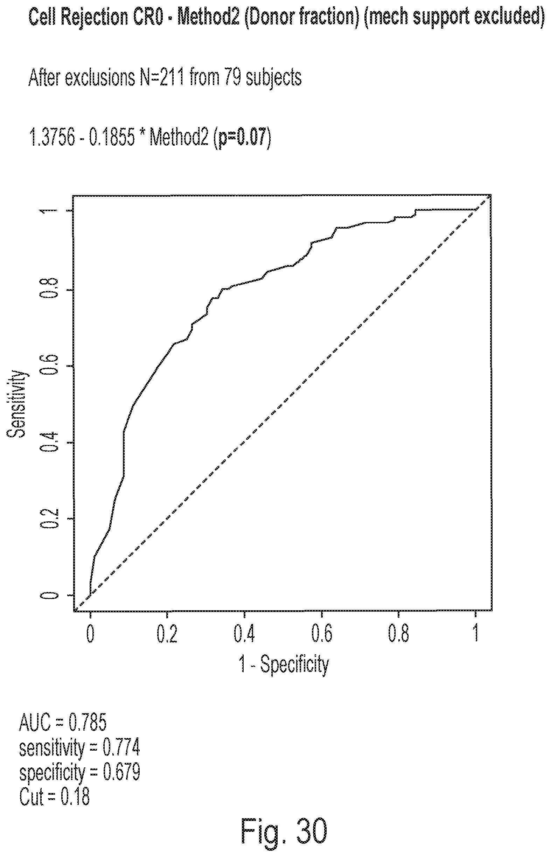

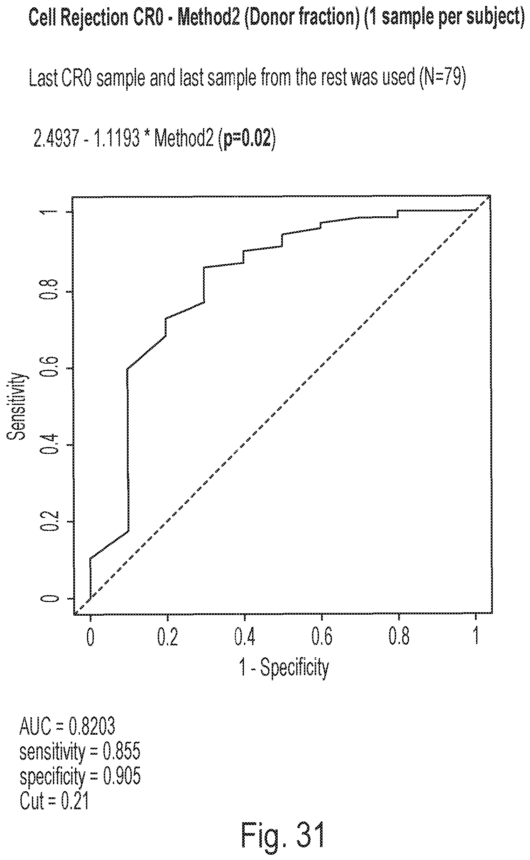

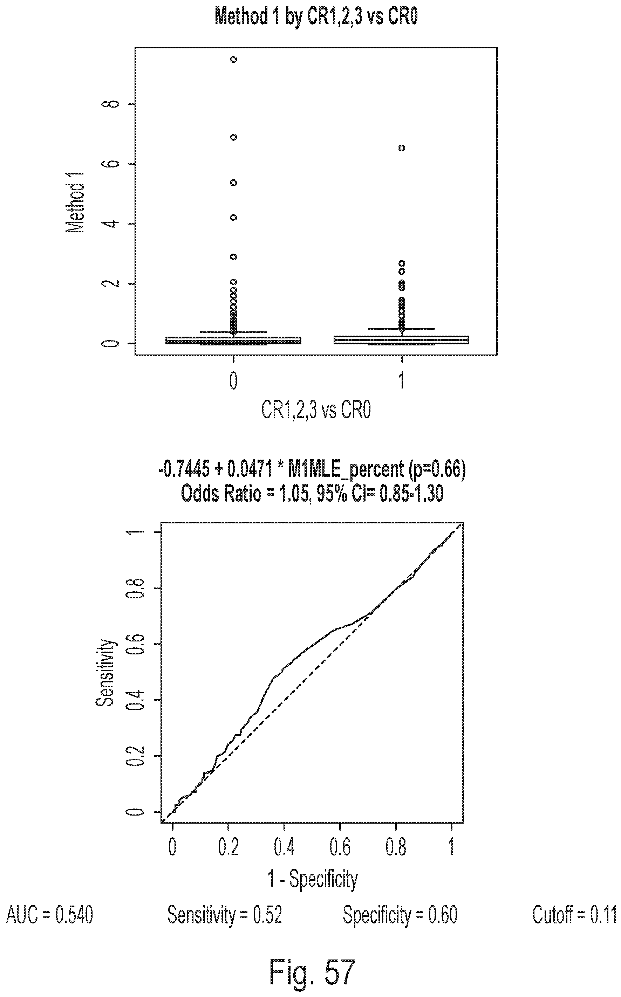

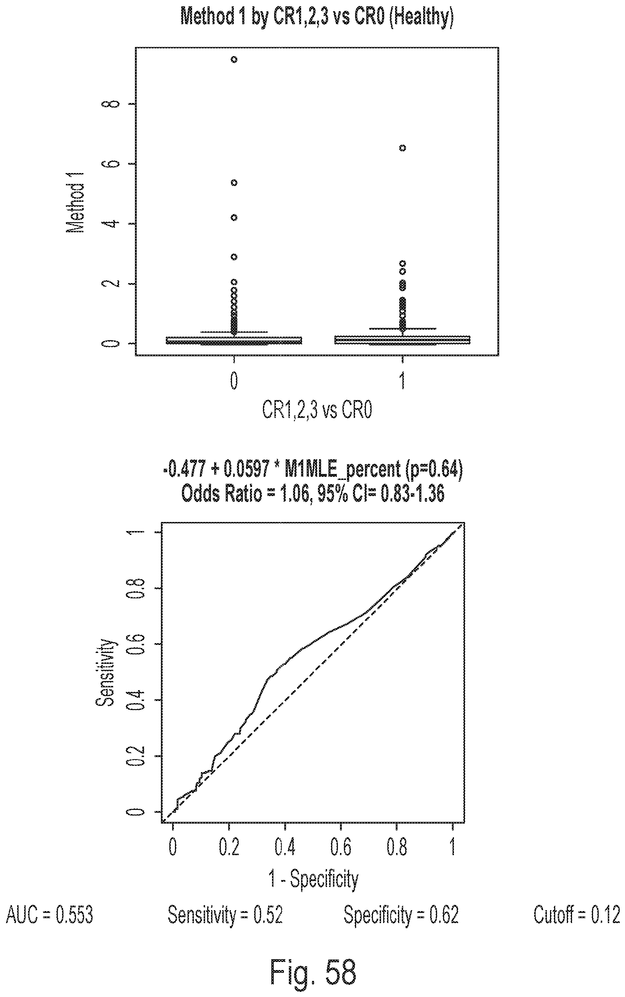

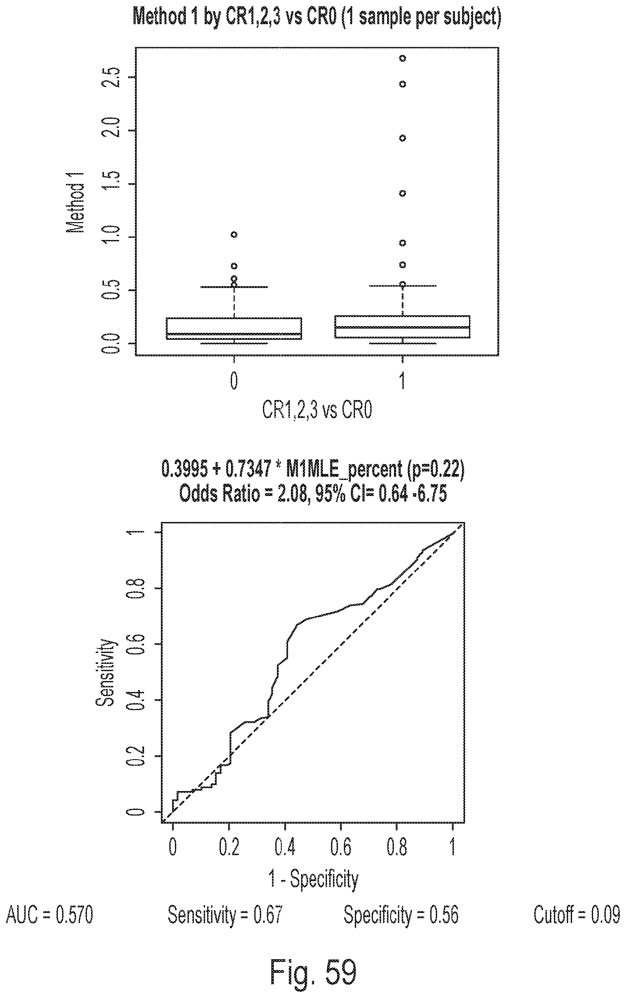

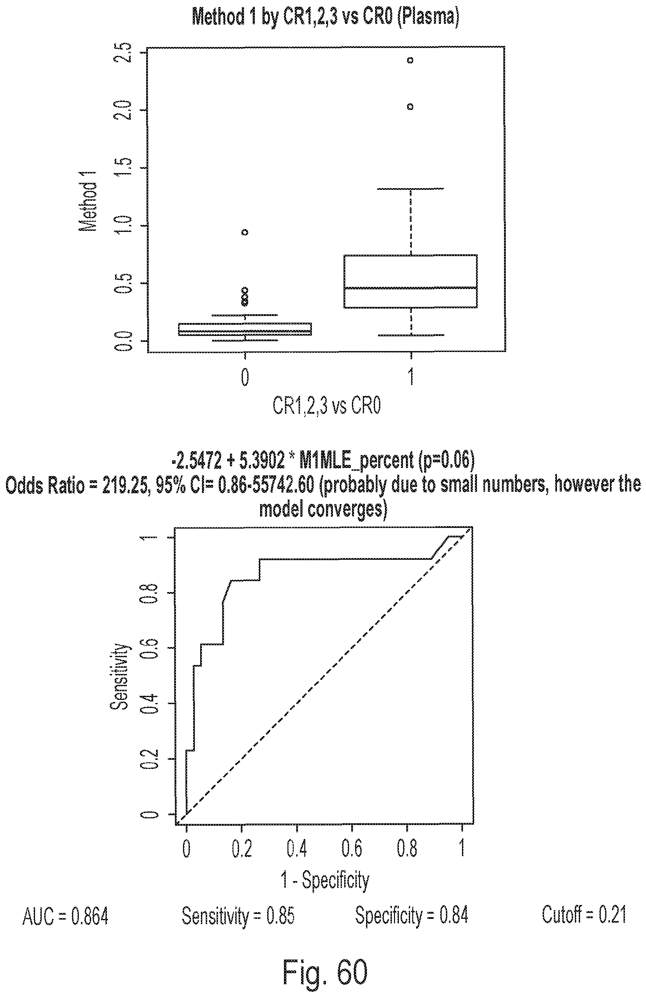

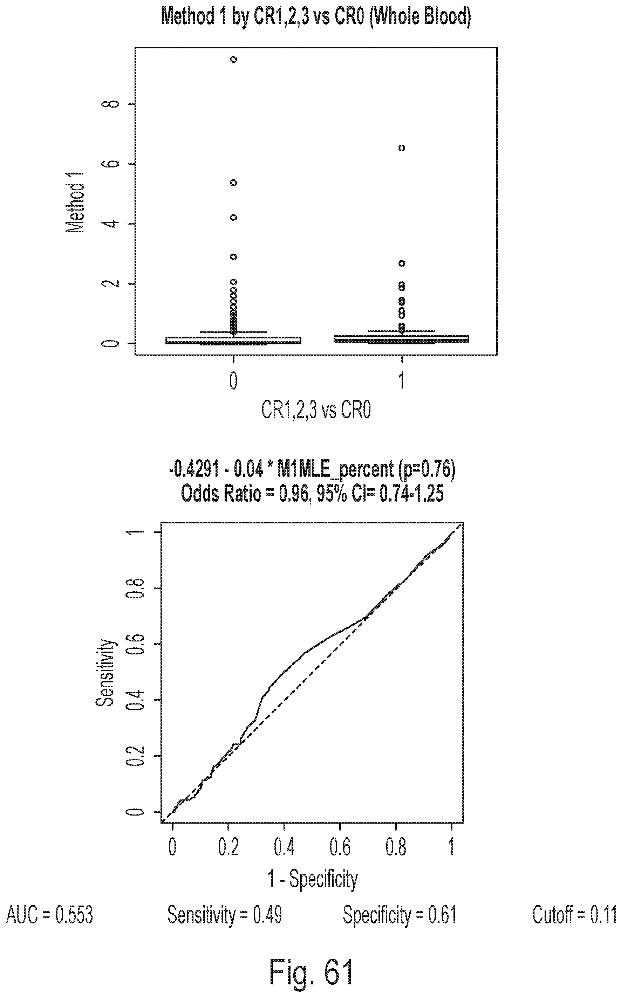

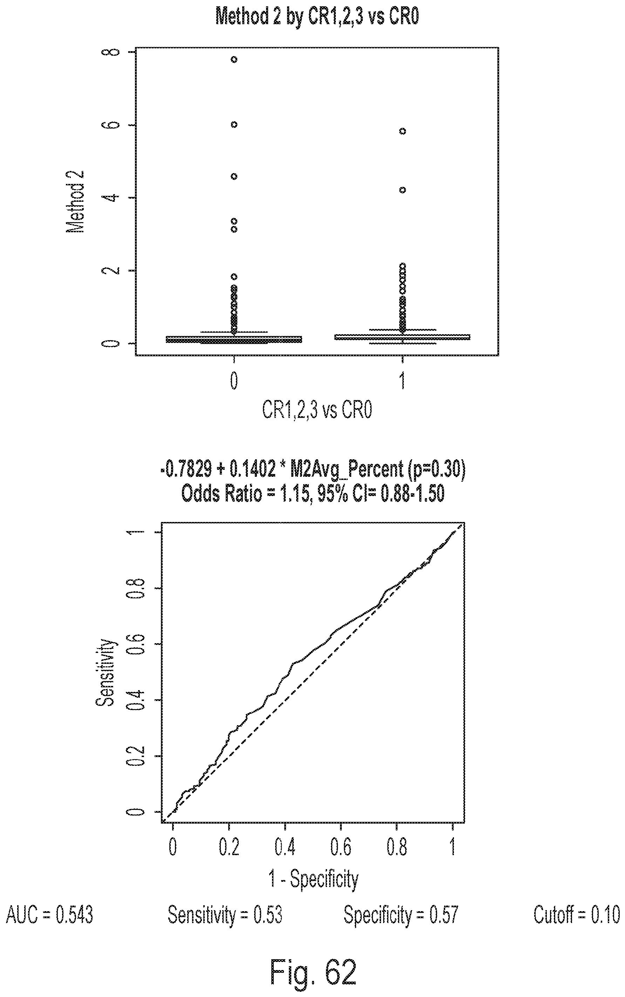

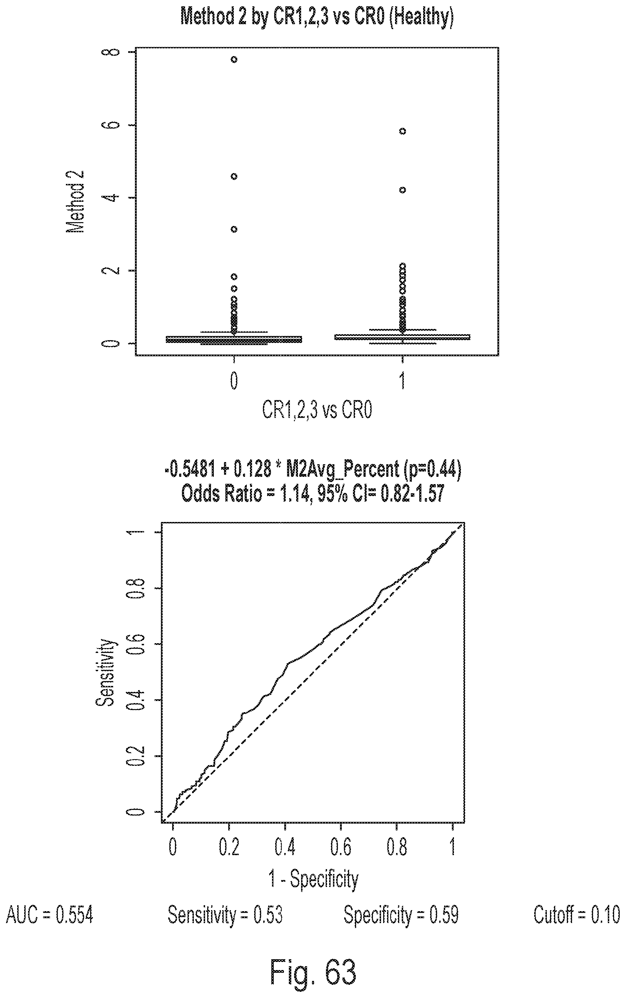

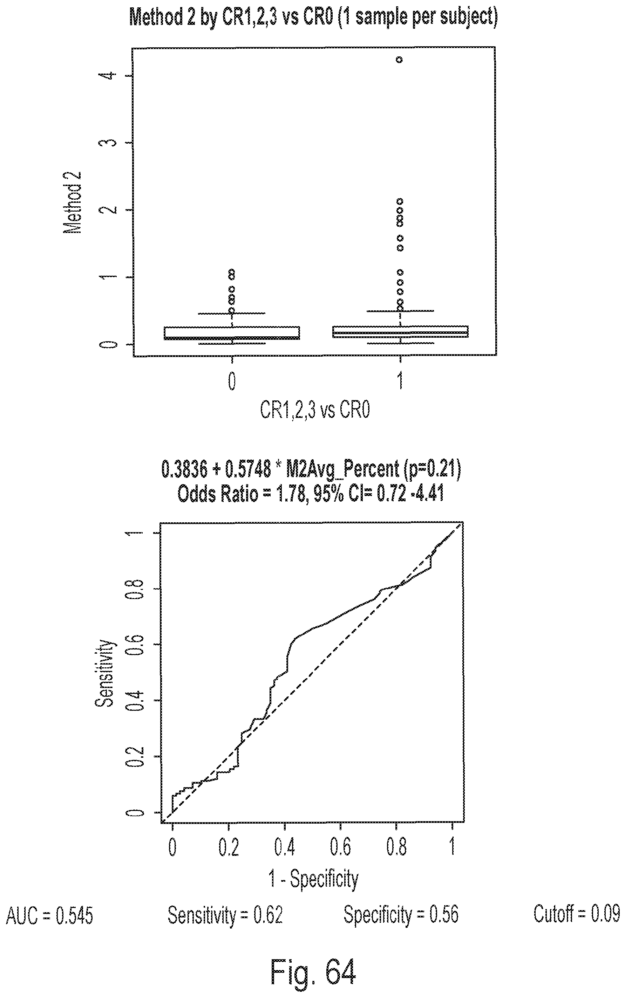

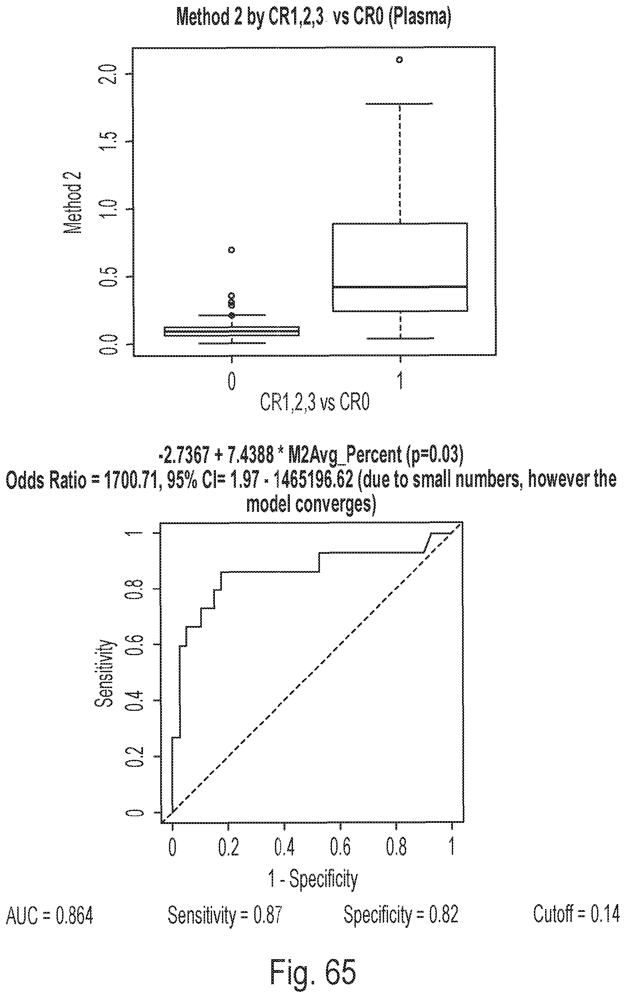

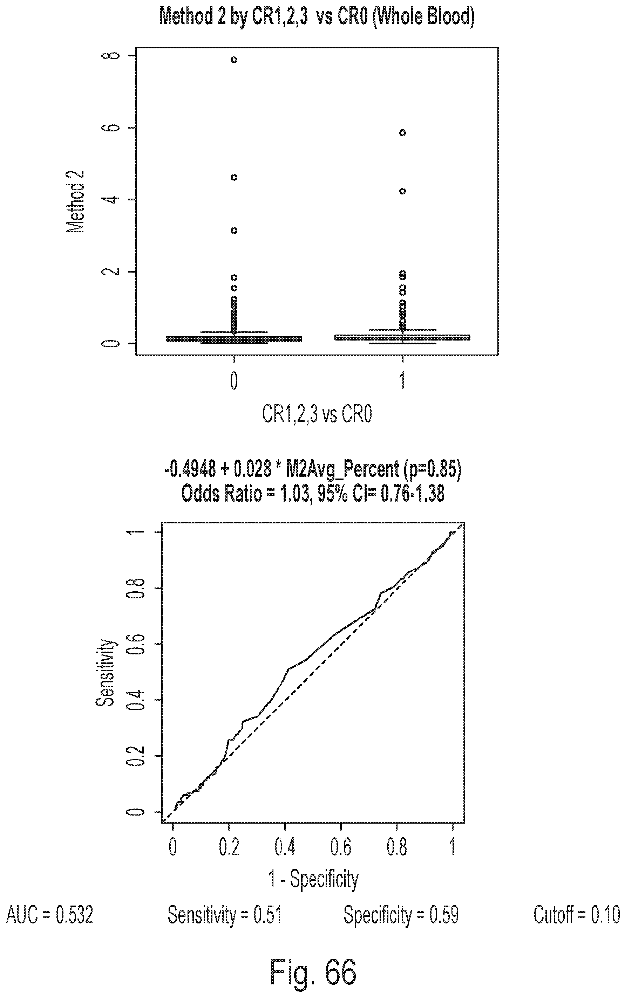

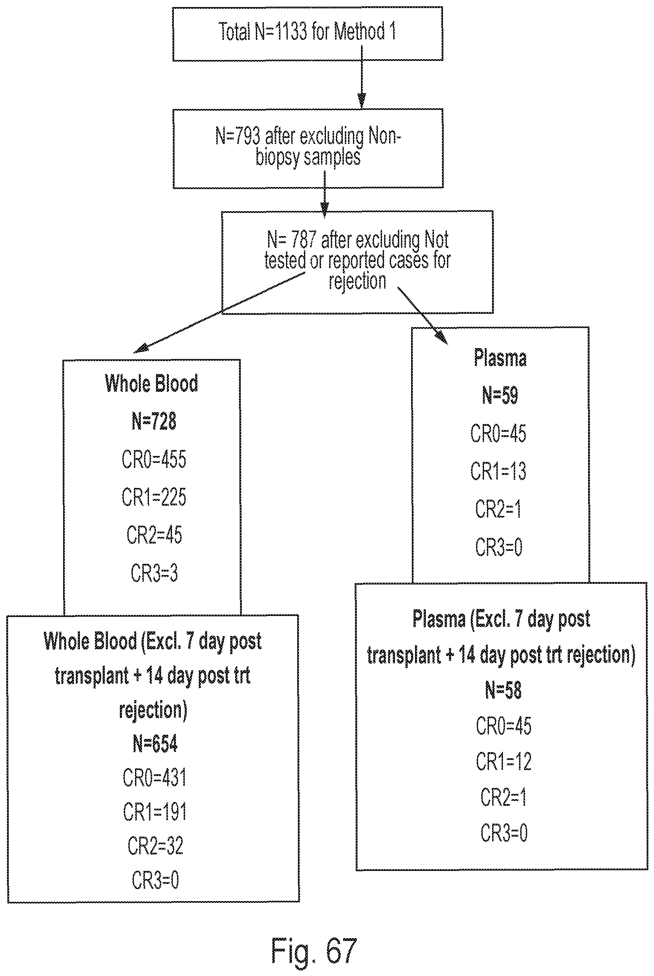

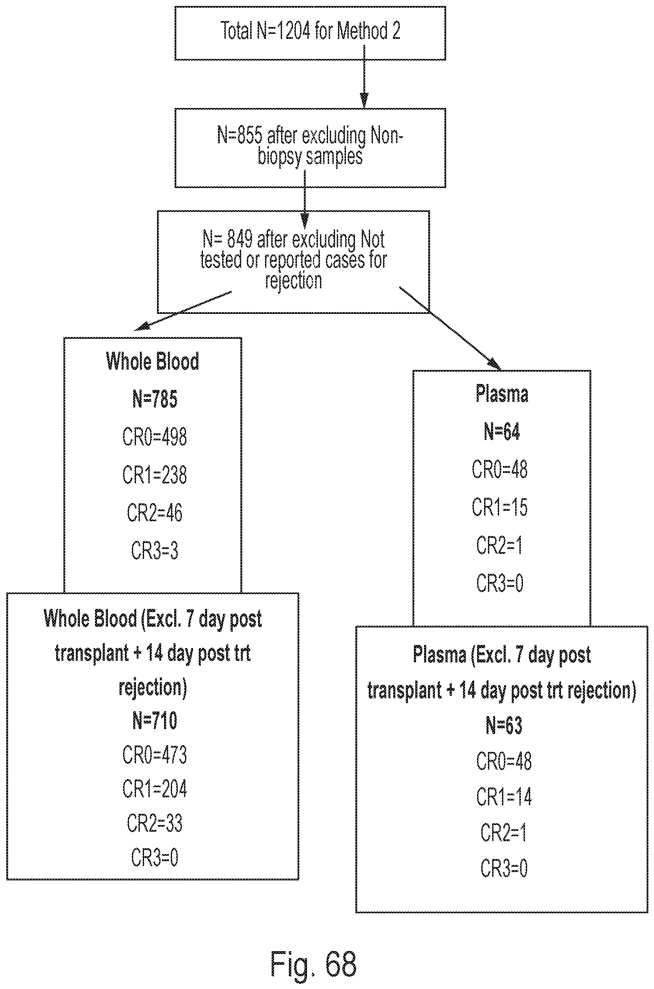

The donor-specific cf-DNA of transplant recipients was quantified using MOMA assays, exemplary steps for such assays are provided herein. **Figures 67** and **68** show the data distribution for MOMA with known genotype **(****Fig. 67****)** and MOMA with unknown donor genotype **(****Fig. 68****).** As shown in exemplary **Figures 7-31** **and** **57-66**, threshold ("cutpoint") values were experimentally determined so that the grades of cellular rejection and/or risk associated thereto could be predicted. Some results were also tabulated and shown in **Tables 1-24** below.

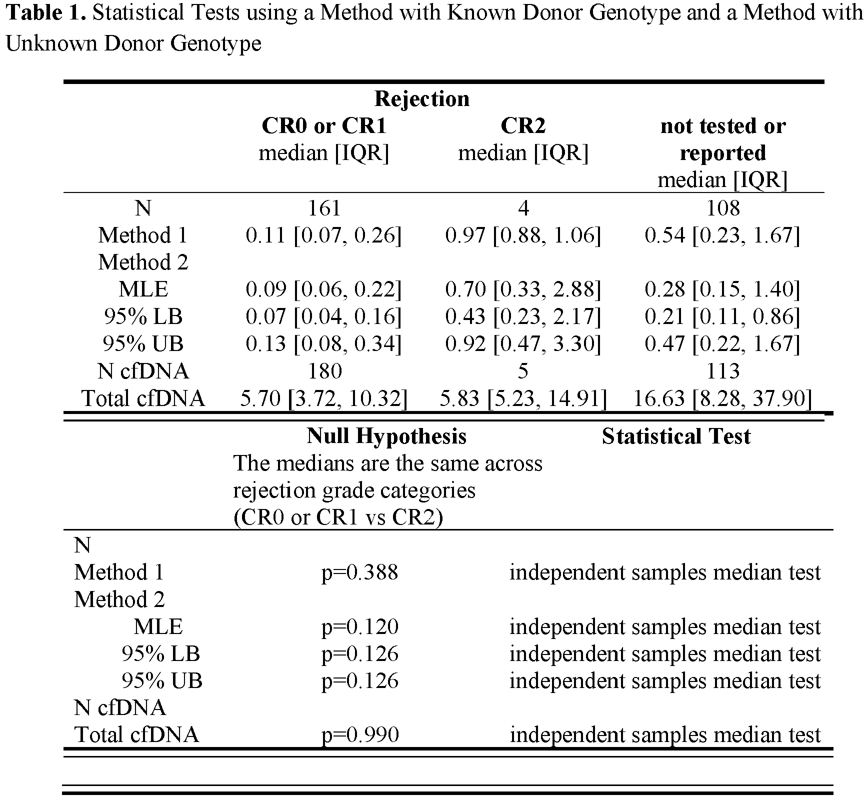

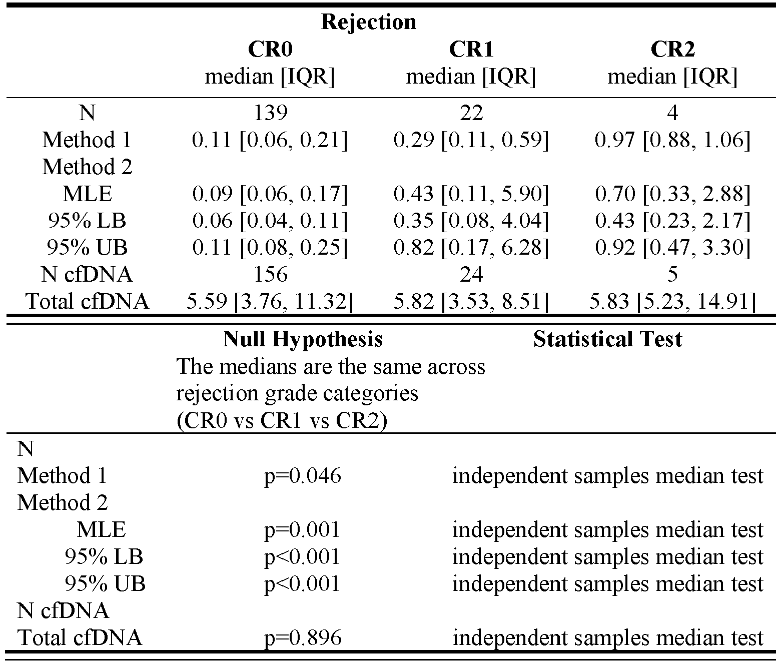

**Table 2. Cross-tabulations Using Experimentally-determined CR0 Cutpoints (MOMA with Known Donor Genotype)**

| **Table of gr1 by cell_rejection0 (p=0.02)** | | | |
|---|---|---|---|
| **gr1** | **cell_rejection0** | | |
| **Frequency** | **0** | **1** | **Total** |
| **Row Pct** | | | |
| **Col Pct** | | | |
| **Method1<=0.18** | 13 | 88 | 101 |
| | 12.87 | 87.13 | |
| | 23.64 | 70.40 | |
| **Method 1>0.18** | 42 | 37 | 79 |
| | 53.16 | 46.84 | |
| | 76.36 | 29.60 | |
| **Total** | 55 | 125 | 180 |
| **Frequency Missing = 34** | | | |

**Table 3. Cross-tabulations UsingExperimentally-determined CR0 Cutpoints (MOMA with Unknown Donor Genotype)**

| **Table of gr1 by cell_rejection0 (p=0.11)** | | | | | **Table of gr2 by cell_rejection0 (p=0.04)** | | | |
|---|---|---|---|---|---|---|---|---|
| **gr1** | **cell_rejection0** | | | | **gr2** | **cell_rejection0** | | |
| **Frequency** | **No** | **Yes** | **Total** | | **Frequency** | **No** | **Yes** | **Total** |
| **Row Pct** | | | | | **Row Pct** | | | |
| **Col Pct** | | | | | **Col Pct** | | | |
| **Method2<=0.18** | 29 | 103 | 132 | | **Method2<=0.21** | 32 | 107 | 139 |
| | 21.97 | 78.03 | | | | 23.02 | 76.98 | |
| | 35.80 | 77.44 | | | | 39.51 | 80.45 | |
| **Method2>0.18** | 52 | 30 | 82 | | **Method2>0.21** | 49 | 26 | 75 |
| | 63.41 | 36.59 | | | | 65.33 | 34.67 | |
| | 64.20 | 22.56 | | | | 60.49 | 19.55 | |
| **Total** | 81 | 133 | 214 | | **Total** | 81 | 133 | 214 |

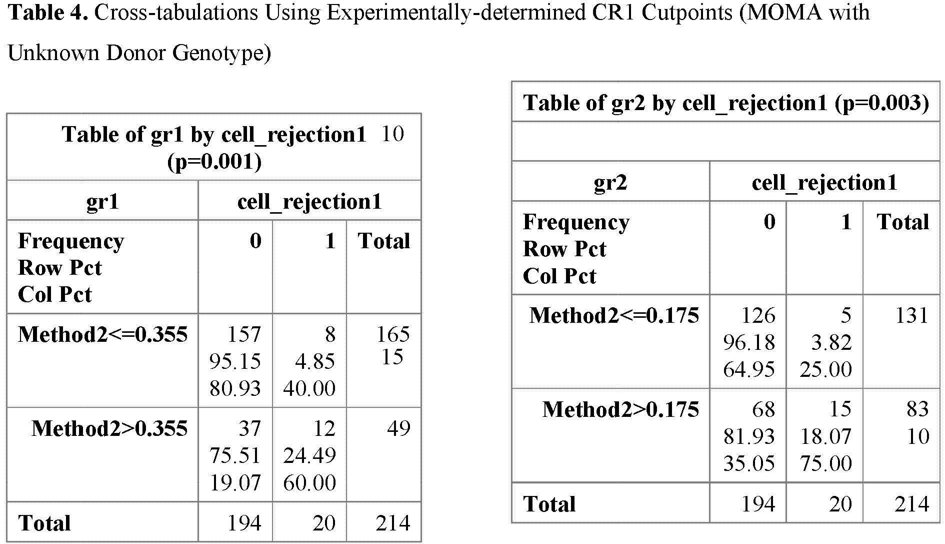

**Table 5. Cross-tabulations Using Experimentally-determined CR2 Cutpoints (MOMA with Known Donor Genotype)**

| **Table of gr1 by cell_rejection** | | | |
|---|---|---|---|
| **gr1** | **CR2** | | |
| **Frequency** | **0** | **1** | **Total** |
| **Percent** | | | |
| **Row Pct** | | | |
| **Col Pct** | | | |
| **0** | 159 | 0 | 159 |
| | 88.33 | 0.00 | 88.33 |
| | 100.00 | 0.00 | |
| | 89.33 | 0.00 | |
| **Method 1 >0.874** | 19 | 2 | 21 |
| | 10.56 | 1.11 | 11.67 |
| | 90.48 | 9.52 | |
| | 10.67 | 100.00 | |
| **Total** | 178 | 2 | 180 |
| | 98.89 | 1.11 | 100.00 |
| **Frequency Missing = 34** | | | |

| **Table of gr2 by cell_rejection** | | | |
|---|---|---|---|
| **gr2** | **CR2** | | |
| **Frequency** | **0** | **1** | **Total** |
| **Percent** | | | |
| **Row Pct** | | | |
| **Col Pct** | | | |
| **0** | 158 | 0 | 158 |
| | 87.78 | 0.00 | 87.78 |
| | 100.00 | 0.00 | |
| | 88.76 | 0.00 | |
| **Method 1 >0.85** | 20 | 2 | 22 |
| | 11.11 | 1.11 | 12.22 |
| | 90.91 | 9.09 | |
| | 11.24 | 100.00 | |
| **Total** | 178 | 2 | 180 |
| | 98.89 | 1.11 | 100.00 |
| **Frequency Missing = 34** | | | |

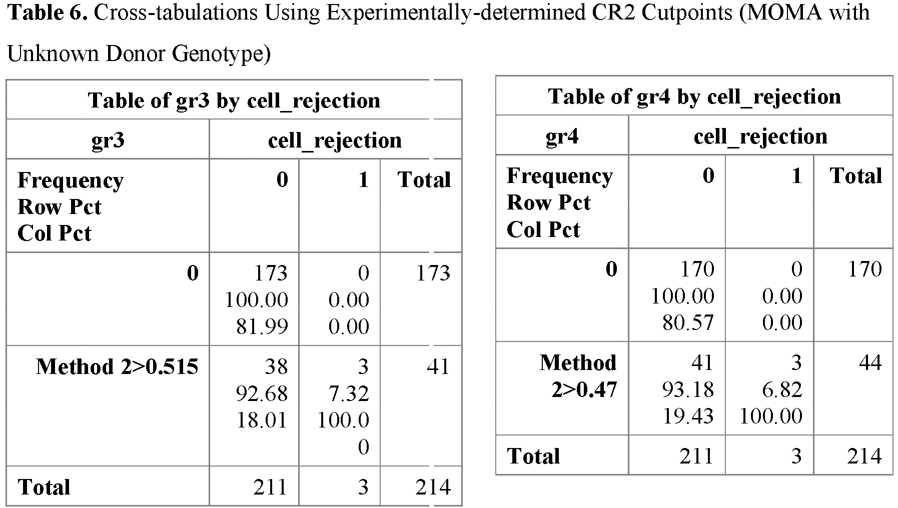

**Table 7. CR1/2/3 vs. CR0 (MOMA with known donor genotype)**

| (includes "not healthy" samples) | | | | | | | | |
|---|---|---|---|---|---|---|---|---|
| **Analysis Variable: MIMLE_ percent M1MLE (%%) (Calculated donor fraction method 1)** | | | | | | | | |
| **rej_cr1_2_3** | **N** | **Mean** | **Std Dev** | **Median** | **Lower Quartile** | **Upper Quartile** | **Minimum** | **Maximum** |
| CR0=0 | 476 | 0.23 | 0.67 | 0.09 | 0.05 | 0.19 | 0.00 | 9.52 |
| CR1/2/3=1 | 236 | 0.25 | 0.55 | 0.12 | 0.05 | 0.23 | 0.00 | 6.55 |

**Table 8. CR1/2/3 vs. CR0 (MOMA with known donor genotype) - Healthy (none of the following: death, cardiac arrest, MCS, treatment for infection, AMR 1 & 2, graft vasculopathy, cancer)**

| **Analysis Variable: M1MLE_percent M1MLE (%%) (Calculated donor fraction method 1)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| CR0=0 | 378 | 0.21 | 0.61 | 0.09 | 0.05 | 0.19 | 0.00 | 9.52 |
| CR1/2/3=1 | 236 | 0.25 | 0.55 | 0.12 | 0.05 | 0.23 | 0.00 | 6.55 |

**Table 9. CR1/2/3 vs. CR0 (MOMA with known donor genotype - 1 sample/subject)**

| In the CR1/2/3 group, the sample used was from the first rejection; in the CR0 group, it was the first sample taken. | | | | | | | | |
|---|---|---|---|---|---|---|---|---|
| **Analysis Variable: M1MLE_ percent M1MLE (%%) (Calculated donor fraction method 1)** | | | | | | | | |
| **rej_cr1_2_3** | **N** | **Mean** | **Std Dev** | **Median** | **Lower Quartile** | **Upper Quartile** | **Minimum** | **Maximum** |
| CR0=0 | 59 | 0.18 | 0.22 | 0.08 | 0.04 | 0.24 | 0.00 | 1.04 |
| CR1/2/3=1 | 103 | 0.26 | 0.43 | 0.15 | 0.05 | 0.26 | 0.00 | 2.69 |

**Table 10. CR1/2/3 vs. CR0 (MOMA with known donor genotype using plasma) - Healthy (none of the following: death, cardiac arrest, MCS, treatment for infection, AMR 1 & 2, graft vasculopathy, cancer)**

| **Analysis Variable: MIMLE_percent M1MLE (%%) (Calculated donor fraction method 1)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| **CR0=0** | 37 | 0.14 | 0.17 | 0.08 | 0.05 | 0.15 | 0.00 | 0.93 |
| **CR1/2/3=1** | 13 | 0.74 | 0.74 | 0.45 | 0.28 | 0.73 | 0.03 | 2.44 |

**Table 11. CR1/2/3 vs. CR0 (MOMA with known donor genotype using whole blood) - Healthy (none of the following: death, cardiac arrest, MCS, treatment for infection, AMR 1 & 2, graft vasculopathy, cancer)**

| **Analysis Variable: MIMLE_ percent M1MLE (%%) (Calculated donor fraction method 1)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| **CR0=0** | 341 | 0.21 | 0.64 | 0.09 | 0.05 | 0.19 | 0.00 | 9.52 |
| **CR1/2/3=1** | 223 | 0.22 | 0.52 | 0.11 | 0.05 | 0.20 | 0.00 | 6.55 |

**Table 12. Cross-tabulations for MOMA with known donor genotype (whole blood and plasma)**

| **Table of gr_09 by rej_cr1_2_3 (p=0.003) OR (M1>0.09 vs M1<=0.09 )(95% CI)= 1.66 (1.19-2.33)** | | | |
|---|---|---|---|
| **gr_09** | **rej_cr1_2_3** | | |
| **Frequency** | | | |
| **Row Pct** | | | |
| **Col Pct** | **0** | **1** | **Total** |
| **M1<=0.09 (0)** | 248 | 98 | 346 |
| | 71.68 | 28.32 | |
| | 52.10 | 41.53 | |
| **M1>0.09 (1)** | 228 | 138 | 366 |
| | 62.30 | 37.70 | |
| | 47.90 | 58.47 | |
| **Total** | 476 | 236 | 712 |

| **Table of gr_11 by rej_cr1_2_3 (p=0.002) OR (M1>0.11 vs M1<=0.11 )(95% CI)= 1.73 (1.23-2.44)** | | | |
|---|---|---|---|
| **gr_11** | **rec_cr1_2_3** | | |
| **Frequency Row Pct Col Pct** | **0** | **1** | **Total** |
| **M1<=0.11 (0)** | 286 | 114 | 400 |
| | 71.50 | 28.50 | |
| | 60.08 | 48.31 | |
| **M1>0.11 (1)** | 190 | 122 | 312 |
| | 60.90 | 39.10 | |
| | 39.92 | 51.69 | |
| **Total** | 476 | 236 | 712 |

| **Table of gr_12 by rej_cr1_2_3 (p=0.002) OR (M1>0.12 vs M1<=0.12 )(95% CI)= 1.72 (1.23-2.40)** | | | |
|---|---|---|---|
| **gr_12** | **rej_cr1_2_3** | | |
| **Frequency Row_Pct Col Pct** | **0** | **1** | **Total** |
| **M1<=0.12 (0)** | 294 | 119 | 413 |
| | 71.19 | 28.81 | |
| | 61.76 | 50.42 | |
| **M1>0.12 (1)** | 182 | 117 | 299 |
| | 60.87 | 39.13 | |
| | 38.24 | 49.58 | |
| **Total** | 476 | 236 | 712 |

| **Table of gr_21 by rej_cr1_2_3 (p=0.11) OR (M1>0.21 vs M1<=0.21 )(95% CI)= 1.38 (0.92-2.06)** | | | |
|---|---|---|---|
| **or 21** | **rec_cr1_2_3** | | |
| **Frequency Row Pct Col Pct** | **0** | **1** | **Total** |
| **M1<=0.21 (0)** | 369 | 171 | 540 |
| | 68.33 | 31.67 | |
| | 77.52 | 72.46 | |
| **M1>0.21 (1)** | 107 | 65 | 172 |
| | 62.21 | 37.79 | |
| | 22.48 | 27.54 | |
| **Total** | 476 | 236 | 712 |

**Table 13. Cross-tabulation for MOMA with known donor genotype (plasma)**

| **Table of gr_21 by rej_cr1_2_3 3 (p=0.0004) OR (M1>0.21 vs M1<=0.21 )(95% CI)= 36.05 (4.92-264.22)** | | | |
|---|---|---|---|
| **gr_21** | **rej_cr1_2_3** | | |
| **Frequency Row Pet Col Pct** | **0** | **1** | **Total** |
| **M1<=0.21 (0)** | 38 | 2 | 40 |
| | 95.00 | 5.00 | |
| | 84.44 | 15.38 | |
| **M1>0.21 (1)** | 7 | 11 | 18 |
| | 38.89 | 61.11 | |
| | 15.56 | 84.62 | |
| **Total** | 45 | 13 | 58 |

**Table 14. Cross-tabulation for MOMA with known donor genotype (whole blood)**

| **Table of gr_11 by rej_cr1_2_3 (p=0.01) OR (M1>0.11 vs M1<=0.11 )(95% CI)= 1.56 (1.10-2.23)** | | | |
|---|---|---|---|
| **or g_11** | **rec_cr1_2_3** | | |
| **Frequency Row Pct Col Pct** | **0** | **1** | **Total** |
| **M1<=0.11 (0)** | 255 | 113 | 368 |
| | 69.29 | 30.71 | |
| | 59.16 | 50.67 | |
| **M1>0.11 (1)** | 176 | 110 | 286 |
| | 61.54 | 38.46 | |
| | 40.84 | 49.33 | |
| **Total** | 431 | 223 | 654 |

**Table 15. CR1/2/3 vs. CR0 (MOMA with unknown donor genotype)**

| (includes "not healthy" samples) | | | | | | | | |
|---|---|---|---|---|---|---|---|---|
| **Analysis Variable: M2Avg_Percent M2Avg (%%) (Calculated donor fraction method 2) (metrics related to method 2)** | | | | | | | | |
| **rej_cr1_2_3** | **N** | **Mean** | **Std Dev** | **Median** | **Lower Quartile** | **Upper Quartile** | **Minimum** | **Maximum** |
| CR0=0 | 521 | 0.21 | 0.55 | 0.09 | 0.07 | 0.17 | 0.00 | 7.85 |
| CR1/2/3=1 | 252 | 0.25 | 0.55 | 0.10 | 0.07 | 0.21 | 0.00 | 5.83 |

**Table 16. CR1/2/3 vs. CR0 (MOMA with unknown donor genotype) - Healthy (none of the following: death, cardiac arrest, MCS, treatment for infection, AMR 1 & 2, graft vasculopathy, cancer)**

| **Analysis Variable: M2Avg_Percent M2Avg (%%) (Calculated donor fraction method 2) (metrics related to method 2)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| **CR0=0** | 423 | 0.19 | 0.50 | 0.09 | 0.06 | 0.16 | 0.00 | 7.85 |
| **CR1/2/3=1** | 252 | 0.25 | 0.55 | 0.10 | 0.07 | 0.21 | 0.00 | 5.83 |

**Table 17. CR1/2/3 vs. CR0 (MOMA with unknown donor genotype - 1 sample/subject)**

| In the CR1/2/3 group, the sample used was from the first rejection; in the CR0 group, it was the first sample taken. | | | | | | | | |
|---|---|---|---|---|---|---|---|---|
| **Analysis Variable: M2Avg_Percent M2Avg (%%) (Calculated donor fraction method 2) (metrics related to method 2)** | | | | | | | | |
| **rej_cr1_2_3** | **N** | **Mean** | **Std Dev** | **Median** | **Lower Quartile** | **Upper Quartile** | **Minimum** | **Maximum** |
| **CR0=0** | 68 | 0.19 | 0.23 | 0.09 | 0.06 | 0.23 | 0.00 | 1.07 |
| **CR1/2/3=1** | 114 | 0.29 | 0.55 | 0.12 | 0.07 | 0.24 | 0.00 | 4.22 |

**Table 18. CR1/2/3 vs. CR0 (MOMA with unknown donor genotype using plasma) - Healthy (none of the following: death, cardiac arrest, MCS, treatment for infection, AMR 1 & 2, graft vasculopathy, cancer)**

| **Analysis Variable: M2Avg_Percent M2Avg (%%) (Calculated donor fraction method 2) (metrics related to method 2)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| **CR0=0** | 40 | 0.12 | 0.12 | 0.09 | 0.06 | 0.12 | 0.00 | 0.70 |
| **CR1/2/3=1** | 15 | 0.68 | 0.67 | 0.42 | 0.19 | 1.16 | 0.04 | 2.10 |

**Table 19. CR1/2/3 vs. CR0 (MOMA with unknown donor genotype using whole blood) - Healthy (none of the following: death, cardiac arrest, MCS, treatment for infection, AMR 1 & 2, graft vasculopathy, cancer)**

| **Analysis Variable: M2Avg_Percent M2Avg (%%) (Calculated donor fraction method 2) (metrics related to method 2)** | | | | | | | | |
|---|---|---|---|---|---|---|---|---|
| **rej_cr1_2_3** | **N** | **Mean** | **Std Dev** | **Median** | **Lower Quartile** | **Upper Quartile** | **Minimum** | **Maximum** |
| **CR0=0** | 383 | 0.20 | 0.52 | 0.09 | 0.06 | 0.17 | 0.00 | 7.85 |
| **CR1/2/3=1** | 237 | 0.22 | 0.53 | 0.10 | 0.07 | 0.20 | 0.00 | 5.83 |

**Table 20. Cross-tabulations for MOMA with unknown donor genotype (whole blood and plasma)**

| **Table of gr_10 by rej_cr1_2_3 (p=0.03) OR (M2>0.10 vs M2<=0.10 )(95% CI)= 1.43 (1.03-1.99)** | | | |
|---|---|---|---|
| **gr_10** | **rej_cr1_2_3** | | |
| **Frequency Row Pct Col Pct** | **0** | **1** | **Total** |
| **M2<=0.10 (0)** | 308 | 128 | 436 |
| | 70.64 | 29.36 | |
| | 59.12 | 50.79 | |
| **M2>0.10 (1)** | 213 | 124 | 337 |
| | 63.20 | 36.80 | |
| | 40.88 | 49.21 | |
| **Total** | 521 | 252 | 773 |

| **Table of gr_14 by rej_cr1_2_3 (p=0.03) OR (M2>0.14 vs M2<=0.14 )(95% CI)= 1.46 (1.03-2.06)** | | | |
|---|---|---|---|
| **gr_14** | **rej_cr1_2_3** | | |
| **Frequency Row Pct Col Pct** | **0** | **1** | **Total** |
| **M2<=0.14 (0)** | 369 | 160 | 529 |
| | 69.75 | 30.25 | |
| | 70.83 | 63.49 | |
| **M2>0.14 (1)** | 152 | 92 | 244 |
| | 62.30 | 37.70 | |
| | 29.17 | 36.51 | |
| **Total** | 521 | 252 | 773 |

**Table 21. Cross-tabulation for MOMA with unknown donor genotype (plasma)**

| **Table of gr_14 by rej_cr1_2_3 (p=0.0002) OR (M2>0.14 vs M2<=0.14 )(95% CI)= 26.63 (4.83-146.72)** | | | |
|---|---|---|---|
| **gr_14** | **rej_cr1_2_3** | | |
| **Frequency Row Pct Col Pct** | **0** | **1** | **Total** |
| **M2<=0.14 (0)** | 39 | 2 | 41 |
| | 95.12 | 4.88 | |
| | 81.25 | 13.33 | |
| **M2>0.14 (1)** | 9 | 13 | 22 |
| | 40.91 | 59.09 | |
| | 18.75 | 86.67 | |
| **Total** | 48 | 15 | 63 |

**Table 22. Cross-tabulation for MOMA with unknown donor genotype (whole blood)**

| **Table of gr_10 by rej_cr1_2_3 3 (p=0.13) OR (M2>0.10 vs M2<=0.10 )(95% CI)= 1.30 (0.93-1.82)** | | | |
|---|---|---|---|
| **gr_10** | **rej_cr1_2_3** | | |
| **Frequency Row Pct Col Pct** | **0** | **1** | **Total** |
| **M2<=0.10 (0)** | 277 | 126 | 403 |
| | 68.73 | 31.27 | |
| | 58.56 | 53.16 | |
| **M2>0.10 (1)** | 196 | 111 | 307 |
| | 63.84 | 36.16 | |
| | 41.44 | 46.84 | |
| **Total** | 473 | 237 | 710 |

**Table 23. MOMA with known donor genotype across rejections (7 day post-transplant, 14 day post treatment for rejection excluded)**

| | **Analysis Variable: MIMLE_ percent M1MLE (%%) (Calculated donor fraction method 1)** | | | | | | | | |
|---|---|---|---|---|---|---|---|---|---|
| **Shipped As** | **Cellular rejection grade** | **N** | **Mean** | **Std Dev** | **Median** | **Lower Quartile** | **Upper Quartile** | **Minimum** | **Maximum** |
| **Whole Blood** | **CR0** | 431 | 0.239 | 0.697 | 0.090 | 0.050 | 0.210 | 0.000 | 9.520 |
| | **CR1** | 191 | 0.179 | 0.267 | 0.110 | 0.050 | 0.200 | 0.000 | 1.930 |
| | **CR2** | 32 | 0.440 | 1.208 | 0.140 | 0.055 | 0.260 | 0.030 | 6.550 |
| **Plasma** | **CR0** | 45 | 0.136 | 0.159 | 0.080 | 0.050 | 0.150 | 0.000 | 0.930 |
| | **CR1** | 12 | 0.597 | 0.565 | 0.425 | 0.250 | 0.710 | 0.030 | 2.030 |
| | **CR2** | 1 | 2.440 | . | 2.440 | 2.440 | 2.440 | 2.440 | 2.440 |
| **Total** | **CR0** | 476 | 0.229 | 0.665 | 0.090 | 0.050 | 0.190 | 0.000 | 9.520 |
| | **CR1** | 203 | 0.204 | 0.307 | 0.120 | 0.050 | 0.220 | 0.000 | 2.030 |
| | **CR2** | 33 | 0.501 | 1.239 | 0.140 | 0.060 | 0.280 | 0.030 | 6.550 |

WB + Plasma across rejections:
   **CR0 vs CR2 p=0.04 (CR2 higher than CR0); CR1 vs CR2 p=0.01 (CR2 higher than CR1);** CR0 vs CR1 p=0.39;
Whole Blood across rejections:
   CR0 vs CR2 p=0.16; **CR1 vs CR2 p=0.04 (CR2 higher than CR1) ;** CR0 vs CR1 p=0.11
   Plasma across rejections: **CR0 vs CR1 p<0.0001 (CR1 higher than CR0)**

**Table 24. MOMA with unknown donor genotype across rejections (7 day post-transplant, 14 day post treatment for rejection excluded)**

| | **Analysis Variable : M2Avg_Percent M2Avg (%%) (Calculated donor fraction method 2) (metrics related to method 2)** | | | | | | | | |
|---|---|---|---|---|---|---|---|---|---|
| **Shipped As** | **Cellular rejection grade** | **N** | **Mean** | **Std Dev** | **Median** | **Lower Quartile** | **Upper Quartile** | **Minimum** | **Maximum** |
| **Whole Blood** | **CR0** | 473 | 0.216 | 0.570 | 0.090 | 0.065 | 0.170 | 0.000 | 7.845 |
| | **CR1** | 204 | 0.200 | 0.387 | 0.100 | 0.065 | 0.195 | 0.000 | 4.220 |
| | **CR2** | 33 | 0.363 | 1.036 | 0.105 | 0.070 | 0.225 | 0.030 | 5.825 |
| **Plasma** | **CR0** | 48 | 0.119 | 0.113 | 0.085 | 0.063 | 0.120 | 0.000 | 0.700 |
| | **CR1** | 14 | 0.581 | 0.565 | 0.393 | 0.190 | 0.600 | 0.040 | 1.760 |
| | **CR2** | 1 | 2.100 | . | 2.100 | 2.100 | 2.100 | 2.100 | 2.100 |
| **Total** | **CR0** | 521 | 0.207 | 0.545 | 0.090 | 0.065 | 0.165 | 0.000 | 7.845 |
| | **CR1** | 218 | 0.224 | 0.410 | 0.100 | 0.065 | 0.205 | 0.000 | 4.220 |
| | **CR2** | 34 | 0.414 | 1.062 | 0.105 | 0.070 | 0.250 | 0.030 | 5.825 |

WB + Plasma across rejections:
   **CR0 vs CR2 p=0.05; CR1 vs CR2 p=0.08;**
   CR0 vs CR1 p=0.83;
Whole Blood across rejections:
   CR0 vs CR2 p=0.20; CR1 vs CR2 p=0.14 (CR2 higher than CR1) ; CR0 vs CR1 p=0.62
   Plasma across rejections: **CR0 vs CR1 p<0.0001 (CR1 higher than CR0)**

### Example 6 - Donor Specific Cell Free DNA as a Non-Invasive Indicator Following Cardiac Transplantation

### Objective

The current gold standard for surveillance of heart transplant recipients is endomyocardial biopsy (EMB) which puts children at particular risk for injury, limits the frequency of reasonable screening, and is inherently limited in sensitivity because rejection can occur as a patchy process. A non-invasive diagnostic screening tool for pediatric and adult heart transplant recipients based on the precise quantification of circulating donor specific cell-free DNA (DS cfDNA) was developed in order to replace the need for invasive surveillance biopsy.

### Methods

All cardiac transplant recipients followed at the Children's Hospital of Wisconsin (CHW) were invited to participate in this blinded study. Three to ten milliliters (ml) of anticoagulated blood were collected to assess circulating levels of cf-DNA in the following clinical scenarios: days 1,4,7, and 28 following transplant, within 24 hours prior to any EMB, days 1,4,7, and 28 after initiation of treatment for rejection. Samples were immediately coded, de-identified, and delivered to the laboratory for processing using the MyT AI(heart) test (TAI diagnostics, Milwaukee, WI). Clinical, laboratory, cardiac biopsy, angiography, catheterization and echocardiographic data were all recorded at the time of sample collection. The pathology report of all biopsy reports was reviewed and 2004 International Society for Heart and Lung Transplantation (ISHLT) grade was recorded. Dates and times of all critical events including treatment for infection, treatment for rejection, cardiac arrest, cardiac retransplantation, initiation of mechanical circulatory support, and death were recorded. The donor specific (ds) cell-free DNA (cf-DNA) was quantified using a targeted approached and compared to associated biopsy and angiography result using two distinct methods: with donor genotype (method 1) and without donor genotype (method 2).

### Results

A total of 158 samples from 87 unique transplant recipient subjects both adult and pediatric passed QC standards and were available for analysis. One individual participated in the study both after initial transplantation and after re-transplantation and was analyzed as two unique subjects given two unique donor/recipient mismatched DNA. Mean patient age at transplant was 7.9 +/- 7.5 years (range 0.03 to 24.2 years). Mean age at blood sample was 12.7 +/- 8.1 years (range 0.08 to 30.2 years). 59.6% (51/87) subjects were male, and 65.5% (57/87) were white. Mean time from transplant to blood sample was 4.8 +/- 4.2 years. 134 biopsies were grade CR0, 21 biopsies were grade CR1, and 3 biopsies were grade CR2. Mean donor-specific cf-DNA fraction was 0.11% (IQR 0.06-0.21%) in samples associated with grade CR0 biopsies, 0.37% (IQR 0.15-0.72%) in samples associated with grade CR1, and 0.97% (IQR 0.88-1.06%) in samples associated with grade CR2 (p=0.027). The empirical optimal cutpoint for ruling out grade CR2 rejection based on the associated ROC curve was 0.87% [95% CI 0.78-0.97% (p=0.009)]. The area under the curve was 0.97. The sensitivity was 100% and specificity was 93%.

As another example, without donor genotype, the mean donor cf-DNA fraction was 0.25% (IQR 0.17-0.39%) in samples associated with grade CR0 biopsies, 0.89% (IQR 0.44-5.35%) in samples associated with grade CR1 biopsies, and 1.22% (IQR 1.04-5.18%) in samples associated with grade CR2 biopsies (p<0.001). The empirical optimal cutpoint for ruling out grade CR2 rejection based on the associated ROC curve was 0.89% [95% CI 0.46-1.70% (p=0.725)]. The area under the curve was 0.95. Sensitivity was 100% and specificity was 89%.

### Conclusions

It was found that use of a targeted, high-throughput assay for the quantification of donor-specific cf-DNA has excellent sensitivity for acute cellular rejection surveillance in heart transplant recipients. Strikingly, a stepwise increase in donor-specific cf-DNA among CR0, CR1, and CR2 associated biopsies was observed, suggesting progressive allograft injury across increasing rejection grades.

### Example 7 - Donor-specific Cell-free DNA (DS cf-DNA) Correlation with Cardiac Allograft Vasculopathy

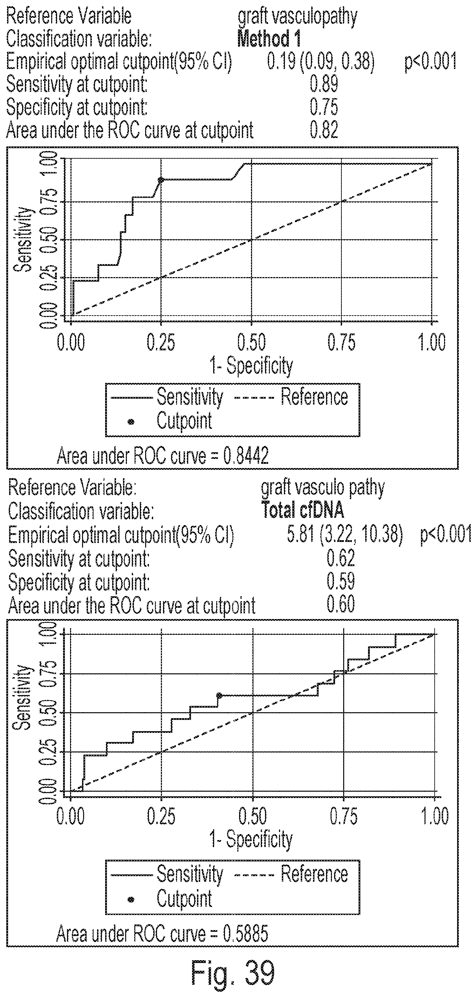

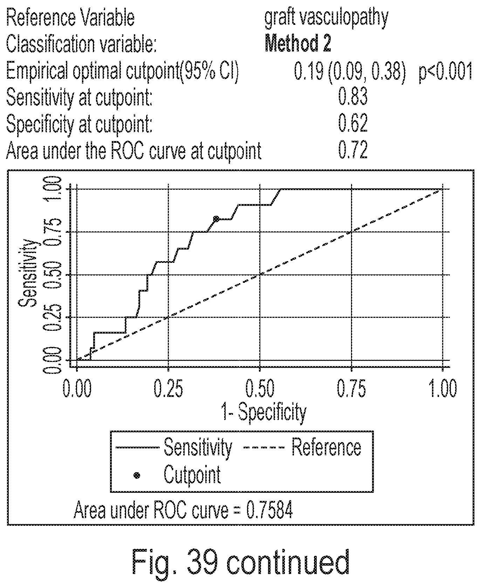

The donor-specific cf-DNA of transplant recipients (n=273) was quantified using MOMA. As shown in **Fig. 39****,** threshold ("cutpoint") values for cardiac allograft vasculopathy (graft vasculopathy) were experimentally determined using two different methods (top row), with known donor genotype (Method 1) and with unknown donor genotype (Method 2). Additionally, total cell-free DNA was examined and a threshold was experimentally determined (bottom row). Further, **Table 25** below shows additional statistics.

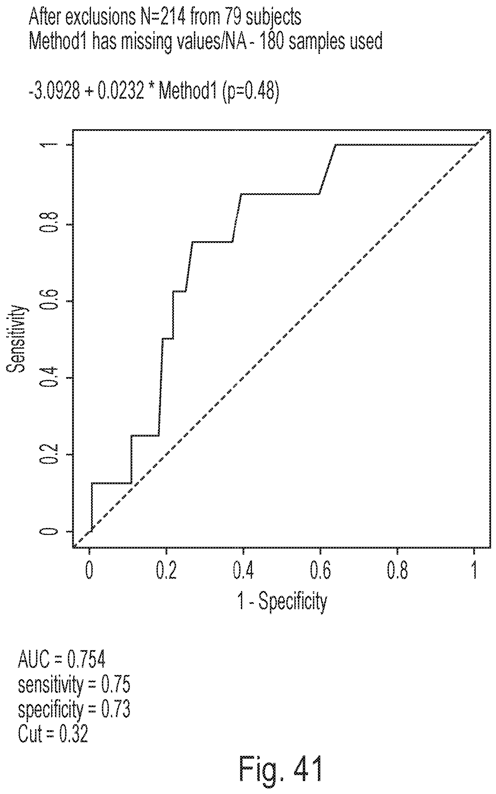

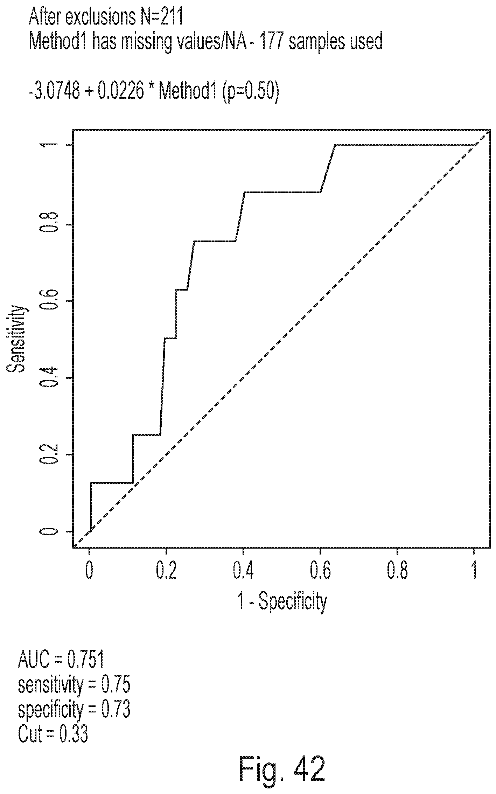

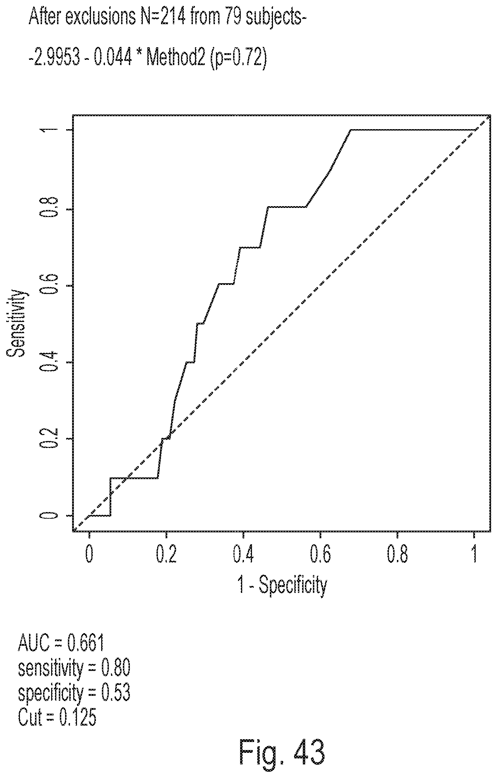

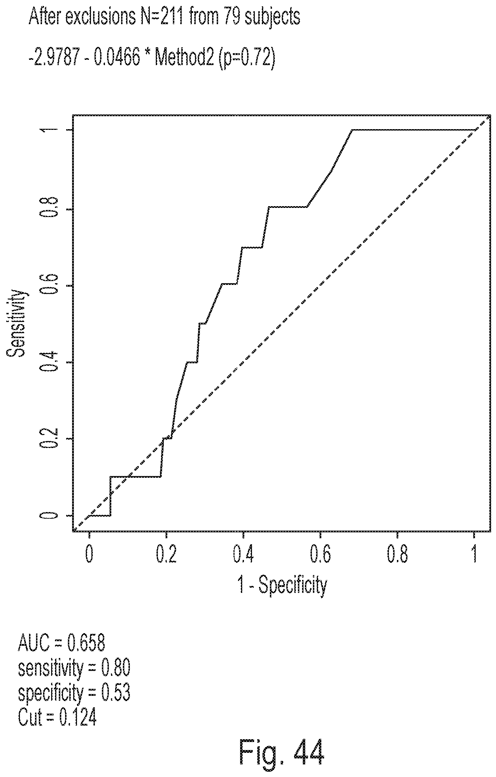

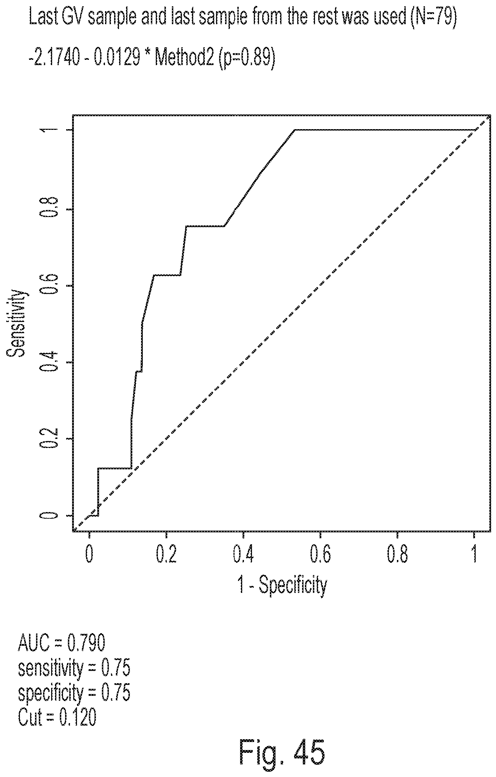

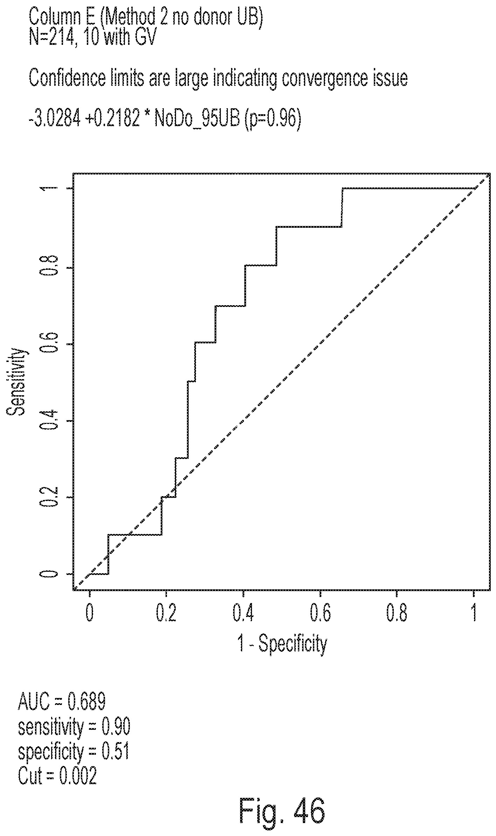

Additional examples are provided in **Figs. 41-46****.** Using the entire sample population (N=214), experimental thresholds were determined when the donor genotype was known **(****Fig. 41****)** and unknown **(****Fig. 43****).** The same thresholds were determined after excluding subjects on mechanical support from the data. The results are shown in **Fig. 42** and **Fig. 44****.** **Fig. 45** shows the results when only the final sample from each subject is used (N=79) (when the donor genotype was unknown).

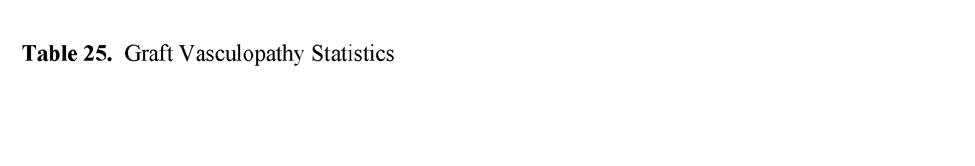

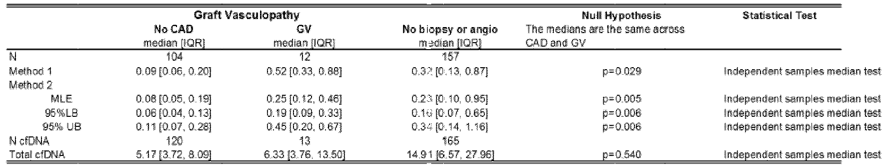

### Example 8 - Donor-specific Cell-free DNA (DS cf-DNA) Correlation with Cardiac Arrest

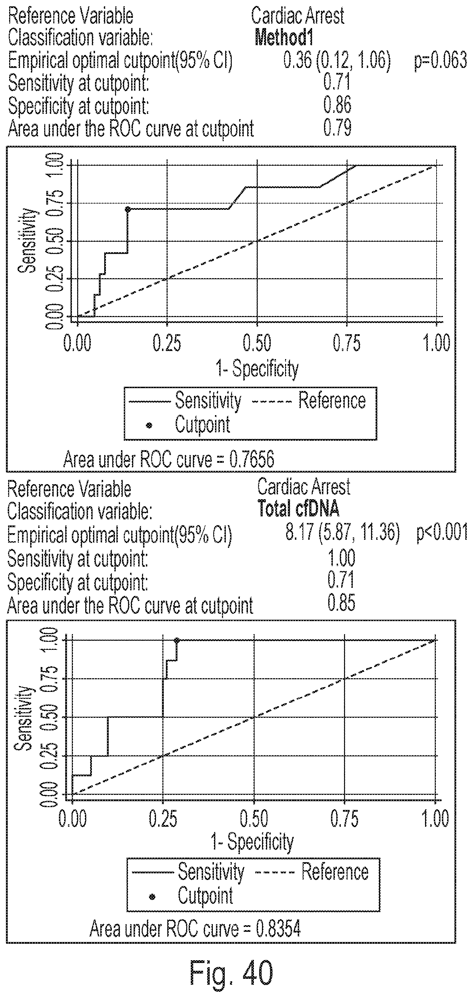

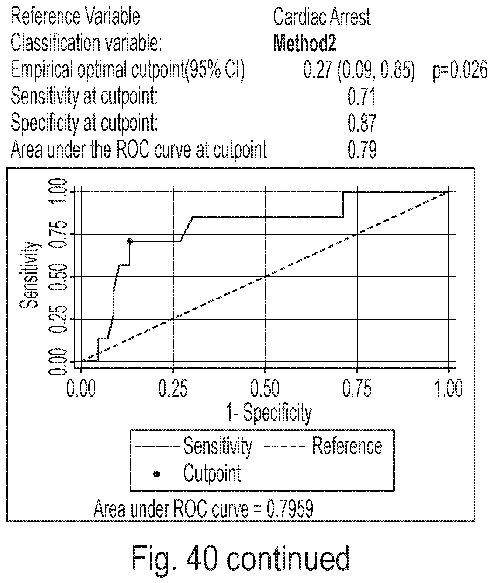

Likewise, the donor-specific cf-DNA of transplant recipients (n=71-77) was quantified using MOMA. As shown in **Fig. 40****,** threshold ("cutpoint") values for cardiac arrest were experimentally determined using two different methods (top row). Further, **Table 26** below shows additional statistics.

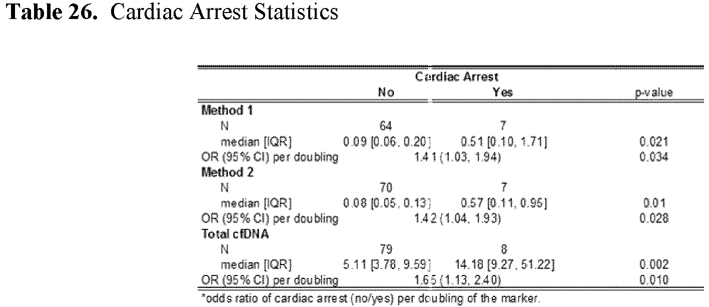

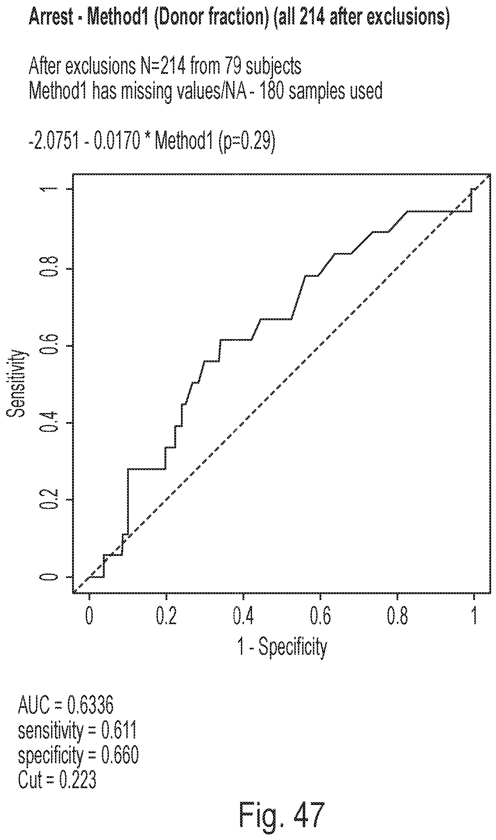

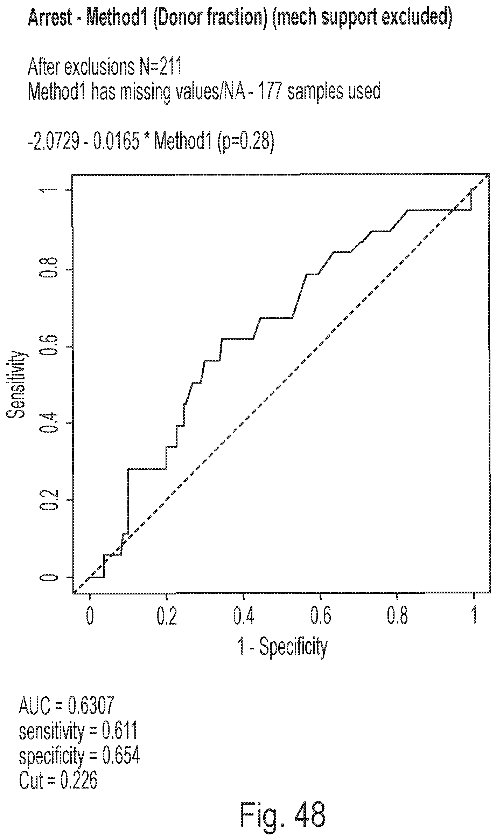

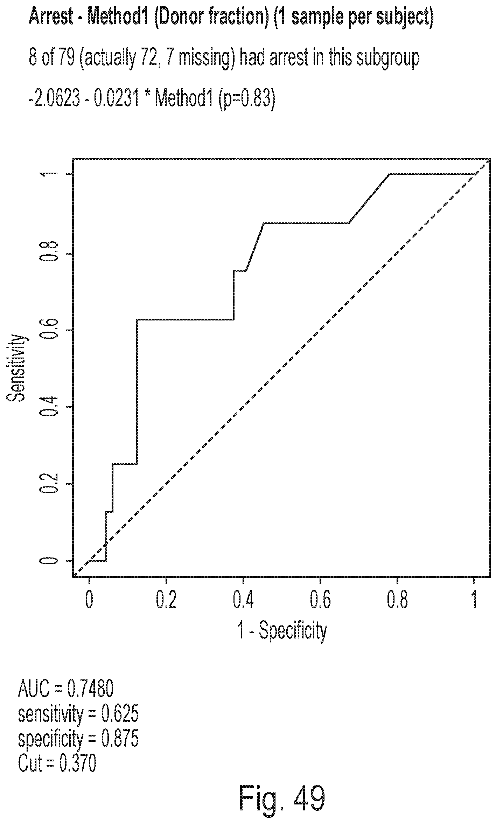

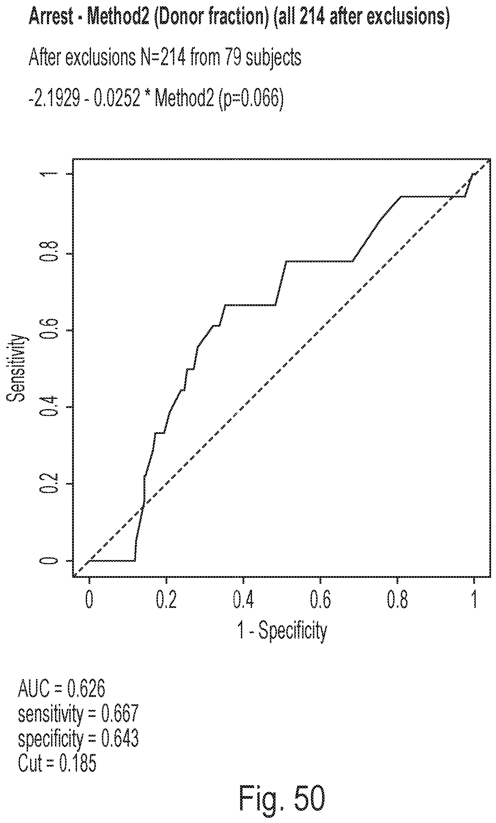

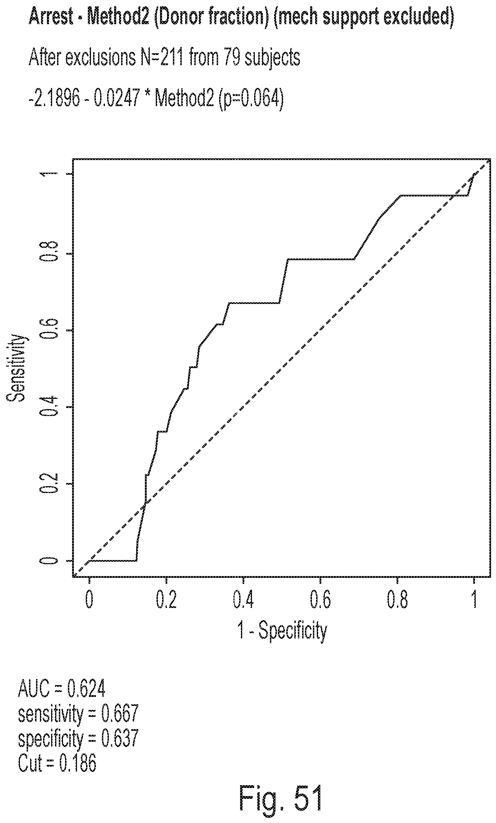

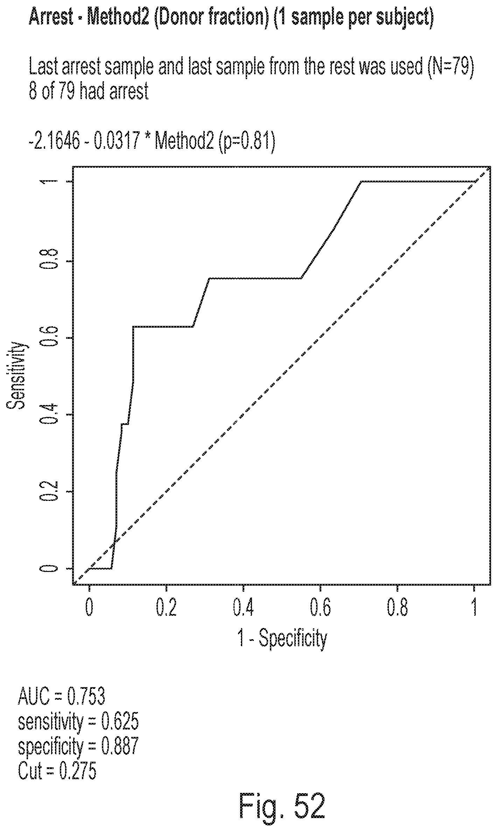

**Figs. 47-52** show the experimental determination of thresholds (cutpoints) for cardiac arrest found using different methods. **Figs. 47-49** show thresholds obtained using MOMA (with known donor genotype) in three populations: the full sample population **(****Fig. 47****),** the full sample population excluding those subjects on mechanical support **(****Fig. 48****),** and the final sample obtained from each subject **(****Fig. 49****).** **Figs. 50-52** show thresholds obtained using MOMA (with unknown donor genotype) in three populations: the full sample population **(****Fig. 50****),** the full sample population excluding those subjects on mechanical support **(****Fig. 51****),** and the final sample obtained from each subject **(****Fig. 52****).** The statistics for the tests are presented in **Table 27.**

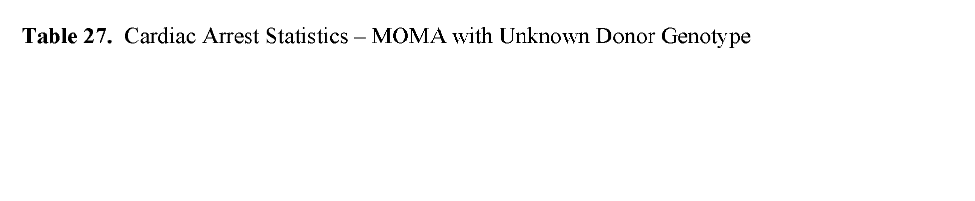

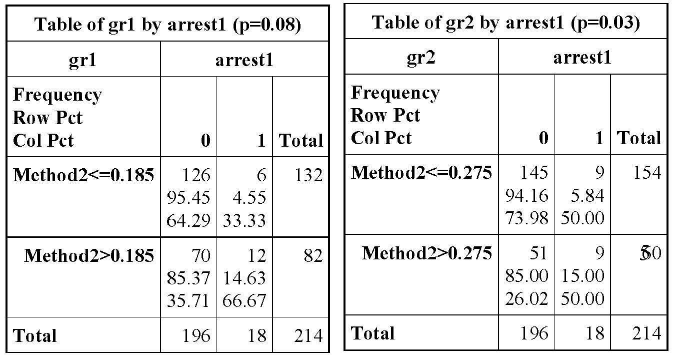

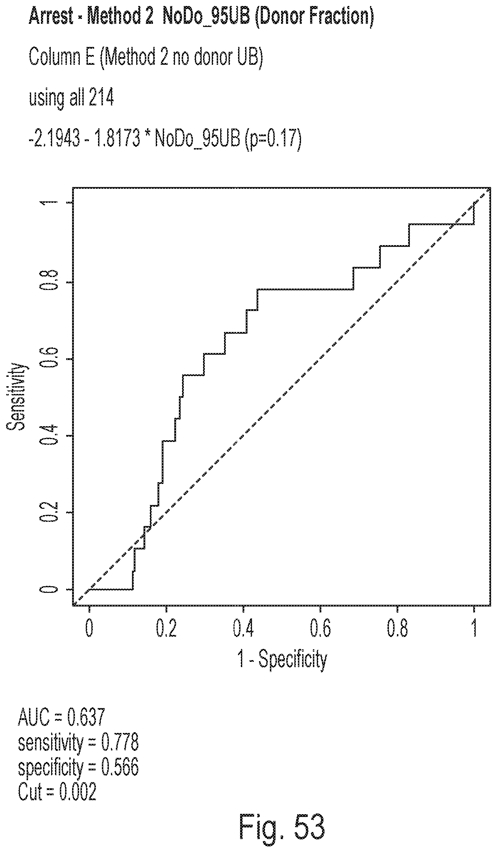

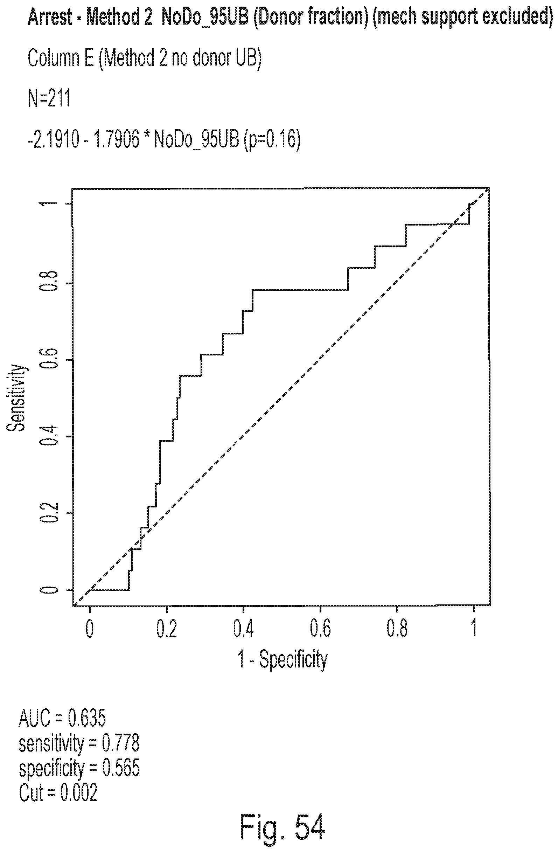

**Figs. 53** and **54** show additional results. In **Fig. 53****,** the full population of samples (N=214) was used. **Fig. 54** shows the results when the population was limited to samples from subjects who were not on mechanical support. Other results of the study are shown in **Table 28** below.

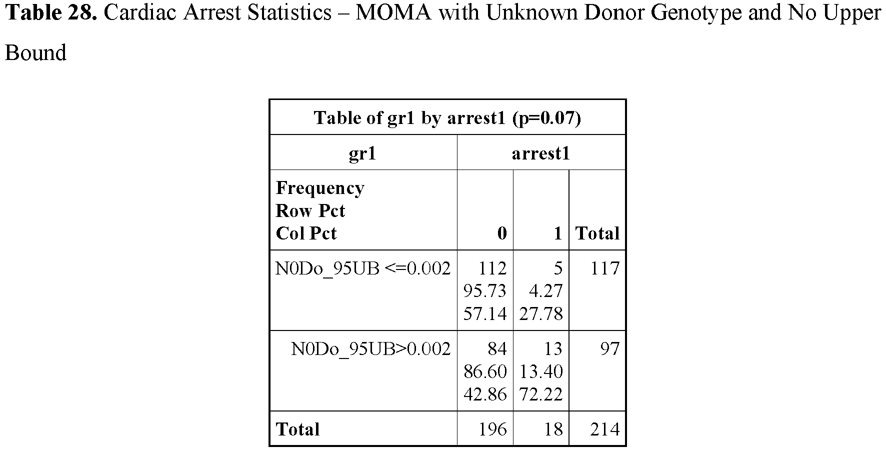

### Example 9 - Donor Specific Cell Free DNA as a Non-Invasive Indicator Following Cardiac Transplantation

### Objective

The current gold standard for surveillance of heart transplant recipients is endomyocardial biopsy (EMB) which puts children at particular risk for injury, limits the frequency of reasonable screening, and is inherently limited in sensitivity because rejection can occur as a patchy process. A non-invasive diagnostic screening tool for pediatric and adult heart transplant recipients based on the precise quantification of circulating donor specific cell-free DNA (DS cfDNA) was developed in order to replace the need for invasive surveillance biopsy.

### Methods

All cardiac transplant recipients followed at the Children's Hospital of Wisconsin (CHW) were invited to participate in this blinded study. Three to ten milliliters (ml) of anticoagulated blood were collected to assess circulating levels of cf-DNA in the following clinical scenarios: days 1,4,7, and 28 following transplant, within 24 hours prior to any EMB, days 1,4,7, and 28 after initiation of treatment for rejection. Samples were immediately coded, de-identified, and delivered to the laboratory for processing using the MyTAI(heart) test (TAI diagnostics, Milwaukee, WI). Clinical, laboratory, cardiac biopsy, angiography, catheterization and echocardiographic data were all recorded at the time of sample collection. The pathology report of all biopsy reports was reviewed and 2004 International Society for Heart and Lung Transplantation (ISHLT) grade was recorded. Dates and times of all critical events including treatment for infection, treatment for rejection, cardiac arrest, cardiac retransplantation, initiation of mechanical circulatory support, and death were recorded. The donor specific (ds) cell-free DNA (cf-DNA) was quantified using a targeted approached and compared to associated biopsy and angiography results using two distinct methods: with donor genotype (method 1) and without donor genotype (method 2).

### Results

### Cardiac Allograft Vasculopathy

A total of 158 samples from 87 unique transplant recipient subjects both adult and pediatric passed QC standards and were available for analysis. One individual participated in the study both after initial transplantation and after re-transplantation and was analyzed as two unique subjects given two unique donor/recipient mismatched DNA. Mean patient age at transplant was 7.9 +/- 7.5 years (range 0.03 to 24.2 years). Mean age at blood sample was 12.7 +/- 8.1 years (range 0.08 to 30.2 years). 59.6% (51/87) subjects were male, and 65.5% (57/87) were white. Mean time from transplant to blood sample was 4.8 +/- 4.2 years.

116 blood samples were collected within 24 hours prior to selective coronary angiography. Of these, 12 demonstrated graft vasculopathy as defined by the 2010 ISHLT grading system, and 104 showed no graft vasculopathy. Comparison of donor-specific cfDNA fractions among angiography-associated samples were summarized. Mean donor-specific fraction was 0.09% (IQR 0.06-0.20%) for samples not associated with CAV and 0.52% (IQR 0.33-0.88%) for samples associated with CAV (p=0.029). Empirical optimal cutpoint for ruling out CAV was 0.19% [95% CI 0.09-0.38% (p<0.001)].

As another example, 116 blood samples were collected within 24 hours prior to selective coronary angiography. Of these, 11 demonstrated graft vasculopathy as defined by the 2010 ISHLT grading system (Mehra et al., J Heart Lung Transplant 29, 717-727 (2010)), and 99 showed no graft vasculopathy. A comparison of donor-specific cf-DNA fractions among angiography-associated samples is summarized in **Table 29.**

Using MOMA, the mean donor fraction was 0.09% (IQR 0.06-0.20%) for samples not associated with CAV and 0.47% (IQR 0.27-0.71%) for samples associated with CAV (p=0.05) with donor genotype (Mehra, M.R., et al. International Society for Heart and Lung Transplantation working formulation of a standardized nomenclature for cardiac allograft vasculopathy-2010. J Heart Lung Transplant 29, 717-727 (2010). The empirical optimal cutpoint for ruling out CAV was 0.19% [95% CI 0.09-0.38% (p<0.001)].

Without donor genotype, the mean donor fraction was 0.27% (IQR 0.16-0.54%) for samples not associated with CAV and 0.55% (IQR 0.38-1.22%) for samples associated with CAV (p=0.057). The empirical optimal cutpoint for ruling out CAV was 0.37% [95% CI 0.24-0.57% (p<0.001)].

In yet another analysis, 116 blood samples were collected from 66 subjects within 24 hours before selective coronary angiography. Eleven samples demonstrated cardiac allograft vasculopathy (CAV) (seven grade 1, two grade 2, and three grade 3), and 105 showed no CAV. Using Method 1 (with known donor genotype), DF was 0.09% (IQR: 0.06% to 0.20%) for samples not associated with CAV and 0.47% (IQR: 0.27% to 0.71%) for CAV-associated samples (p = 0.05). Using Method 2 (with unknown donor genotype), DF was 0.27% (IQR: 0.16% to 0.52%) for samples not associated with CAV and 0.55% (IQR: 0.38% to 1.22%) for CAV-associated samples (p = 0.057).

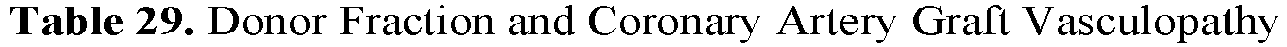

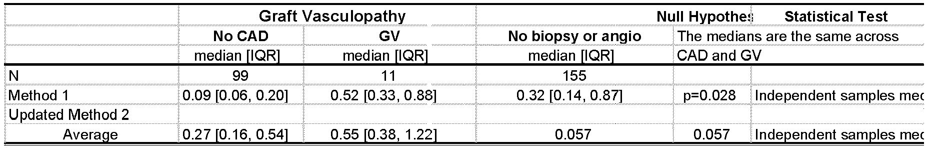

### Acute Cellular Rejection

In another analysis, blood samples from 88 subjects before endomycardial biopsy (EMB) were obtained. Donor fraction (DF) was reported as a percentage of total circulating recipient cfDNA. The mean age at blood sample was 12.7 ± 8.1 years (range, 0.1 to 30.2 years). Fundamental cardiac diagnosis was cardiomyopathy in 42.0% and congenital heart disease in 56.8%. A total of 59.0% of the subjects were male and 69.3% (61 of 88) were Caucasian. Among 158 biopsy-associated samples (148 of which were asymptomatic surveillance biopsies), 134 were associated with cellular rejection grade 0 (CR0), 21 with cellular rejection grade 1 (CR1), 3 with cellular rejection grade 2 (CR2), and 0 with cellular rejection grade 3 (CR3).

Using Method 1 (with known donor genotype), DF increased across rejection grades: 0.11% (interquartile range [IQR]: 0.06% to 0.21%) in CR0-associated samples, 0.37% (IQR: 0.15% to 0.72%) in CR1-associated samples, and 0.97% (IQR: 0.88% to 1.06%) in CR2-associated samples. Comparing CR0 (0.11%; IQR: 0.06% to 0.21%) to CR1 or CR2 (0.48%; IQR: 0.19% to 0.89%), p = 0.02.

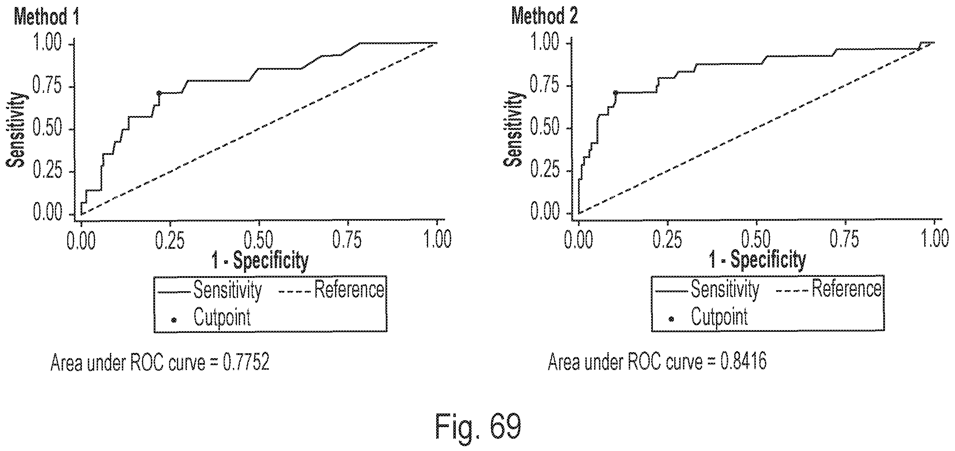

Using Method 2 (with unknown donor genotype), DF also increased across rejection grades: 0.25% (IQR: 0.17% to 0.39%) in CR0-associated samples, 0.89% (IQR: 0.44% to 5.35%) in CR1-associated samples, and 1.22% (IQR: 1.04% to 5.18%) in CR2-associated samples. Comparing CR0 (0.25%; IQR: 0.19% to 0.39%) to CR1 or CR2 (1.05%; IQR: 0.47% to 5.26%), p < 0.001. Receiver-operating characteristic curves with optimal cutpoints were determined and are presented in **Fig. 69****.**

### Association of Acute Cellular Rejection and Cardiac Allograft Vasculopathy at Specified Cutpoints

The association with acute cellular rejection and cardiac allograft vasculopathy at specified DF cutpoints was analyzed (**Table 30**). All 140 samples were analyzed by Method 1 (with known donor genotype) at a DF cutpoint of 0.2% and CR0 was compared to CR1 and CR2 (p = 0.0022). All 158 samples were analyzed by Method 2 (with unknown donor genotype) at a DF cutpoint of 0.2% and CR0 was compared to CR1 and CR2 (p = 0.0141). The 102 samples associated with angiography at a DF cutpoint of 0.2% were analyzed using Method 1 (with known donor genotype); 7 samples were true positives for CAV, 1 sample was a false negative, 70 were true negatives, and 24 were false positives (p < 0.001). Using Method 2 (with unknown donor genotype), 116 samples were analyzed at a DF cutpoint of 0.2%, and 11 samples were true positive for CAV, 0 were false negative, 38 were true negative, and 67 were false positive (p = 0.015).

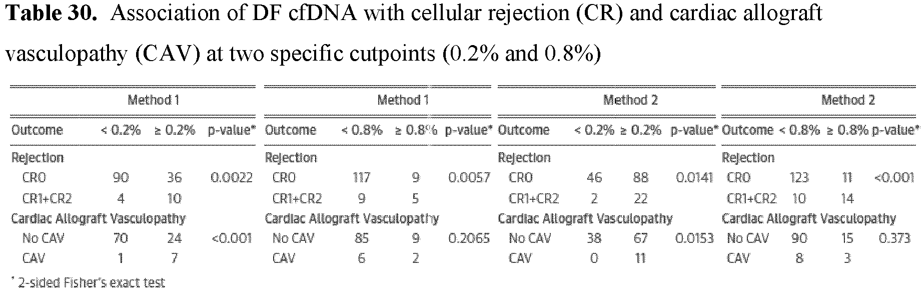

### Conclusions

It was found that use of a targeted, high-throughput assay for the quantification of donor-specific cf-DNA has exquisite sensitivity for surveillance in heart transplant recipients and for ruling out the presence of acute cellular rejection in heart transplant recipients. DF increased from CR0- to CR1- to CR2-associated biopsies, suggesting its ability to detect progressive injury to the donor organ. Additionally, marked elevations in donor specific fraction correlate to significant allograft injury, including acute episodic rejection and chronic rejection in the form of coronary artery graft vasculopathy. These findings suggest that precise quantification of DF is possible in clinical practice and that the observed similarity in results between both assay methods (with known or with unknown donor genotypes) demonstrate that accuracy in quantification is preserved even when donor DNA is not available for genotyping.

### Example 10 - Donor-specific Cell-free DNA (DS cf-DNA) Correlation with Antibody-mediated Rejection

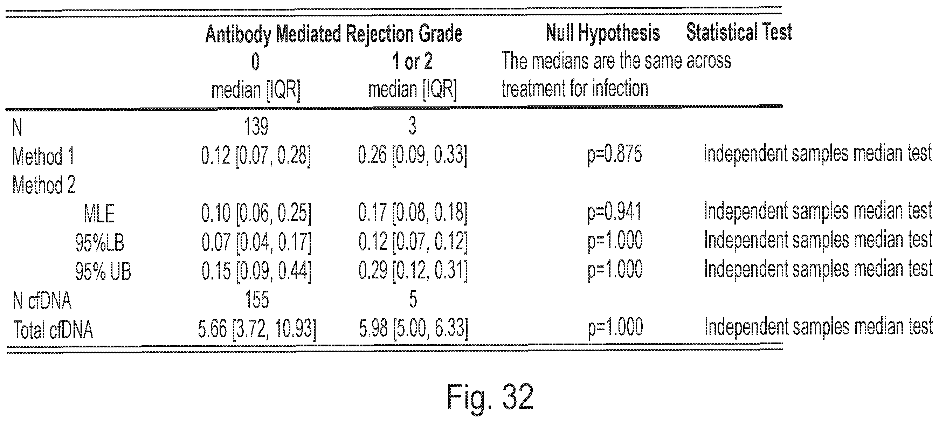

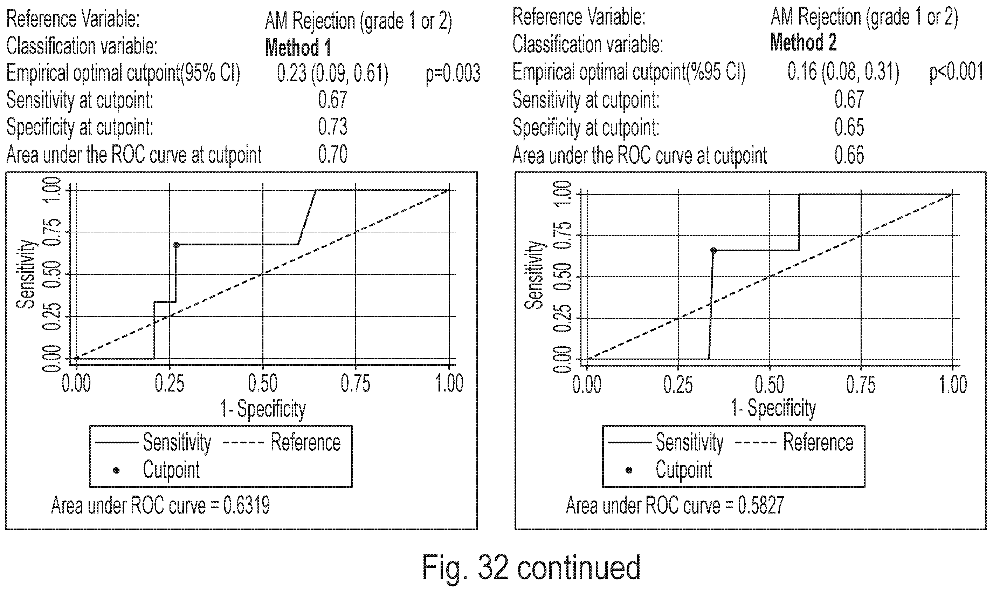

The donor-specific cf-DNA of transplant recipients (n=142) was quantified using MOMA. As shown in **Fig. 32****,** threshold ("cutpoint") values for antibody-mediated rejection grade 0 and grades 1 and 2 were experimentally determined.

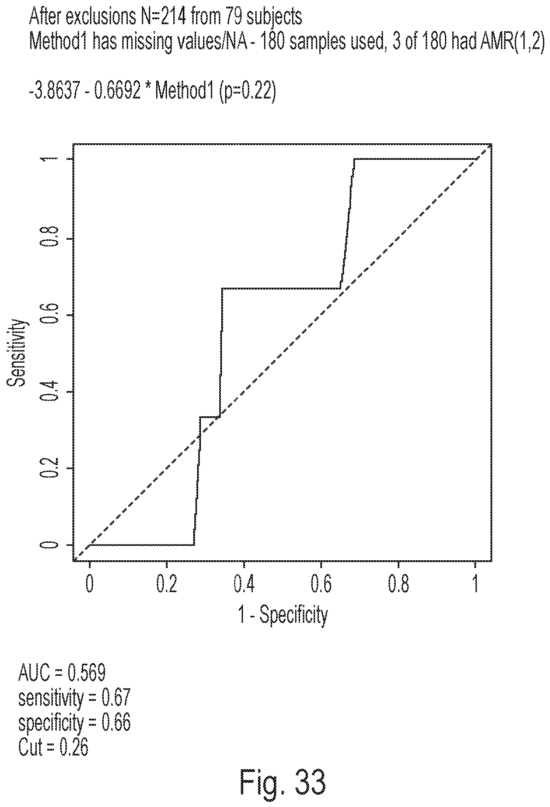

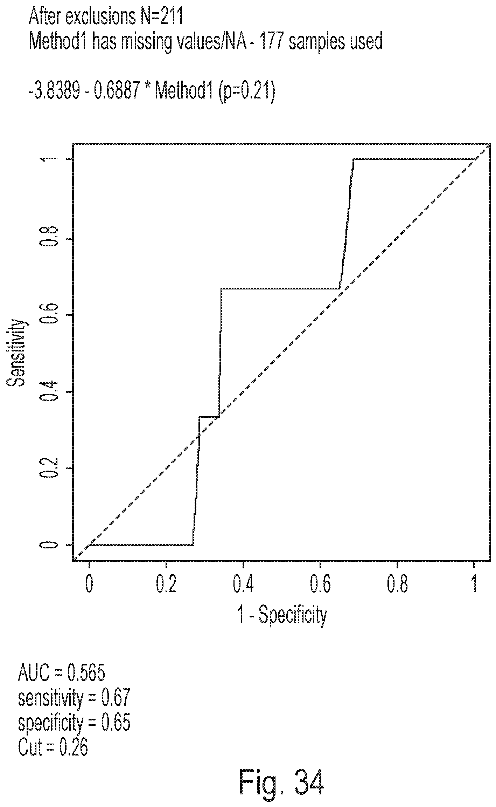

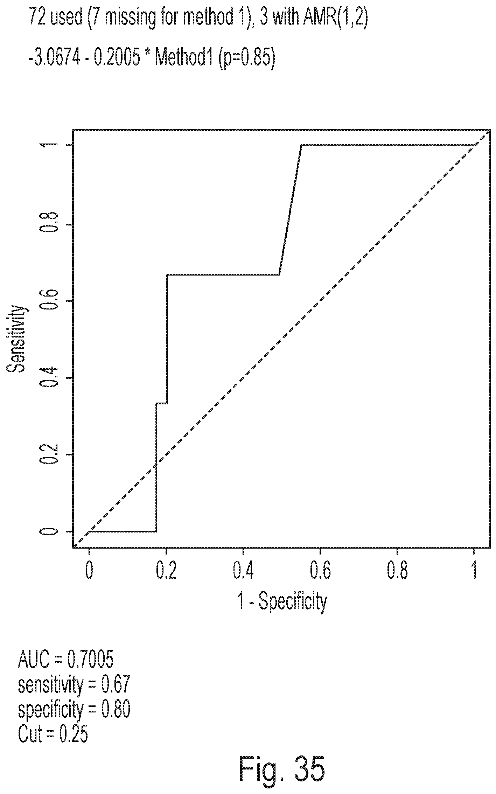

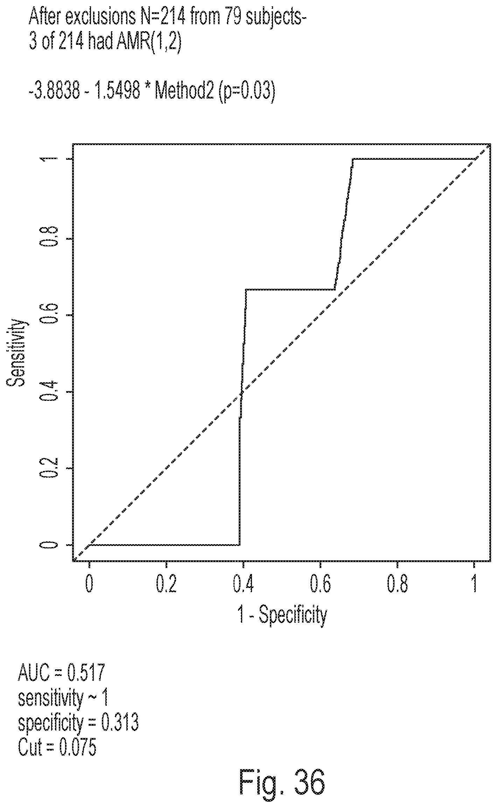

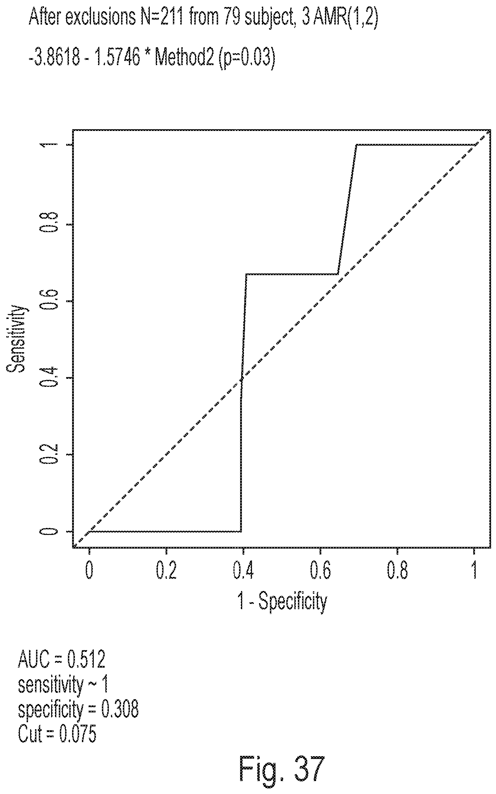

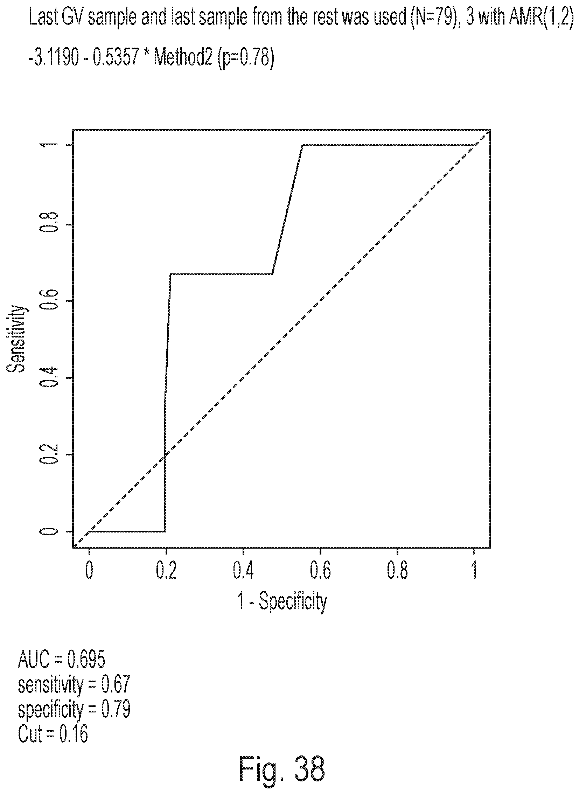

Further examples of threshold (cutpoint) determinations are shown in **Figs. 33-38****.** **Figs. 33** and **36** show the determination of a threshold using 214 samples, with the donor genotype known **(****Fig. 33****)** and unknown **(****Fig. 36****).** The same samples were analyzed, excluding the population using mechanical support, and the results are shown in **Figs. 34** and **37****.** Examining only the final sample from each subject (n=79) yielded a cutpoint of 0.25 when the donor genotype was known **(****Fig. 35****)** and 0.16 **(****Fig. 38****)** when the donor genotype was not known.

In another example, the mean donor-specific fraction was 0.12% (IQR 0.07-0.29%) for samples associated with grade pAMR0 and was 0.26% (IQR 0.09-0.33%) for samples associated with grade pAMR1 or 2 (p=0.905) when the donor genotype was known. When the donor genotype was not known, the mean donor fraction was 0.29% (IQR 0.18-0.61%) for samples associated with grade pAMR0 and was 0.39 (IQR 0.12-0.44%) for samples associated with grade pAMR1 or 2 (p=0.969). The empirical optimal cutpoint for ruling out pAMR1 or 2 based on the associated ROC curve was 0.38% [95%CI 0.19-0.74% (p=0.005)].

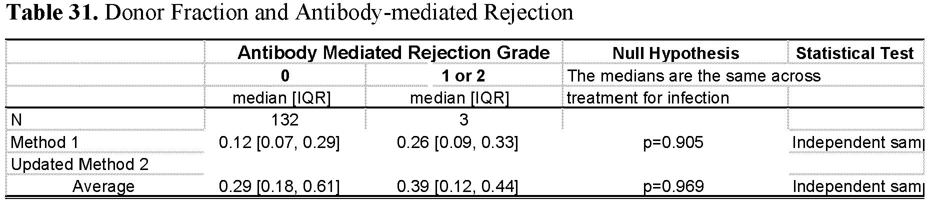

### Example 11 - Endomyocardial Biopsy Induces Quantifiable Cellular Injury

Paired blood samples from 21 asymptomatic patients were drawn pre- and postsurveillance biopsy (bx). Quantitative DF cfDNA was determined using the myTAI-HEART^{™} test (a proprietary quantitative genotyping assay from TAI Diagnostics, Wauwatosa, WI). Excluding patients with known graft vasculopathy, cancer, mechanical circulatory support, or any cellular rejection with grade >1, 17 sample pairs were available. Bioptome size and number of bx samples taken were recorded and analyzed.

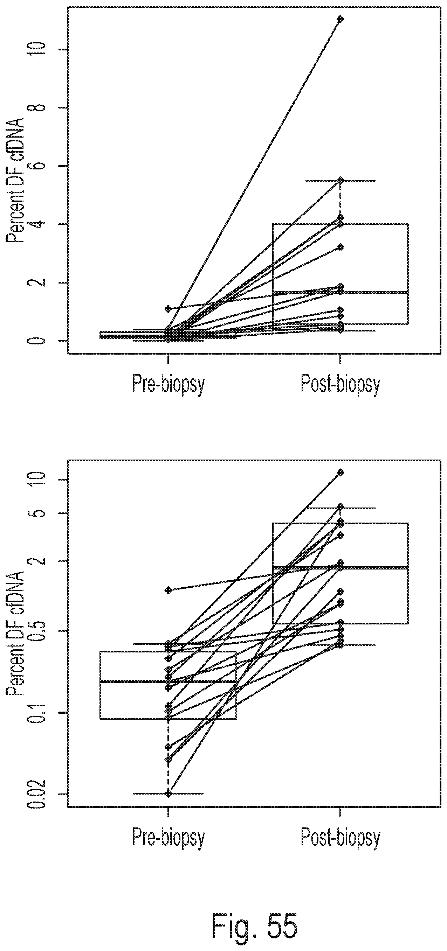

Donor fraction (DF) ranged from 0.02% pre-bx through 11.1% post-bx with a median of 0.43%. The DF consistently increased post-bx, with a median increase of 8.2x (range 1.5x - 213x). Patient ages ranged from 4 to 32 years (median, 12 years). Patient weights ranged from 17 to 90 kg (median, 49 kg). Both age and weight are independently associated with DF change (p<0.01). Pts at or under 16 years of age had an average DF increase of 24x versus pts at or above age 24 with an average DF increase of 2.7x. Age and weight were correlated, thus similar effects were seen by weight at time of draw. DF change did not correlate with bioptome size (p=0.4). The time between bx and the second blood draw ranged from 1 to 36 minutes and was not correlated to DF increase. Samples drawn soon after bx had more dramatic DF increases than more delayed samples consistent with the short half-life of cfDNA; the five fastest (mean 2 minutes) had an average DF increase of 19.8x, versus the five latest (mean 7.6 minutes) saw just 4.1x. Initial DF is indicative of organ health before bx. A patient with elevated DF (>0.9%) pre-biopsy saw less DF increase post bx (p<0.01). The data is shown in **Fig. 55****.**

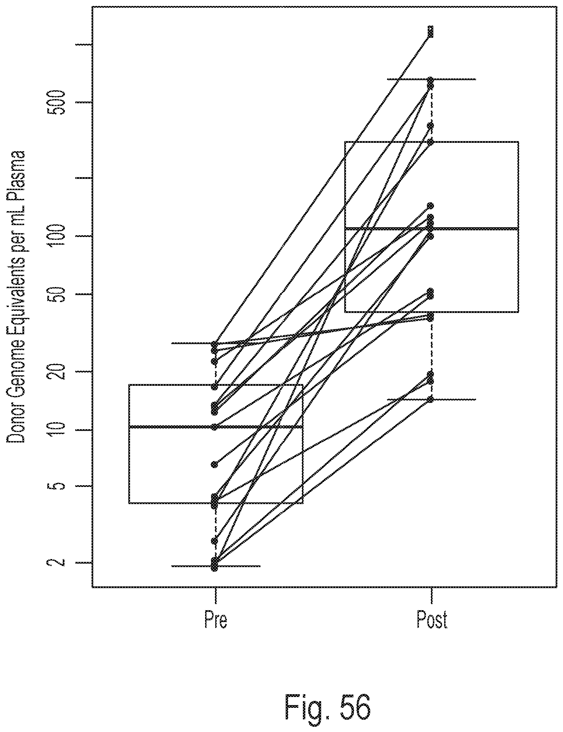

In another analysis, the quantity of ds-cfDNA was reported as donor genomic equivalents (GE) and ranged from 1.9 (median, 12) pre-biopsy through 1200 (median, 136) post-biopsy. Paired samples are shown in **Fig. 56****.** The GE of ds-cfDNA increased post-biopsy in all patients (p<0.02), with a median increase of 8.2x (range, 0.34x-345x). Patient ages ranged from 4 to 32 years (median, 12 years). Patient weights ranged from 17 to 90 kg (median, 49 kg). Both age and weight were associated with GE change (p<0.01). Patients under 17 years of age had an average GE increase of 29x compared to patients older than 23, which saw an average GE increase of 1.1x. Age and weight were found to be correlated, thus similar effects were seen by weight at time of draw. GE change did not correlate with bioptome size (p=4). The time between biopsy and the second blood draw ranged from 1 to 36 minutes and increased time correlated to increased total cell-free DNA (p=0.037). Initial ds-cfDNA in GE is indicative of organ health before biopsy. Patients with elevated GE (>20, n=4) pre-biopsy, saw less GE increase post-biopsy (p<0.01).

Standard endomyocardial biopsy was found to induce a significant and measurable injury to the transplanted heart, influenced strongly by patient body size and pre-biopsy level of ds-cfDNA. Longer times between the biopsy and blood sample correlated with increased total cell-free DNA. This serves as evidence of the sensitivity of ds-cfDNA as a marker of cardiac injury.

## Claims

1. A method of assessing a sample from a transplant subject, the method comprising:
(a) determining an amount of donor-specific cell-free DNA (DS cf-DNA) in a sample from the subject, wherein the subject has or is suspected of having a cellular rejection grade, such as of CR1, CR2 or lower, CR1 or greater, or CR2 or greater, and wherein the amount of DS cf-DNA is determined by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target; and
(b) reporting and/or recording the amount of DS cf-DNA, wherein
a cellular rejection grade of CR0 is assigned to the subject if the amount of DS cf-DNA is less than 0.2;
a cellular rejection grade of CR1 is assigned to the subject if the amount of DS cf-DNA is between 0.2 and 0.8; or
a cellular rejection grade of CR2 or greater is assigned to the subject if the amount of DS cf-DNA is greater than 0.8,
optionally wherein the method further comprises:
(c) comparing the amount of DS cf-DNA to a threshold DS cf-DNA or at least one prior DS-cf-DNA value and/or wherein the method further comprises:
(d) assigning a cellular rejection grade for the subject, or as being at increased risk, based on the determined amount of DS cf-DNA compared to the threshold DS cf-DNA value and/or at least one prior DS cf-DNA amount.

2. A method of determining a treatment or monitoring regimen for a transplant subject, the method comprising:
(a) obtaining an amount of donor-specific cell-free DNA (DS cf-DNA) in a sample that has been obtained from the subject, wherein the subject has or is suspected of having a cellular rejection grade, such as of CR1, CR2 or lower, CR1 or greater, or CR2 or greater and wherein the amount of DS cf-DNA is obtained by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target;
(b) comparing the amount of DS cf-DNA to a threshold DS cf-DNA value and/or at least one prior DS cf-DNA amount; and
(c) determining a treatment or monitoring regimen for the subject based on the determined amount of DS cf-DNA compared to the threshold DS cf-DNA value and/or at least one prior DS cf-DNA amount, wherein
a cellular rejection grade of CR0 is assigned to the subject if the amount of DS cf-DNA is less than 0.2;
a cellular rejection grade of CR1 is assigned to the subject if the amount of DS cf-DNA is between 0.2 and 0.8; or
a cellular rejection grade of CR2 or greater is assigned to the subject if the amount of DS cf-DNA is greater than 0.8,
optionally wherein the method further comprises assigning a cellular rejection grade to the subject based on the determined amount of DS cf-DNA compared to the threshold DS cf-DNA value and/or at least one prior DS cf-DNA amount.

3. The method of any one of the preceding claims, wherein:
(i) the subject has or is suspected of having a cellular rejection grade, such as of CR0 or CR1 or such as CR2 or CR3; and/or
(ii) the subject has been determined to have a cellular rejection grade, such as of CR1, CR2 or lower, CR1 or greater, or CR2 or greater; and/or
(iii) wherein the subject has been determined to have a cellular rejection grade, such as of CR0 or CR1 or such as CR2 or CR3; and/or
(iv) the subject was determined to have the cellular rejection grade with one or more additional test(s) prior applied to assess transplant rejection in the subject or the method further comprises performing one or more additional test(s) to assess transplant rejection in the subject; and/or
(v) a cellular rejection grade of CR0 is assigned to the subject if the amount DS cf-DNA is less than 0.14, 0.15; and/or
(vi) one or more additional test(s) is indicated if the amount of DS cf-DNA is between 0.14 and 0.5, 0.15 and 0.5, 0.2 and 0.5 or 0.3 and 0.5.

4. A method of assessing a sample from a transplant subject, the method comprising:
(a) determining an amount of donor-specific cell-free DNA (DS cf-DNA) in a sample from the subject, wherein the subject has, is suspected of having, has had, or is at risk of having a condition or disease of the transplanted organ, wherein the condition or disease of the transplanted organ is cardiac allograft vasculopathy and/or risk of cardiac arrest, and wherein the amount of DS cf-DNA is determined by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target; and
(b) reporting and/or recording the amount of DS cf-DNA, wherein
cardiac allograft vasculopathy and/or risk of cardiac arrest is indicated if the amount of DS cf-DNA is greater than 0.2,
optionally wherein the method further comprises:
(c) comparing the amount of DS cf-DNA to a threshold DS cf-DNA value or at least one prior DS cf-DNA value, and/or wherein the condition or disease is indicated, or the subject is at increased risk, based on the determined amount of DS cf-DNA compared to the threshold DS cf-DNA value and/or at least one prior DS cf-DNA amount.

5. A method of determining a treatment or monitoring regimen for a transplant subject, the method comprising:
(a) obtaining an amount of donor-specific cell-free DNA (DS cf-DNA) in a sample that has been obtained from the subject, wherein the subject has, is suspected of having, has had, or is at risk of having a condition or disease of the transplanted organ, wherein the condition or disease of the transplanted organ is cardiac allograft vasculopathy and/or risk of cardiac arrest, and wherein the amount of DS cf-DNA is obtained by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target;
(b) comparing the amount of DS cf-DNA to a threshold DS cf-DNA value and/or at least one prior DS cf-DNA amount; and
(c) determining a treatment or monitoring regimen for the subject based on the determined amount of DS cf-DNA compared to the threshold DS cf-DNA value and/or at least one prior DS cf-DNA amount, wherein
cardiac allograft vasculopathy and/or risk of cardiac arrest is indicated if the amount of DS cf-DNA is greater than 0.2,
optionally wherein the condition or disease is indicated, or the subject is at increased risk, based on the determined amount of DS cf-DNA compared to the threshold DS cf-DNA value and/or at least one prior DS cf-DNA amount.

6. The method of claim 4 or 5, wherein:
(i) the subject has had or is at risk of having cardiac arrest; and/or
(ii) the subject has been determined to have cardiac allograft vasculopathy or has had a cardiac arrest; and/or
(iii) the subject was determined to have cardiac allograft vasculopathy with one or more additional test(s) prior applied to assess the subject or the method further comprises performing one or more additional test(s) to assess the subject; and/or
(iv) cardiac graft vasculopathy or risk of cardiac arrest is indicated if the amount DS cf-DNA is equal to or greater than 0.3.

7. A method of assessing a sample from a transplant subject, the method comprising:
(a) determining an amount of donor-specific cell-free DNA (DS cf-DNA) in a sample from the subject, wherein the subject has or is suspected of having antibody-mediated rejection and wherein the amount of DS cf-DNA is determined by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target; and
(b) reporting and/or recording the amount of DS cf-DNA, wherein
the subject is classified as having antibody-mediated rejection if the amount of DS cf-DNA is greater than 0.2,
optionally wherein the method further comprises:
(c) comparing the amount of DS cf-DNA to a threshold DS cf-DNA value or at least one prior DS cf-DNA value and/or wherein the method further comprises:
(d) classifying the subject as having antibody-mediated rejection, or at an increased risk, based on the determined amount of DS cf-DNA compared to the threshold DS cf-DNA value and/or at least one prior DS cf-DNA amount.

8. A method of determining a treatment or monitoring regimen for a transplant subject, the method comprising:
(a) obtaining an amount of donor-specific cell-free DNA (DS cf-DNA) in a sample that has been obtained from the subject, wherein the subject has or is suspected of having antibody-mediated rejection and wherein the amount of DS cf-DNA is obtained by: for a plurality of single nucleotide variant (SNV) targets, performing an amplification-based quantification assay, such as a polymerase chain reaction (PCR) quantification assay, on the sample, or a portion thereof, with at least two primer pairs, wherein each primer pair comprises a forward primer and a reverse primer, wherein one of the at least two primer pairs comprises a 3' penultimate mismatch in a primer relative to one allele of the SNV target, but a 3' double mismatch relative to another allele of the SNV target and specifically amplifies the one allele of the SNV target, and another of the at least two primer pairs specifically amplifies to another allele of the SNV target;
(b) comparing the amount of DS cf-DNA to a threshold DS cf-DNA value and/or at least one prior DS cf-DNA amount; and
(c) determining a treatment or monitoring regimen for the subject based on the determined amount of DS cf-DNA compared to the threshold DS cf-DNA value and/or at least one prior DS cf-DNA amount, wherein,
the subject is classified as having antibody-mediated rejection if the amount of DS cf-DNA is greater than 0.2,
optionally wherein the method further comprises classifying the subject as having antibody-mediated rejection, or at an increased risk, based on the determined amount of DS cf-DNA compared to the threshold DS cf-DNA value and/or at least one prior DS cf-DNA amount.

9. The method of claim 7 or 8, wherein:
(i) the subject has been determined to have antibody-mediated rejection; and/or
(ii) the subject was determined to have antibody-mediated rejection with one or more additional test(s) prior applied to assess transplant rejection in the subject or the method further comprises performing one or more additional test(s) to assess the subject.

10. The method of any one of the preceding claims, wherein:
(i) the one or more additional tests comprises a biopsy, optionally wherein the biopsy is endomyocardial biopsy (EMB); and/or
(ii) the amount is provided in a report, optionally wherein the amount is provided in a report that also contains at least one prior amount of DS-cfDNA that was in a sample from the subject; and/or
(iii) the amount is recorded in a database, optionally wherein the database also contains at least one prior amount of DS cf-DNA that was in a sample from the subject.

11. The method of any one of the preceding claims, wherein the genotype of the donor is known or wherein the genotype of the donor is unknown.

12. The method of any one of the preceding claims, wherein:
(i) at least one amount is determined in a sample taken from the subject 4, 5, 6, 7, or 8 days following an organ transplant; and/or
(ii) an amount of DS cf-DNA that is greater than the threshold value and/or is increased relative to the amount from an earlier time point represents an increased or increasing risk; and/or
(iii) an amount of DS cf-DNA that is lower than the threshold value and/or is decreased relative to the amount from an earlier time point represents a decreased or decreasing risk; and/or
(iv) the determining a monitoring regimen comprises determining the amount of DS cf-DNA in the subject over time or at a subsequent point in time, or suggesting such monitoring to the subject; optionally wherein the subject is assessed daily or weekly or wherein the subject is assessed monthly or bimonthly; and/or
(v) the subject is assessed for up to 6 months, up to 8 months, up to 10 months, or up to one year; and/or
(vi) the time between samples is decreased if the amount of DS cf-DNA is increased relative to the threshold or an amount from an earlier time point; and/or
(vii) the additional amount(s) of DS cf-DNA are recorded or reported, optionally wherein the additional amount(s) are provided in a report and/or wherein the additional amount(s) are recorded in a database; and/or
(viii) the determining a monitoring regimen comprises using or suggesting the use of one or more additional test(s) to assess transplant rejection in the subject, optionally wherein the one or more additional test(s) comprises a biopsy, optionally wherein the biopsy is endomyocardial biopsy (EMB); and/or
(ix) the one or more additional test(s) is indicated if the amount of DS cf-DNA is equal to or greater than any one of the thresholds provided herein; and/or
(x) the determining a treatment regimen comprises selecting or suggesting a treatment for the subject; and/or
(xi) the determining a treatment regimen comprises treating the subject; and/or
(xii) the treatment comprises an anti-rejection therapy; and/or
(xiii) the treatment comprises a treatment for cardiac allograft vasculopathy, such as retransplantation, percutaneous coronary interventions (PCI), coronary artery bypass grafting (CABG), transmyocardial laser revascularization or heparin-induced/mediated extracorporeal LDL plasmapheresis (HELP); and/or
(xiv) when cardiac allograft vasculopathy is indicated, the treatment comprises treatment with a statin, antihypertensive agent or anti-cytomegalovirus (anti-CMV) agent, optionally wherein the antihypertensive agent is a calcium channel blocker; and/or
(xv) when increased risk of cardiac arrest is indicated, the treatment comprises treatment for cardiac arrest, such as percutaneous coronary intervention (coronary angioplasty), coronary artery bypass grafting, or addition of an implantable cardioverter defibrillator (ICD); and/or
(xvi) when increased risk of cardiac arrest is indicated, the treatment comprises an anti-arrhythmic agent, an involuntary nervous system blocker, or blood pressure medication; and/or
(xvii) the determining a treatment regimen comprises providing information about a treatment to the subject; and/or
(xviii) the sample is a blood, plasma or serum sample, optionally wherein the blood sample is a plasma sample; and/or
(xix) the transplant subject is a heart transplant subject.

## Patentansprüche

1. Verfahren zum Bewerten einer Probe von einem Transplantationssubjekt, das Verfahren umfassend:
(a) Bestimmen einer Menge an spenderspezifischer zellfreier DNA (DS cf-DNA) in einer Probe von dem Subjekt, wobei das Subjekt einen zellulären Abstoßungsgrad wie CR1, CR2 oder niedriger, CR1 oder höher oder CR2 oder höher aufweist oder diese Verdacht besteht, und wobei die Menge an DS cf-DNA bestimmt wird durch: für eine Vielzahl von Einzelnukleotidvarianten-(SNV-)Zielen Durchführen eines amplifikationsbasierten Quantifizierungsassays, wie eines Polymerase-Kettenreaktions- (PCR-)Quantifizierungsassays, an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3'-Ende relativ zu einem anderen Allel des SNV-Ziels umfasst und spezifisch das eine Allel des SNV-Ziels amplifiziert und ein anderes der zumindest zwei Primerpaare spezifisch zu einem anderen Allel des SNV-Ziels amplifiziert; und
(b) Berichten und/oder Aufzeichnen der Menge an DS cf-DNA, wobei
dem Subjekt ein zellulärer Abstoßungsgrad von CR0 zugewiesen wird, falls die Menge an DS cf-DNA weniger als 0,2 beträgt;
dem Subjekt ein zellulärer Abstoßungsgrad von CR1 zugewiesen wird, falls die Menge an DS cf-DNA zwischen 0,2 und 0,8 beträgt; oder
dem Subjekt ein zellulärer Abstoßungsgrad von CR2 oder größer zugewiesen wird, falls die Menge an DS cf-DNA größer als 0,8 ist,
wobei das Verfahren optional ferner Folgendes umfasst:
(c) Vergleichen der Menge an DS cf-DNA mit einer Schwellen-DS cf-DNA oder zumindest einem vorherigen DS-cf-DNA-Wert und/oder wobei das Verfahren ferner Folgendes umfasst:
(d) Zuweisen eines zellulären Abstoßungsgrads für das Subjekt oder dass es ein erhöhtes Risiko hat, basierend auf der bestimmten Menge an DS cf-DNA im Vergleich zu dem Schwellen-DS cf-DNA-Wert und/oder zumindest einer vorherigen DS cf-DNA-Menge.

2. Verfahren zum Bestimmen eines Behandlungs- oder Überwachungsschemas für ein Transplantationssubjekt, das Verfahren umfassend:
(a) Erhalten einer Menge an spenderspezifischer zellfreier DNA (DS cf-DNA) in einer Probe, die von dem Subjekt erhalten wurde, wobei das Subjekt einen zellulären Abstoßungsgrad, wie CR1, CR2 oder niedriger, CR1 oder größer oder CR2 oder größer, aufweist oder dieser Verdacht besteht, und wobei die Menge an DS cf-DNA erhalten wird durch: für eine Vielzahl von Einzelnukleotidvarianten- (SNV-)Zielen Durchführen eines amplifikationsbasierten Quantifizierungsassays, wie eines Polymerase-Kettenreaktions- (PCR-)Quantifizierungsassays, an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3'-Ende relativ zu einem anderen Allel des SNV-Ziels umfasst und spezifisch das eine Allel des SNV-Ziels amplifiziert und ein anderes der zumindest zwei Primerpaare spezifisch zu einem anderen Allel des SNV-Ziels amplifiziert;
(b) Vergleichen der Menge an DS cf-DNA mit einem Schwellen-DS cf-DNA-Wert und/oder zumindest einer vorherigen DS cf-DNA-Menge;
(c) Bestimmen eines Behandlungs- oder Überwachungsschemas für das Subjekt basierend auf der bestimmten Menge an DS cf-DNA im Vergleich zu dem Schwellen-DS cf-DNA-Wert und/oder zumindest einer vorherigen DS cf-DNA-Menge, wobei
dem Subjekt ein zellulärer Abstoßungsgrad von CR0 zugewiesen wird, falls die Menge an DS cf-DNA weniger als 0,2 beträgt;
dem Subjekt ein zellulärer Abstoßungsgrad von CR1 zugewiesen wird, falls die Menge an DS cf-DNA zwischen 0,2 und 0,8 beträgt; oder
dem Subjekt ein zellulärer Abstoßungsgrad von CR2 oder größer zugewiesen wird, falls die Menge an DS cf-DNA größer als 0,8 ist,
wobei das Verfahren ferner optional Zuweisen eines zellulären Abstoßungsgrads an das Subjekt basierend auf der bestimmten Menge an DS cf-DNA im Vergleich zu dem Schwellen-DS cf-DNA-Wert und/oder zumindest einer vorherigen DS cf-DNA-Menge umfasst.

3. Verfahren nach einem vorhergehenden Anspruch, wobei:
(i) das Subjekt einen zellulären Abstoßungsgrad, wie CR0 oder CR1 oder wie CR2 oder CR3, aufweist oder dieser Verdacht besteht; und/oder
(ii) bestimmt wurde, dass das Subjekt einen zellulären Abstoßungsgrad wie CR1, CR2 oder niedriger, CR1 oder größer oder CR2 oder größer aufweist; und/oder
(iii) wobei bestimmt wurde, dass das Subjekt einen zellulären Abstoßungsgrad aufweist, wie CR0 oder CR1 oder wie CR2 oder CR3; und/oder
(iv) mit einem oder mehreren zusätzlichen Test(s), der/die vorherig angewendet wurde/n bestimmt wurde, dass das Subjekt den zellulären Abstoßungsgrad aufweist, um die Transplantatabstoßung bei dem Subjekt zu bewerten, oder das Verfahren ferner das Durchführen eines oder mehrerer zusätzlicher Test(s) umfasst, um die Transplantatabstoßung bei dem Subjekt zu bewerten; und/oder
(v) dem Subjekt ein zellulärer Abstoßungsgrad von CR0 zugewiesen wird, falls die Menge an DS cf-DNA weniger als 0,14, 0,15 beträgt; und/oder
(vi) ein oder mehrere zusätzliche Tests indiziert sind, falls die Menge an DS cf-DNA zwischen 0,14 und 0,5, 0,15 und 0,5, 0,2 und 0,5 oder 0,3 und 0,5 liegt.

4. Verfahren zum Bewerten einer Probe eines Transplantatsubjekts, das Verfahren umfassend: (a) Bestimmen einer Menge an spenderspezifischer zellfreier DNA (DS cf-DNA) in einer Probe von dem Subjekt, wobei das Subjekt einen Zustand oder eine Erkrankung des transplantierten Organs aufweist, bei ihm der Verdacht besteht, dass es eine/e solche/n aufweist, erlitten hat oder ein solches Risiko besteht, wobei der Zustand oder die Erkrankung des transplantierten Organs eine kardiale Allotransplantat-Vaskulopathie und/oder das Risiko eines Herzstillstands ist und wobei die Menge an DS cf-DNA bestimmt wird durch: für eine Vielzahl von Einzelnukleotidvarianten- (SNV-)Zielen, Durchführen eines amplifikationsbasierten Quantifizierungsassays, wie eines Polymerase-Kettenreaktions- (PCR-)Quantifizierungsassays, an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3'-Ende relativ zu einem anderen Allel des SNV-Ziels umfasst und spezifisch das eine Allel des SNV-Ziels amplifiziert und ein anderes der zumindest zwei Primerpaare spezifisch zu einem anderen Allel des SNV-Ziels amplifiziert; und
(b) Berichten und/oder Aufzeichnen der Menge an DS cf-DNA, wobei
eine kardiale Allotransplantat-Vaskulopathie und/oder das Risiko eines Herzstillstands indiziert ist, falls die Menge an DS cf-DNA größer als 0,2 ist,
wobei das Verfahren optional ferner Folgendes umfasst:
(c) Vergleichen der Menge an DS cf-DNA mit einem Schwellen-DS cf-DNA-Wert oder zumindest einem vorherigen DS cf-DNA-Wert und/oder wobei der Zustand oder die Erkrankung indiziert ist oder das Subjekt ein erhöhtes Risiko hat, basierend auf der bestimmten Menge an DS cf-DNA im Vergleich zu dem Schwellen-DS cf-DNA-Wert und/oder zumindest einer vorherigen DS cf-DNA-Menge.

5. Verfahren zum Bestimmen eines Behandlungs- oder Überwachungsschemas für ein Transplantationssubjekt, das Verfahren umfassend:
(a) Erhalten einer Menge an spenderspezifischer zellfreier DNA (DS cf-DNA) in einer Probe, die von dem Subjekt erhalten wurde, wobei das Subjekt einen Zustand oder eine Erkrankung des transplantierten Organs aufweist, bei ihm der Verdacht besteht, dass es eine/e solche/n aufweist, erlitten hat oder ein solches Risiko besteht, wobei der Zustand oder die Erkrankung des transplantierten Organs eine kardiale Allotransplantat-Vaskulopathie und/oder das Risiko eines Herzstillstands ist, und wobei
die Menge an DS cf-DNA erhalten wird durch: für eine Vielzahl von Einzelnukleotidvarianten- (SNV)-Zielen Durchführen eines amplifikationsbasierten Quantifizierungsassays, wie eines Polymerase-Kettenreaktions- (PCR-)Quantifizierungsassays, an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3'-Ende relativ zu einem anderen Allel des SNV-Ziels umfasst und spezifisch das eine Allel des SNV-Ziels amplifiziert, und ein anderes der zumindest zwei Primerpaare spezifisch zu einem anderen Allel des SNV-Ziels amplifiziert, und
(b) Vergleichen der Menge an DS cf-DNA mit einem Schwellen-DS cf-DNA-Wert und/oder zumindest einer vorherigen DS cf-DNA-Menge;
(c) Bestimmen eines Behandlungs- oder Überwachungsschemas für das Subjekt basierend auf der bestimmten Menge an DS cf-DNA im Vergleich zu dem Schwellen-DS cf-DNA-Wert und/oder zumindest einer vorherigen DS cf-DNA-Menge, wobei
eine kardiale Allotransplantat-Vaskulopathie und/oder das Risiko eines Herzstillstands indiziert ist, falls die Menge an DS cf-DNA größer als 0,2 ist,
wobei der Zustand oder die Erkrankung optional indiziert ist oder das Subjekt ein erhöhtes Risiko hat, basierend auf der bestimmten Menge an DS cf-DNA im Vergleich zu dem Schwellen-DS cf-DNA-Wert und/oder zumindest einer vorherigen DS cf-DNA-Menge.

6. Verfahren nach Anspruch 4 oder 5, wobei:
(i) das Subjekt einen Herzstillstand erlitten hat oder ein solches Risiko besteht; und/oder
(ii) bestimmt wurde, dass das Subjekt eine kardiale Allotransplantat-Vaskulopathie aufweist oder einen Herzstillstand erlitten hat; und/oder
(iii) mit einem oder mehreren zusätzlichen Test(s), der/die zuvor angewendet wurde/n bestimmt wurde, dass das Subjekt eine kardiale Allotransplantat-Vaskulopathie aufweist, um das Subjekt zu bewerten, oder das Verfahren ferner das Durchführen eines oder mehrerer zusätzlicher Test(s) umfasst, um das Subjekt zu bewerten; und/oder
(iv) eine kardiale Transplantat-Vaskulopathie oder das Risiko eines Herzstillstands indiziert ist, falls die Menge DS cf-DNA gleich oder größer als 0,3 ist.

7. Verfahren zum Bewerten einer Probe von einem Transplantatsubjekt, das Verfahren umfassend: (a) Bestimmen einer Menge an spenderspezifischer zellfreier DNA (DS cf-DNA) in einer Probe von dem Subjekt, wobei das Subjekt eine Antikörper-vermittelte Abstoßung aufweist oder der Verdacht besteht, dass es eine Antikörper-vermittelte Abstoßung aufweist, und wobei die Menge an DS cf-DNA bestimmt wird durch: für eine Vielzahl von Einzelnukleotidvarianten-(SNV-)Zielen Durchführen eines amplifikationsbasierten Quantifizierungsassays, wie ein Polymerase-Kettenreaktions- (PCR-)Quantifizierungsassay an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3'-Ende relativ zu einem anderen Allel des SNV-Ziels umfasst und spezifisch das eine Allel des SNV-Ziels amplifiziert, und ein anderes der zumindest zwei Primerpaare spezifisch zu einem anderen Allel des SNV-Ziels amplifiziert, und
(b) Berichten und/oder Aufzeichnen der Menge an DS cf-DNA, wobei
das Subjekt als eine Antikörper-vermittelte Abstoßung aufweisend klassifiziert wird, falls die Menge an DS cf-DNA größer als 0,2 ist, wobei das Verfahren ferner optional Folgendes umfasst:
(c) Vergleichen der Menge an DS cf-DNA mit einem Schwellen-DS cf-DNA-Wert oder zumindest einem vorherigen DS-cf-DNA-Wert und/oder wobei das Verfahren ferner Folgendes umfasst:
(d) Klassifizieren des Subjekts als eine Antikörper-vermittelte Abstoßung aufweisend, oder dass es ein erhöhtes Risiko hat, basierend auf der bestimmten Menge an DS cf-DNA im Vergleich zu dem Schwellen-DS cf-DNA-Wert und/oder zumindest einer vorherigen DS cf-DNA-Menge.

8. Verfahren zum Bestimmen eines Behandlungs- oder Überwachungsschemas für ein Transplantationssubjekt, das Verfahren umfassend:
(a) Erhalten einer Menge an spenderspezifischer zellfreier DNA (DS cf-DNA) in einer Probe, die von dem Subjekt erhalten wurde, wobei das Subjekt eine Antikörper-vermittelte Abstoßung aufweist oder ein solcher Verdacht besteht, und wobei die Menge an DS cf-DNA erhalten wird durch: für eine Vielzahl von Einzelnukleotidvarianten- (SNV-)Zielen Durchführen eines amplifikationsbasierten Quantifizierungsassays, wie eines Polymerase-Kettenreaktions-(PCR-)Quantifizierungsassays, an der Probe oder einem Teil davon mit zumindest zwei Primerpaaren, wobei jedes Primerpaar einen Vorwärtsprimer und einen Rückwärtsprimer umfasst, wobei eines der zumindest zwei Primerpaare eine vorletzte Fehlpaarung am 3'-Ende in einem Primer relativ zu einem Allel des SNV-Ziels, aber eine doppelte Fehlpaarung am 3' -Ende relativ zu einem anderen Allel des SNV-Ziels umfasst und spezifisch das eine Allel des SNV-Ziels amplifiziert, und ein anderes der zumindest zwei Primerpaare spezifisch zu einem anderen Allel des SNV-Ziels amplifiziert, und
(b) Vergleichen der Menge an DS cf-DNA mit einem Schwellen-DS cf-DNA-Wert und/oder zumindest einer vorherigen DS cf-DNA-Menge;
(c) Bestimmen eines Behandlungs- oder Überwachungsschemas für das Subjekt basierend auf der bestimmten Menge an DS cf-DNA im Vergleich zu dem Schwellen-DS cf-DNA-Wert und/oder zumindest einer vorherigen DS cf-DNA-Menge, wobei
das Subjekt als eine Antikörper-vermittelte Abstoßung aufweisend klassifiziert wird, falls die Menge an DS cf-DNA größer als 0,2 ist,
wobei das Verfahren ferner optional Klassifizieren des Subjekts als eine Antikörper-vermittelte Abstoßung aufweisend, oder dass es ein erhöhtes Risiko hat, basierend auf der bestimmten Menge an DS cf-DNA im Vergleich zu dem Schwellen-DS cf-DNA-Wert und/oder zumindest einer vorherigen DS cf-DNA-Menge umfasst.

9. Verfahren nach Anspruch 7 oder 8, wobei:
(i) bestimmt wurde, dass das Subjekt eine Antikörper-vermittelte Abstoßung aufweist; und/oder
(ii) mit einem oder mehreren zusätzlichen Test(s), der/die zuvor angewendet wurde/n bestimmt wurde, dass das Subjekt eine Antikörper-vermittelte Abstoßung aufweist, um die Transplantatabstoßung bei dem Subjekt zu bewerten, oder das Verfahren ferner das Durchführen eines oder mehrerer zusätzlicher Test(s) umfasst, um das Subjekt zu bewerten.

10. Verfahren nach einem vorhergehenden Anspruch, wobei:
(i) der eine oder die mehreren zusätzlichen Tests eine Biopsie umfassen, wobei optional die Biopsie eine Endomyokardbiopsie (EMB) ist; und/oder
(ii) die Menge in einem Bericht bereitgestellt wird, wobei die Menge optional in einem Bericht bereitgestellt wird, der auch zumindest eine vorherige Menge an DS-cfDNA enthält, die sich in einer Probe von dem Subjekt befand; und/oder
(iii) die Menge in einer Datenbank aufgezeichnet wird, wobei die Datenbank optional auch zumindest eine vorherige Menge an DS cf-DNA enthält, die sich in einer Probe von dem Subjekt befand.

11. Verfahren nach einem vorhergehenden Anspruch, wobei der Genotyp des Spenders bekannt ist oder wobei der Genotyp des Spenders unbekannt ist.

12. Verfahren nach einem vorhergehenden Anspruch, wobei:
(i) zumindest eine Menge in einer Probe bestimmt wird, die dem Subjekt 4, 5, 6, 7 oder 8 Tage nach einer Organtransplantation entnommen wurde; und/oder
(iv) eine Menge an DS cf-DNA, die größer als der Schwellenwert ist und/oder relativ zu der Menge von einem früheren Zeitpunkt erhöht ist, ein erhöhtes oder sich erhöhendes Risiko darstellt; und/oder
(iii) eine Menge an DS cf-DNA, die niedriger als der Schwellenwert ist und/oder gegenüber der Menge von einem früheren Zeitpunkt verringert ist, ein verringertes oder sich verringerndes Risiko darstellt; und/oder
(iv) das Bestimmen eines Überwachungsschemas das Bestimmen der Menge an DS cf-DNA in dem Subjekt im Laufe der Zeit oder zu einem späteren Zeitpunkt umfasst oder dem Subjekt eine solche Überwachung vorgeschlagen wird; wobei das Subjekt optional täglich oder wöchentlich oder wobei das Subjekt monatlich oder zweimonatlich bewertet wird; und/oder
(v) das Subjekt für bis zu 6 Monate, bis zu 8 Monate, bis zu 10 Monate oder bis zu einem Jahr bewertet wird; und/oder
(vi) die Zeit zwischen den Proben verringert wird, falls die Menge an DS cf-DNA relativ zu dem Schwellenwert oder einer Menge von einem früheren Zeitpunkt erhöht ist; und/oder
(vii) die zusätzliche(n) Menge(n) an DS cf-DNA aufgezeichnet oder berichtet werden, wobei die zusätzliche(n) Menge(n) optional in einem Bericht bereitgestellt werden und/oder wobei die zusätzliche(n) Menge(n) in einer Datenbank aufgezeichnet werden; und/oder
(viii) das Bestimmen eines Überwachungsschemas das Verwenden oder Vorschlagen der Verwendung eines oder mehrerer zusätzlicher Test(s) umfasst, um die Transplantatabstoßung bei dem Subjekt zu bewerten, wobei optional der eine oder die mehreren zusätzlichen Test(s) eine Biopsie umfassen, wobei optional die Biopsie eine Endomyokardbiopsie (EMB) ist; und/oder
(ix) der eine oder die mehreren zusätzlichen Test(s) indiziert sind, falls die Menge an DS cf-DNA gleich oder größer als einer der hierin bereitgestellten Schwellen ist; und/oder
(x) das Bestimmen eines Behandlungsschemas das Auswählen oder Empfehlen einer Behandlung für das Subjekt umfasst; und/oder
(xi) das Bestimmen eines Behandlungsschemas das Behandeln des Subjekts umfasst; und/oder
(xii) die Behandlung eine Anti-Abstoßungstherapie umfasst; und/oder
(xiii) die Behandlung eine Behandlung für kardiale Allotransplantat-Vaskulopathie umfasst, wie Retransplantation, perkutane Koronarinterventionen (PCI), koronare Bypass-Transplantation (CABG), transmyokardiale Laser-Revaskularisierung oder Heparininduzierte/vermittelte extrakorporale LDL-Plasmapherese (HELP); und/oder
(xiv) wenn eine kardiale Allotransplantat-Vaskulopathie indiziert ist, die Behandlung eine Behandlung mit einem Statin, einem Antihypertensivum oder einem Anti-Cytomegalovirus-(Anti-CMV-)Mittel umfasst, wobei das Antihypertensivum optional ein Kalziumkanalblocker ist; und/oder
(xv) wenn ein erhöhtes Risiko für einen Herzstillstand indiziert ist, die Behandlung eine Behandlung für einen Herzstillstand umfasst, wie eine perkutane Koronarintervention (Koronarangioplastie), Koronararterien-Bypass-Transplantation oder Hinzufügen eines implantierbaren Kardioverter-Defibrillators (ICD); und/oder
(xvi) wenn ein erhöhtes Risiko für einen Herzstillstand indiziert ist, die Behandlung ein Antiarrhythmikum, eine Blockade des unwillkürlichen Nervensystems oder ein Blutdruckmedikament umfasst; und/oder
(xvii) das Bestimmen eines Behandlungsschemas das Bereitstellen von Informationen über eine Behandlung für das Subjekt umfasst; und/oder
(xviii) die Probe eine Blut-, Plasma- oder Serumprobe ist, wobei die Blutprobe optional eine Plasmaprobe ist; und/oder
(xix) das Transplantationssubjekt ein Herztransplantationssubjekt ist.

## Revendications

1. Méthode d'évaluation d'un échantillon provenant d'un sujet transplanté, la méthode comprenant :
(a) la détermination d'une quantité d'ADN acellulaire spécifique à un donneur (DS cf-DNA) dans un échantillon provenant du sujet, ledit sujet présentant ou étant suspecté de présenter un degré de rejet cellulaire, tel que CR1, CR2 ou inférieur, CR1 ou supérieur, ou CR2 ou supérieur, et ladite quantité de DS cf-DNA étant déterminée par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une desdites au moins deux paires d'amorces comprenant un avant dernier mésappariement en 3' dans une amorce par rapport à un allèle de la cible à SNV, mais un double mésappariement en 3' par rapport à un autre allèle de la cible à SNV et amplifiant spécifiquement ledit un allèle de la cible à SNV, et une autre desdites au moins deux paires d'amorces amplifiant spécifiquement jusqu'à un autre allèle de la cible à SNV ; et
(b) l'indication et/ou l'enregistrement de la quantité de DS cf-DNA
un degré de rejet cellulaire de CR0 étant attribué au sujet si la quantité de DS cf-DNA est inférieure à 0,2 ;
un degré de rejet cellulaire de CR1 étant attribué au sujet si la quantité de DS cf-DNA est comprise entre 0,2 et 0,8 ; ou
un degré de rejet cellulaire supérieur ou égal à CR2 étant attribué au sujet si la quantité de DS cf-DNA est supérieure à 0,8,
éventuellement ladite méthode comprenant en outre :
(c) la comparaison de la quantité de DS cf-DNA à un DS cf-DNA seuil ou à au moins une valeur de DS-cf-DNA antérieure et/ou ladite méthode comprenant en outre :
(d) l'attribution d'un degré de rejet cellulaire pour le sujet, ou comme étant à risque accru, sur la base de la quantité déterminée de DS cf-DNA par rapport à la valeur de DS cf-DNA seuil et/ou d'au moins une quantité de DS cf-DNA antérieure.

2. Méthode de détermination d'un schéma de traitement ou de surveillance pour un sujet transplanté, la méthode comprenant :
(a) l'obtention d'une quantité d'ADN acellulaire spécifique à un donneur (DS cf-DNA) dans un échantillon qui a été prélevé chez le sujet, ledit sujet présentant ou étant suspecté de présenter un degré de rejet cellulaire, tel que CR1, CR2 ou inférieur, CR1 ou supérieur, ou CR2 ou supérieur, et ladite quantité de DS cf-DNA étant obtenue par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une desdites au moins deux paires d'amorces comprenant un avant dernier mésappariement en 3' dans une amorce par rapport à un allèle de la cible à SNV, mais un double mésappariement en 3' par rapport à un autre allèle de la cible à SNV et amplifiant spécifiquement ledit un allèle de la cible à SNV, et une autre desdites au moins deux paires d'amorces amplifiant spécifiquement jusqu'à un autre allèle de la cible à SNV ;
(b) la comparaison de la quantité de DS cf-DNA à une valeur de DS cf-DNA seuil et/ou à au moins une quantité de DS cf-DNA antérieure ; et
(c) la détermination d'un schéma de traitement ou de surveillance pour le sujet sur la base de la quantité déterminée de DS cf-DNA par rapport à la valeur de DS cf-DNA seuil et/ou d'au moins une quantité de DS cf-DNA antérieure,
un degré de rejet cellulaire de CR0 étant attribué au sujet si la quantité de DS cf-DNA est inférieure à 0,2 ;
un degré de rejet cellulaire de CR1 étant attribué au sujet si la quantité de DS cf-DNA est comprise entre 0,2 et 0,8 ; ou
un degré de rejet cellulaire supérieur ou égal à CR2 étant attribué au sujet si la quantité de DS cf-DNA est supérieure à 0,8,
éventuellement, ladite méthode comprenant en outre l'attribution d'un degré de rejet cellulaire au sujet sur la base de la quantité déterminée de DS cf-DNA par rapport à la valeur de DS cf-DNA seuil et/ou d'au moins une quantité de DS cf-DNA antérieure.

3. Méthode selon l'une quelconque des revendications précédentes :
(i) ledit sujet présentant ou étant suspecté de présenter un degré de rejet cellulaire, tel que CR0 ou CR1 ou tel que CR2 ou CR3 ; et/ou
(ii) ledit sujet ayant été déterminé comme présentant un degré de rejet cellulaire, tel que CR1, CR2 ou inférieur, CR1 ou supérieur, ou CR2 ou supérieur ; et/ou
(iii) ledit sujet ayant été déterminé comme présentant un degré de rejet cellulaire, tel que CR0 ou CR1 ou tel que CR2 ou CR3 ; et/ou
(iv) ledit sujet ayant été déterminé comme présentant le degré de rejet cellulaire avec un ou plusieurs test(s) supplémentaire(s) préalablement appliqué(s) pour évaluer le rejet de greffe chez le sujet ou ladite méthode comprenant en outre la réalisation d'un ou de plusieurs test(s) supplémentaire(s) pour évaluer le rejet de greffe chez le sujet ; et/ou
(v) un degré de rejet cellulaire de CR0 étant attribué au sujet si la quantité de DS cf-DNA est inférieure à 0,14, 0,15 ; et/ou
(vi) un ou plusieurs test(s) supplémentaire(s) étant indiqué(s) si la quantité de DS cf-DNA est comprise entre 0,14 et 0,5, 0,15 et 0,5, 0,2 et 0,5 ou 0,3 et 0,5.

4. Méthode d'évaluation d'un échantillon provenant d'un sujet transplanté, la méthode comprenant :
(a) la détermination d'une quantité d'ADN acellulaire spécifique à un donneur (DS cf-DNA) dans un échantillon provenant du sujet, ledit sujet souffrant, étant suspecté de souffrir, ayant souffert ou étant à risque de souffrir d'une condition ou d'une maladie de l'organe transplanté, ladite condition ou ladite maladie de l'organe transplanté étant une vasculopathie d'allogreffe cardiaque et/ou un risque d'arrêt cardiaque, et ladite quantité de DS cf-DNA étant déterminée par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une desdites au moins deux paires d'amorces comprenant un avant dernier mésappariement en 3' dans une amorce par rapport à un allèle de la cible à SNV, mais un double mésappariement en 3' par rapport à un autre allèle de la cible à SNV et amplifiant spécifiquement ledit un allèle de la cible à SNV, et une autre desdites au moins deux paires d'amorces s'amplifiant spécifiquement jusqu'à un autre allèle de la cible à SNV ; et
(b) la notification et/ou l'enregistrement de la quantité de DS cf-DNA
une vasculopathie d'allogreffe cardiaque et/ou un risque d'arrêt cardiaque étant indiqué si la quantité de DS cf-DNA est supérieure à 0,2,
éventuellement ladite méthode comprenant en outre :
(c) la comparaison de la quantité de DS cf-DNA à une valeur de DS cf-DNA seuil ou à au moins une valeur de DS cf-DNA antérieure, et/ou ladite condition ou ladite maladie étant indiquée, ou ledit sujet étant à risque accru, sur la base de la quantité de DS cf-DNA déterminée par rapport à la valeur de DS cf-DNA seuil et/ou à au moins une quantité de DS cf-DNA antérieure.

5. Méthode de détermination d'un schéma de traitement ou de surveillance pour un sujet transplanté, la méthode comprenant :
(a) l'obtention d'une quantité d'ADN acellulaire spécifique à un donneur (DS cf-DNA) dans un échantillon qui a été prélevé chez le sujet, ledit sujet souffrant, étant soupçonné de souffrir, ayant souffert ou étant à risque de souffrir d'une condition ou d'une maladie de l'organe transplanté, ladite condition ou ladite maladie de l'organe transplanté étant une vasculopathie d'allogreffe cardiaque et/ou un risque d'arrêt cardiaque, et ladite quantité de DS cf-DNA étant obtenue par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une des au moins deux paires d'amorces comprenant un avant dernier mésappariement en 3' dans une amorce par rapport à un allèle de la cible à SNV, mais un double mésappariement en 3' par rapport à un autre allèle de la cible SNV et amplifiant spécifiquement l'allèle de la cible SNV, et une autre desdites au moins deux paires d'amorces s'amplifiant spécifiquement jusqu'à un autre allèle de la cible à SNV ;
(b) la comparaison de la quantité de DS cf-DNA à une valeur de DS cf-DNA seuil et/ou à au moins une quantité de DS cf-DNA antérieure ; et
(c) la détermination d'un schéma de traitement ou de surveillance pour le sujet sur la base de la quantité déterminée de DS cf-DNA par rapport à la valeur de DS cf-DNA seuil et/ou d'au moins une quantité de DS cf-DNA antérieure,
une vasculopathie d'allogreffe cardiaque et/ou un risque d'arrêt cardiaque étant indiqué si la quantité de DS cf-DNA est supérieure à 0,2,
éventuellement, ladite condition ou ladite maladie étant indiquée, ou ledit sujet étant à risque accru, sur la base de la quantité déterminée de DS cf-DNA par rapport à la valeur de DS cf-DNA seuil et/ou d'au moins une quantité de DS cf-DNA antérieure.

6. Méthode selon la revendication 4 ou 5 :
(i) ledit sujet ayant subi ou risquant de subir un arrêt cardiaque ; et/ou
(ii) ledit sujet ayant été déterminé comme souffrant d'une vasculopathie d'allogreffe cardiaque ou ayant subi un arrêt cardiaque ; et/ou
(iii) ledit sujet ayant été déterminé comme souffrant d'une vasculopathie d'allogreffe cardiaque avec un ou plusieurs test(s) supplémentaire(s) préalablement appliqué(s) pour évaluer le sujet ou ladite méthode comprenant en outre la réalisation d'un ou de plusieurs test(s) supplémentaire(s) pour évaluer le sujet ; et/ou
(iv) une vasculopathie de greffe cardiaque ou un risque d'arrêt cardiaque étant indiqué si la quantité de DS cf-DNA est supérieure ou égale à 0,3.

7. Méthode d'évaluation d'un échantillon provenant d'un sujet transplanté, la méthode comprenant :
(a) la détermination d'une quantité d'ADN acellulaire spécifique à un donneur (DS cf-DNA) dans un échantillon provenant du sujet, ledit sujet présentant ou étant suspecté de présenter un rejet à médiation par anticorps et ladite quantité de DS cf-DNA étant déterminée par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une desdites au moins deux paires d'amorces comprenant un avant dernier mésappariement en 3' dans une amorce par rapport à un allèle de la cible à SNV, mais un double mésappariement en 3' par rapport à un autre allèle de la cible à SNV et amplifiant spécifiquement l'allèle de la cible à SNV, et une autre desdites au moins deux paires d'amorces s'amplifiant spécifiquement jusqu'à un autre allèle de la cible à SNV ; et
(b) la notification et/ou l'enregistrement de la quantité de DS cf-DNA
ledit sujet étant classé comme présentant un rejet à médiation par anticorps si la quantité de DS cf-DNA est supérieure à 0,2,
éventuellement ladite méthode comprenant en outre :
(c) la comparaison de la quantité de DS cf-DNA à une valeur de DS cf-DNA seuil ou à au moins une valeur DS cf-DNA antérieure et/ou ladite méthode comprenant en outre :
(d) le classement du sujet comme présentant un rejet à médiation par anticorps, ou à risque accru, sur la base de la quantité déterminée de DS cf-DNA par rapport à la valeur de DS cf-DNA seuil et/ou d'au moins une quantité de DS cf-DNA antérieure.

8. Méthode de détermination d'un schéma de traitement ou de surveillance pour un sujet transplanté, la méthode comprenant :
(a) l'obtention d'une quantité d'ADN acellulaire spécifique à un donneur (DS cf-DNA) dans un échantillon qui a été prélevé chez le sujet, le sujet présentant ou étant suspecté de présenter un rejet à médiation par anticorps, et ladite quantité de DS cf-DNA étant obtenue par : pour une pluralité de cibles à variant mononucléotidique (SNV), la réalisation d'un dosage de quantification basé sur l'amplification, tel qu'un dosage de quantification par amplification en chaîne par polymérase (PCR), sur l'échantillon, ou une partie de celui-ci, avec au moins deux paires d'amorces, chaque paire d'amorces comprenant une amorce directe et une amorce inverse, l'une desdites au moins deux paires d'amorces comprenant un avant dernier mésappariement en 3' dans une amorce par rapport à un allèle de la cible à SNV, mais un double mésappariement en 3' par rapport à un autre allèle de la cible à SNV et amplifiant spécifiquement ledit un allèle de la cible à SNV, et une autre desdites au moins deux paires d'amorces s'amplifiant spécifiquement jusqu'à un autre allèle de la cible à SNV ;
(b) la comparaison de la quantité de DS cf-DNA à une valeur de DS cf-DNA seuil et/ou à au moins une quantité de DS cf-DNA antérieure ; et
(c) la détermination d'un schéma de traitement ou de surveillance pour le sujet sur la base de la quantité déterminée de DS cf-DNA par rapport à la valeur de DS cf-DNA seuil et/ou d'au moins une quantité de DS cf-DNA antérieure,
ledit sujet étant classé comme présentant un rejet à médiation par anticorps si la quantité de DS cf-DNA est supérieure à 0,2,
éventuellement ladite méthode comprenant en outre le classement du sujet comme présentant un rejet à médiation par anticorps, ou à risque accru, sur la base de la quantité déterminée de DS cf-DNA par rapport à la valeur de DS cf-DNA seuil et/ou d'au moins une quantité de DS cf-DNA antérieure.

9. Méthode selon la revendication 7 ou 8 :
(i) ledit sujet ayant été déterminé comme présentant un rejet à médiation par anticorps ; et/ou
(ii) ledit sujet ayant été déterminé comme présentant un rejet à médiation par anticorps avec un ou plusieurs test(s) supplémentaire(s) préalablement appliqué(s) pour évaluer le rejet de greffe chez le sujet ou ladite méthode comprenant en outre la réalisation d'un ou de plusieurs test(s) supplémentaire(s) pour évaluer le sujet.

10. Méthode selon l'une quelconque des revendications précédentes :
(i) lesdits un ou plusieurs tests supplémentaires comprenant une biopsie, éventuellement ladite biopsie étant une biopsie endomyocardique (BEM) ; et/ou
(ii) ladite quantité étant fournie dans un rapport, éventuellement ladite quantité étant fournie dans une notification contenant également au moins une quantité de DS-cfDNA antérieure qui se trouvait dans un échantillon du sujet ; et/ou
(iii) ladite quantité étant enregistrée dans une base de données, éventuellement ladite base de données contenant également au moins une quantité de DS cf-DNA antérieure qui se trouvait dans un échantillon du sujet.

11. Méthode selon l'une quelconque des revendications précédentes, ledit génotype du donneur étant connu ou ledit génotype du donneur étant inconnu.

12. Méthode selon l'une quelconque des revendications précédentes :
(i) au moins une quantité étant déterminée dans un échantillon prélevé chez le sujet 4, 5, 6, 7 ou 8 jours après une greffe d'organe ; et/ou
(ii) une quantité de DS cf-DNA qui est supérieure à la valeur seuil et/ou augmentée par rapport à la quantité d'un point temporel antérieur représentant un risque accru ou croissant ; et/ou
(iii) une quantité de DS cf-DNA qui est inférieure à la valeur seuil et/ou diminuée par rapport à la quantité d'un point temporel antérieur représentant un risque diminué ou décroissant ; et/ou
(iv) ladite détermination d'un schéma de surveillance comprenant la détermination de la quantité de DS cf-DNA chez le sujet au fil du temps ou à un moment ultérieur, ou la suggestion d'une telle surveillance au sujet ; éventuellement, ledit sujet étant évalué quotidiennement ou hebdomadairement ou ledit sujet étant évalué mensuellement ou bimensuellement ; et/ou
(v) ledit sujet étant évalué pendant une période allant jusqu'à 6 mois, jusqu'à 8 mois, jusqu'à 10 mois ou jusqu'à un an ; et/ou
(vi) ledit temps entre les échantillons étant réduit si la quantité de DS cf-DNA est augmentée par rapport au seuil ou à une quantité d'un point temporel antérieur ; et/ou
(vii) lesdites une ou plusieurs quantités supplémentaires de DS cf-DNA étant enregistrées ou notifiées, éventuellement ladite ou lesdites quantités supplémentaires étant fournies dans une notification et/ou ladite ou lesdites quantités supplémentaires étant enregistrées dans une base de données ; et/ou
(viii) ladite détermination d'un schéma de surveillance comprenant l'utilisation ou la suggestion de l'utilisation d'un ou de plusieurs test(s) supplémentaire(s) pour évaluer le rejet de greffe chez le sujet, éventuellement lesdits un ou plusieurs test(s) supplémentaire(s) comprenant une biopsie, éventuellement ladite biopsie étant une biopsie endomyocardique (BEM) ; et/ou
(ix) lesdits un ou plusieurs test(s) supplémentaire(s) étant indiqué(s) si la quantité de DS cf-DNA est supérieure ou égale à l'un quelconque des seuils prévus dans la description ; et/ou
(x) ladite détermination d'un schéma de traitement comprenant la sélection ou la suggestion d'un traitement pour le sujet ; et/ou
(xi) ladite détermination d'un schéma de traitement comprenant le traitement du sujet ; et/ou
(xii) ledit traitement comprenant un traitement anti-rejet ; et/ou
(xiii) ledit traitement comprenant un traitement pour une vasculopathie d'allogreffe cardiaque, telle que la retransplantation, les interventions coronariennes percutanées (ICP), la chirurgie de pontage aorto-coronaire (CABG), la revascularisation transmyocardique au laser ou la plasmaphérèse extracorporelle des LDL induite/médiée par l'héparine (HELP) ; et/ou
(xiv) lorsque la vasculopathie d'allogreffe cardiaque est indiquée, ledit traitement comprenant un traitement avec une statine, un agent antihypertenseur ou un agent anticytomégalovirus (anti-CMV), éventuellement ledit agent antihypertenseur étant un bloqueur des canaux calciques ; et/ou
(xv) lorsqu'un risque accru d'arrêt cardiaque est indiqué, ledit traitement comprenant un traitement pour un arrêt cardiaque, tel qu'une intervention coronarienne percutanée (angioplastie), une chirurgie de pontage aorto-coronaire ou l'ajout d'un défibrillateur cardiaque implantable (DCI) ; et/ou
(xvi) lorsqu'un risque accru d'arrêt cardiaque est indiqué, ledit traitement comprenant un agent anti-arythmique, un inhibiteur du système nerveux involontaire ou un médicament contre la pression artérielle ; et/ou
(xvii) ladite détermination d'un schéma de traitement comprenant la fourniture d'informations sur un traitement au sujet ; et/ou
(xviii) ledit échantillon étant un échantillon de sang, de plasma ou de sérum, éventuellement ledit échantillon de sang étant un échantillon de plasma ; et/ou
(xix) ledit sujet transplanté étant un sujet transplanté cardiaque.
